(19) Europäisches Patentamt
European Patent Office
Office européen des brevets

(11) EP 3 219 744 A1

(12) **EUROPEAN PATENT APPLICATION**
published in accordance with Art. 153(4) EPC

(43) Date of publication:
**20.09.2017 Bulletin 2017/38**

(21) Application number: **15857807.0**

(22) Date of filing: **19.10.2015**

(51) Int Cl.:
***C08G 67/02*** *(2006.01)*
***C08L 83/04*** *(2006.01)*
***C08J 5/00*** *(2006.01)*
***B29C 45/00*** *(2006.01)*
***F24C 15/08*** *(2006.01)*
***A47L 7/00*** *(2006.01)*
***B60J 3/00*** *(2006.01)*
***B60J 5/00*** *(2006.01)*
***E05B 85/20*** *(2014.01)*

(86) International application number:
**PCT/KR2015/011026**

(87) International publication number:
**WO 2016/072641 (12.05.2016 Gazette 2016/19)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(30) Priority:
**07.11.2014 KR 20140154631**
**07.11.2014 KR 20140154632**
**19.11.2014 KR 20140161980**
**19.11.2014 KR 20140161984**
**19.11.2014 KR 20140161985**
**19.11.2014 KR 20140162002**
**19.11.2014 KR 20140162020**
**19.11.2014 KR 20140162021**
**19.11.2014 KR 20140162023**
**19.11.2014 KR 20140162024**
**27.05.2015 KR 20150073986**
**27.05.2015 KR 20150073987**
**27.05.2015 KR 20150073988**
**27.05.2015 KR 20150073990**
**27.05.2015 KR 20150073991**
**27.05.2015 KR 20150073992**
**27.05.2015 KR 20150074015**
**27.05.2015 KR 20150074018**
**27.05.2015 KR 20150074019**
**27.05.2015 KR 20150074021**
**27.05.2015 KR 20150074022**
**27.05.2015 KR 20150074025**
**27.05.2015 KR 20150074026**
**27.05.2015 KR 20150074027**
**27.05.2015 KR 20150074030**
**27.05.2015 KR 20150074031**
**27.05.2015 KR 20150074034**
**27.05.2015 KR 20150074036**

(71) Applicant: **Hyosung Corporation**
**Seoul 121-720 (KR)**

(72) Inventors:
- **CHOI, Jong In**
  **Seoul 08329 (KR)**
- **YOON, Sung Kyoun**
  **Anyang-si**
  **Gyeonggi-do 14044 (KR)**
- **KIM, Ka Young**
  **Ansan-si**
  **Gyeonggi-do 15402 (KR)**
- **KIM, Seong Hwan**
  **Seoul 06189 (KR)**
- **LEE, Jong**
  **Seoul 07541 (KR)**

(74) Representative: **v. Bezold & Partner Patentanwälte - PartG mbB**
**Akademiestraße 7**
**80799 München (DE)**

(54) **POLYKETONE RESIN COMPOSITION WITH EXCELLENT WEAR RESISTANCE**

EP 3 219 744 A1

(57) The present invention relates to a polyketone resin composition which is prepared from a polyketone co-polymer comprising repeating units represented by general formulae (1) and (2) below and exhibits excellent wear resistance and impact resistance, and thus can be used for gears, microwave containers, cams, helmet gears for electric welding, plastic boards, yarn guides, bedding cleaner cams, office supplies, window drums for automobiles, sun visor retainers, door frame inner covers for automobiles, safety belt jointers for automobiles, auto gear slides for automobiles, door latch housings for automobiles, slide guides for automobiles, switch shafts in heating, ventilation and air conditioning(HAVC) systems for automobiles, actuator gears for automobiles, trim mounting clips for automobiles, cup holders for automobiles, roof racks for automobiles, outside door handles for automobiles, air intake garnishes for automobiles, medical transportation trays, medical pipettes, refrigerator door closures, cellular phone polishing fixtures, ATM gears, etc.

$-[-CH_2CH_2-CO]x-$ (1)

$-[-CH_2-CH(CH_3)-CO]y-$ (2)

(wherein x and y denote the mol% of each of the general formula (1) and (2) in a polymer, and y/x is from 0.03 to 0.3)

FIG.1

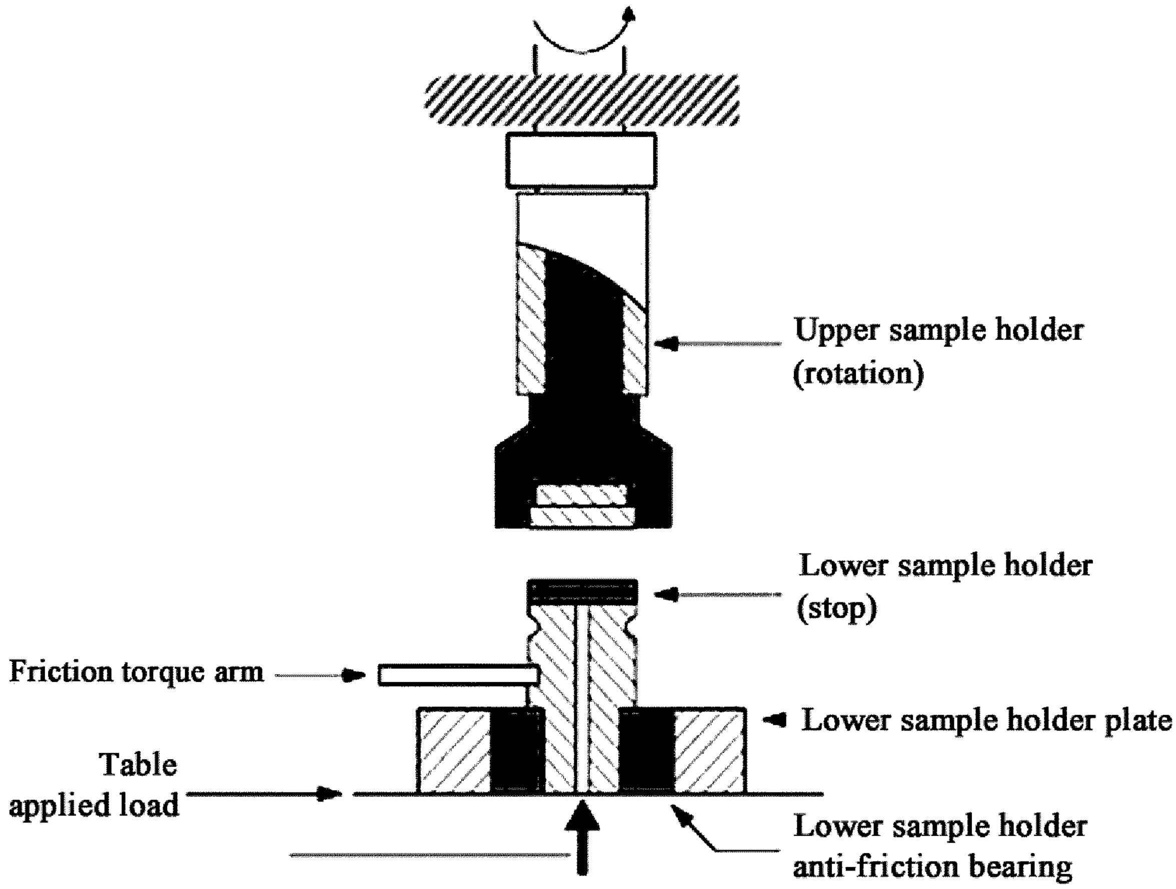

## Description

### Technical Field

The present invention relates to a polyketone resin composition with excellent wear resistance and impact resistance, and more particularly, to a polyketone resin composition which is produced by mixing a wear resistant agent with a polyketone copolymer and can be used for gears, microwave containers, cams, helmet gears for electric welding, plastic boards, yarn guides, bedding cleaner cams, office supplies, window drums for automobiles, sun visor retainers, door frame inner covers for automobiles, safety belt jointers for automobiles, auto gear slides for automobiles, door latch housings for automobiles, slide guides for automobiles, switch shafts in heating, ventilation and air conditioning (HAVC) systems for automobiles, actuator gears for automobiles, trim mounting clips for automobiles, cup holders for automobiles, roof racks for automobiles, outside door handles for automobiles, air intake garnishes for automobiles, medical transportation trays, medical pipettes, refrigerator door closures, cellular phone polishing fixtures, ATM gears, etc.

### Background Art

Engineering plastics such as polyacetal, polyamide, polyester, polycarbonate and etc. are conventionally used in various industrial fields such as gears, microwave containers, cams, helmet gears for electric welding, plastic boards, yarn guides, bedding cleaner cams, office supplies, window drums for automobiles, sun visor retainers, door frame inner covers for automobiles, safety belt jointers for automobiles, auto gear slides for automobiles, door latch housings for automobiles, slide guides for automobiles, switch shafts in heating, ventilation and air conditioning (HAVC) systems for automobiles, actuator gears for automobiles, trim mounting clips for automobiles, cup holders for automobiles, roof racks for automobiles, outside door handles for automobiles, air intake garnishes for automobiles, medical transportation trays, medical pipettes, refrigerator door closures, cellular phone polishing fixtures and etc. The engineering plastics are excellent in mechanical properties, fatigue resistance, oil resistance, and electrical properties, but they are unsatisfactory in wear resistance due to load or external force and impact resistance to withstand external impacts.

Recently, research is underway to replace the engineering plastic with polyketone (PK). The polyketone is obtained by polymerizing carbon monoxide (CO) and olefin such as ethylene and propylene using a transition metal complex such as palladium (Pd) or nickel (Ni) as a catalyst, thereby alternately bonding carbon monoxide and olefin. The polyketone thus produced is not only low in raw material and polymerization process cost as compared with conventional engineering plastics such as polyacetal, polyamide, polyester and polycarbonate but also superior in terms of wear resistance and impact resistance. In addition, it has a same level of strength as conventional engineering plastics.

Looking at techniques for improving the wear resistance of conventional engineering plastics, Korean Registered Patent Application No. 10-1086028 discloses a method of improving a wear resistance of plastics by uniformly mixing particulate carbon black with any one molten plastic material selected from a group comprising polyethylene (PE), polyacetal (POM), polyurethane, and nylon at a weight ratio of 90 to 110:5 to 35. In case of polyketone, United States Patent No. 4,870,133 discloses a technique for blending polytetrafluoroethylene with polyketone to improve melt strength.

However, research to improve the wear resistance of polyketone has not yet been conducted or reported.

### Disclosure

### Technical Problem

The present invention provides a polyketone resin composition with excellent wear resistance and impact resistance and a method for producing the same.

The polyketone resin composition of the present invention can be used as gears, microwave containers, cams, helmet gears for electric welding, plastic boards, yarn guides, bedding cleaner cams, office supplies, window drums for automobiles, sun visor retainers, door frame inner covers for automobiles, safety belt jointers for automobiles, auto gear slides for automobiles, door latch housings for automobiles, slide guides for automobiles, switch shafts in heating, ventilation and air conditioning (HAVC) systems for automobiles, actuator gears for automobiles, trim mounting clips for automobiles, cup holders for automobiles, roof racks for automobiles, outside door handles for automobiles, air intake garnishes for automobiles, medical transportation trays, medical pipettes, refrigerator door closures, cellular phone polishing fixtures, ATM gears and etc.

### Technical Solution

The present invention is directed to providing a vehicle fuel tank manufactured by injection-molding a blend of 100 parts by weight of a polyketone copolymer comprising repeating units represented by following general formula (1)

and (2) and having y/x of 0.03 to 0.3, and 0.1 to 20 parts by weight of at least one or more kinds of wear resistant agent selected from a group comprising silicon, polytetrafluoroethylene, calcium carbonate, maleic acid, molybdenum, glass fiber and magnesium stearate.

$$-(CH_2CH_2\text{-}CO)x- \qquad (1)$$

$$-(CH_2CH(CH_3)\text{-}CO)y- \qquad (2)$$

(x and y are mole% of each of the general formula (1) and (2) in a polymer)

Also, the present invention provides gears, microwave containers, cams, helmet gears for electric welding, plastic boards, yarn guides, bedding cleaner cams, office supplies, window drums for automobiles, sun visor retainers, door frame inner covers for automobiles, safety belt jointers for automobiles, auto gear slides for automobiles, door latch housings for automobiles, slide guides for automobiles, switch shafts in heating, ventilation and air conditioning (HAVC) systems for automobiles, actuator gears for automobiles, trim mounting clips for automobiles, cup holders for automobiles, roof racks for automobiles, outside door handles for automobiles, air intake garnishes for automobiles, medical transportation trays, medical pipettes, refrigerator door closures, cellular phone polishing fixtures manufactured by injection-molding a polyketone copolymer comprising repeating units represented by following general formula (1) and (2) and having y/x of 0.03 to 0.3.

$$-(CH_2CH_2\text{-}CO)x- \qquad (1)$$

$$-(CH_2CH(CH_3)\text{-}CO)y- \qquad (2)$$

(x and y are mole% of each of the general formula (1) and (2) in a polymer)

The present invention provides polyketone molded components manufactured by injection molding polyketone composition manufactured by blending a linear alternating polyketone comprising carbon monoxide and at least one kind of olefinically unsaturated hydrocarbon; and at least one or more kinds of wear resistant agent selected from a group comprising silicon, polytetrafluoroethylene, calcium carbonate, maleic acid, molybdenum, glass fiber and magnesium stearate, and having wear amount of 0.005 g or less measured at speed of 50 rpm, a load of 150 N and wear distance of 3 km, wherein the linear alternating polyketone preferably has a molar ratio of ethylene to propylene of 9 to 24:1, a polyketone content of 80 to 99.9 weight% based on total weight of the polyketone composition and a content of wear resistant agent of 0.1 to 20 weight%, intrinsic viscosity of 1.0 to 2.0 dl/g and a molecular weight distribution of 1.5 to 2.5, and the polyketone molded components are one selected from a group comprising a wear part in OA, an ATM gear, an electric/electronic gear, a city gas meter gear and razor printer toner gear.

The present invention provides microwave containers manufactured by injection molding a linear alternating polyketone polymer comprising carbon monoxide and at least one kind of olefinically unsaturated hydrocarbon and having a residual amount of palladium catalyst of 50 ppm or less and a molecular weight distribution of 1.5 to 2.5, wherein the linear alternating polyketone polymer has a molar ratio of ethylene to propylene of 9 to 24:1, intrinsic viscosity of 1.0 to 2.0 dl/g and wear amount of 1.0 $mm^3$/kg/km or less in base state.

The present invention provides polyketone cams manufactured with polyketone copolymer comprising repeating units represented by general formula (1) and (2) described above, wherein the polyketone copolymer has intrinsic viscosity of 1.0 to 2.0 dl/g, a noise level during wear measurement of 70 dB or less and wear amount measured using a Taber wear tester (manufactured by DAITO ELECTRON CO., LTD., condition: 1 kg of load and wear wheel H-22) according to JIS K-7311 after a test piece was left at 25°C for 2 days of 25 mg or less.

The present invention provides gears attached to an electric welding helmet manufactured with a polyketone resin comprising repeating units represented by general formula (1) and (2) described above, wherein the polyketone resin has intrinsic viscosity of 1.0 to 2.0 dl/g, a molecular weight distribution of 1.5 to 2.5, and the gear has a wear coefficient ($K_{LNP}$) of 200 to 300 measured at 25°C using a thrust washer test device.

The present invention provides polyketone plastic boards for blanking and molding a plastic gear which is a polyketone copolymer comprising repeating units represented by general formula (1) and (2) described above and has wear amount of 0.020 g or less in speed of 50 rpm, a load of 150 N and wear distance of 3 km under JIS K7218 standard, wherein wear resistant agent is added and it is a silicone resin in powder form and contains 0.1 to 15 parts by weight relative to 100 parts by weight of the polyketone copolymer, and the polyketone copolymer has intrinsic viscosity of 1.0 to 2.0 dl/g and a molecular weight distribution of 1.5 to 2.5.

The present invention provides yarn guides, which guide a yarn wound on a bobbin to a needle, comprising; a yarn guide hole through which a yarn passes; a yarn path for guiding the yarn passing through the yarn guide hole to the needles; a guide member and a roller, and manufactured with a polyketone copolymer comprising repeating units represented by general formula (1) and (2) described above, wherein intrinsic viscosity of the polyketone copolymer is

1.0 to 2.0 dl/g, a ligand of a catalyst composition used in polymerization of the polyketone copolymer is ((2,2-dimethyl-1,3-dioxane-5,5-diyl)bis(methylene))bis(bis(2-methoxyphenyl)phosphine) and a molecular weight distribution of the polyketone copolymer is 1.5 to 2.5.

The present invention provides polyketone microwave components manufactured by injection molding a linear alternating polyketone which is polyketone copolymer comprising repeating units represented by general formula (1) and (2) described above, and has y/x of 0.03 to 0.3, wherein intrinsic viscosity of the polyketone is 1.0 to 2.0 dl/g, wear amount of the microwave components manufactured by injection molding is 1.0 $mm^3$/kg/km or less, the microwave components are turntable rollers or turntable brackets, a thermal strain temperature of the microwave components is 130°C or higher and the microwave components have an injection cycle of less than 20 sec.

The present invention provides cams for bedding cleaner, which is fixed to a rotating shaft of a vibrator for vibrating a suction mechanism of a bedding cleaner and linearly reciprocates connecting road, manufactured with polyketone copolymer comprising repeating units represented by general formula (1) and (2) described above, wherein the polyketone copolymer is prepared by a step of preparing a catalyst composition comprising a palladium compound, an acid having a pKa value of 6 or less, and a bidentate compound of phosphorus; a step of preparing a mixed solvent comprising methanol and water; a step of conducting polymerization in presence of the catalyst composition and the mixed solvent to prepare a linear terpolymer of carbon monoxide, ethylene and propene; a step of obtaining polyketone resin removing a remaining catalyst composition in the prepared linear terpolymer with an alcohol solvent, intrinsic viscosity of the polyketone copolymer measured at 25°C by HFIP (hexa-fluoroisopropanol) is 1.0 to 2.0 dl/g and wear amount of cams is 25 mg or less.

The present invention provides office supplies manufactured by injection molding a linear alternating polyketone comprising carbon monoxide and at least one kind of olefinically unsaturated hydrocarbon and having a molecular weight distribution of 1.5 to 2.5 and a residual amount of palladium catalyst of 20 ppm or less, wherein impact strength is 8 to 15 kJ/$m^2$, a ligand of a catalyst composition in polymerization of the linear alternating polyketone is ((2,2-dimethyl-1,3-dioxane-5,5-diyl)bis(methylene))bis(bis(2-methoxyphenyl)phosphine), it is manufactured by injection molding a polyketone composition produced by blending a wear resistant agent and the linear alternating polyketone having wear resistance of 1.0 $mm^3$/kg/km or less in a base state and intrinsic viscosity of 1.0 to 2.0 dl/g and comprising carbon monoxide and at least one kind of olefinic unsaturation hydrocarbon and also having a molecular weight distribution of 1.5 to 2.5 and a residual amount of a palladium catalyst of 20 ppm or less. In this case, wear amount is 0.1 $mm^3$/kg/km or less and content of wear resistant agent is 0.5 to 2.0 weight% based on total polyketone composition, and the wear resistant agent is a silicone oil or silicone gum.

The present invention provides window drums for automobiles manufactured by injection molding a polyketone composition comprising a linear alternating polyketone polymer comprising carbon monoxide and at least one kind of olefinically unsaturated hydrocarbon and having a residual amount of a palladium catalyst of 5 to 50 ppm and a molecular weight distribution of 1.5 to 3.0, wherein the polyketone composition further comprises a silicon-based wear resistant agent, content of the silicon-based wear resistant agent is 2 to 20 weight% based on 100 weight% of the entire polyketone composition, a molar ratio of ethylene to propylene is 99:1 to 85:15, intrinsic viscosity of the linear alternating polyketone polymer is 1.2 to 2.0 dl/g, and the window drums for automobiles has impact strength of 10 kJ/$m^2$ or more and wear resistance in the base state of 0.015 g or less.

The present invention provides sun visor retainers manufactured by injection molding a linear alternating polyketone polymer comprising carbon monoxide and at least one kind of olefinically unsaturated hydrocarbon and having a residual amount of palladium catalyst of 5 to 50 ppm and a molecular weight distribution of 1.5 to 3.0, wherein a molar ratio of ethylene to propylene is 99:1 to 85:15, intrinsic viscosity is 1.2 to 2.0 dl/g, and the sun visor retainers for automobiles have wear resistance Rmax of 1.0 or less.

The present invention provides door frame inner covers for automobiles manufactured by injection molding a blend comprising 60 to 85 weight% of a linear alternating polyketone polymer comprising carbon monoxide and at least one kind of olefinically unsaturated hydrocarbon and having a residual amount of palladium catalyst of 5 to 50 ppm and a molecular weight distribution of 1.5 to 3.0, and 15 to 40 weight% of glass fiber, wherein a molar ratio of ethylene to propylene is 99:1 to 85:15, intrinsic viscosity is 1.2 to 2.0 dl/g, and the door frame inner covers for automobiles have impact strength of 20 kJ/$m^2$ or more and a dimensional change rate of 2% or less.

The present invention provides safety belt jointers for automobiles manufactured by injection molding a polyketone composition comprising a linear alternating polyketone polymer comprising carbon monoxide and at least one kind of olefinically unsaturated hydrocarbon and having a residual amount of palladium catalyst of 5 to 50 ppm and a molecular weight distribution of 1.5 to 3.0, wherein the polyketone composition further comprises at least one kind selected from a group comprising a silicon-based wear resistant agent, a glass fiber and a polytetrafluoroethylene resin, a molar ratio of ethylene to propylene is 99:1 to 85:15, intrinsic viscosity of the linear alternating polyketone polymer is 1.2 to 2.0 dl/g, and the safety belt jointers for automobiles have impact strength of 10 kJ/$m^2$ or more and wear amount is 0.015 g or less in a base state.

The present invention provides auto gear slides for automobiles manufactured by injection molding a polyketone

composition comprising a linear alternating polyketone polymer comprising carbon monoxide and at least one kind of olefinically unsaturated hydrocarbon and having a residual amount of palladium catalyst of 5 to 50 ppm and a molecular weight distribution of 1.5 to 3.0, wherein the polyketone composition further comprises at least one kind selected from a group comprising a silicon-based wear resistant agent, a glass fiber and a polytetrafluoroethylene resin, a molar ratio of ethylene to propylene is 99:1 to 85:15, intrinsic viscosity is 1.2 to 2.0 dl/g, and the auto gear slides for automobiles have wear amount of 0.015 g or less in a base state.

The present invention provides door latch housing for automobiles manufactured by injection molding a polyketone composition comprising a linear alternating polyketone polymer comprising carbon monoxide and at least one kind of olefinically unsaturated hydrocarbon and having a residual amount of palladium catalyst of 5 to 50 ppm and a molecular weight distribution of 1.5 to 3.0, wherein the polyketone composition further comprises a silicon-based wear resistant agent, content of the silicon-based wear resistant agent is 2 to 20 weight% based on 100 weight% of the entire polyketone composition, a molar ratio of ethylene to propylene is 99:1 to 85:15, intrinsic viscosity of the linear alternating polyketone polymer is 1.2 to 2.0 dl/g, and the door latch housing for automobiles has impact strength of 10 $kJ/m^2$ or more and wear resistance in the base state of 0.015 g or less.

The present invention provides slide guides for automobiles manufactured by injection molding a polyketone composition comprising a linear alternating polyketone polymer comprising carbon monoxide and at least one kind of olefinically unsaturated hydrocarbon and having a residual amount of palladium catalyst of 5 to 50 ppm and a molecular weight distribution of 1.5 to 3.0, wherein the polyketone composition further comprises a silicon-based wear resistant agent, content of the silicon-based wear resistant agent is 2 to 20 weight% based on 100 weight% of the entire polyketone composition, a molar ratio of ethylene to propylene is 99:1 to 85:15, intrinsic viscosity of the linear alternating polyketone polymer is 1.2 to 2.0 dl/g, and the slide guides for automobiles has impact strength of 10 $kJ/m^2$ or more and wear resistance in the base state of 0.015 g or less.

The present invention provides switch shafts in heating, ventilation and air conditioning (HAVC) systems for automobiles manufactured by injection molding a polyketone composition comprising a linear alternating polyketone polymer comprising carbon monoxide and at least one kind of olefinically unsaturated hydrocarbon and having a residual amount of palladium catalyst of 5 to 50 ppm and a molecular weight distribution of 1.5 to 3.0, wherein the polyketone composition further comprises at least one kind selected from a group comprising a silicon-based wear resistant agent, a glass fiber and a polytetrafluoroethylene resin, a molar ratio of ethylene to propylene is 99:1 to 85:15, intrinsic viscosity of the linear alternating polyketone polymer is 1.2 to 2.0 dl/g, impact strength of 10 $kJ/m^2$ or more, and wear amount is 0.015 g or less in a base state.

The present invention provides actuator gears for automobiles manufactured by injection molding a polyketone composition comprising a linear alternating polyketone polymer comprising carbon monoxide and at least one kind of olefinically unsaturated hydrocarbon and having a residual amount of palladium catalyst of 5 to 50 ppm and a molecular weight distribution of 1.5 to 3.0, wherein at least one kind selected from a group comprising a silicon-based wear resistant agent, a glass fiber and a polytetrafluoroethylene resin is further included, a molar ratio of ethylene to propylene is 99:1 to 85:15, intrinsic viscosity of the linear alternating polyketone polymer is 1.2 to 2.0 dl/g, and the actuator gear for automobiles have impact strength of 10 $kJ/m^2$ or more and wear amount is 0.015 g or less in a base state.

The present invention provides trim mounting clips for automobiles manufactured by injection molding a polyketone composition comprising a linear alternating polyketone polymer comprising carbon monoxide and at least one kind of olefinically unsaturated hydrocarbon and having a residual amount of palladium catalyst of 5 to 50 ppm and a molecular weight distribution of 1.5 to 3.0, wherein the polyketone composition further comprises at least one kind selected from a group comprising a silicon-based wear resistant agent, a glass fiber and a polytetrafluoroethylene resin, a molar ratio of ethylene to propylene is 99:1 to 85:15, intrinsic viscosity of the linear alternating polyketone polymer is 1.2 to 2.0 dl/g, and the trim mounting clip for automobiles have impact strength of 10 $kJ/m^2$ or more and wear amount of 0.015 g or less in a base state.

The present invention provides cup holders for automobiles manufactured by injection molding a polyketone composition comprising a linear alternating polyketone polymer comprising carbon monoxide and at least one kind of olefinically unsaturated hydrocarbon and having a residual amount of palladium catalyst of 5 to 50 ppm and a molecular weight distribution of 1.5 to 3.0, wherein the polyketone composition further comprises at least one kind selected from a group comprising a silicon-based wear resistant agent, a glass fiber and a polytetrafluoroethylene resin, a molar ratio of ethylene to propylene is 99:1 to 85:15, intrinsic viscosity of the linear alternating polyketone polymer is 1.2 to 2.0 dl/g, and the cup holder for automobiles have impact strength of 10 $kJ/m^2$ or more and wear amount of 0.015 g or less in a base state.

The present invention provides roof racks for automobiles manufactured by injection molding a blend comprising 60 to 90 weight% of a linear alternating polyketone polymer comprising carbon monoxide and at least one kind of olefinically unsaturated hydrocarbon and having a residual amount of palladium catalyst of 5 to 50 ppm and a molecular weight distribution of 1.5 to 3.0, and 10 to 40 weight% of glass fiber, wherein a molar ratio of ethylene to propylene is 99:1 to 85:15, intrinsic viscosity of the linear alternating polyketone polymer is 1.2 to 2.0 dl/g, and the roof racks for

automobiles have impact strength of 10 $kJ/m^2$ and pencil hardness of 3H or more.

The present invention provides outside door handle for automobiles manufactured by injection molding a blend comprising a linear alternating polyketone polymer comprising carbon monoxide and at least one kind of olefinically unsaturated hydrocarbon and having a residual amount of palladium catalyst of 5 to 50 ppm and a molecular weight distribution of 1.5 to 3.0, a glass fiber and a mineral filler, wherein content of the glass fiber is 5 to 30 weight% based on total blend and content of the mineral filler is 10 to 20 weight%. In this case, the linear alternating polyketone polymer has a molar ratio of ethylene to propylene of 99:1 to 85:15 and intrinsic viscosity of 1.2 to 2.0 dl/g, the mineral filler is one selected from a group comprising talc, kaolin, mica, wollastonite, $TiO_2$-coated mica platelets, silica, alumina, borosilicates and oxides, and the outside door handle for automobiles have impact strength of 10 $kJ/m^2$ or more and dimensional change rate of 1.5% or less.

The present invention provides air intake garnish for automobiles manufactured by injection molding a blend comprising 60 to 85 weight% of a linear alternating polyketone polymer comprising carbon monoxide and at least one kind of olefinically unsaturated hydrocarbon and having a residual amount of palladium catalyst of 5 to 50 ppm and a molecular weight distribution of 1.5 to 3.0, and 15 to 40 weight% of glass fiber, wherein a molar ratio of ethylene to propylene is 99:1 to 85:15, intrinsic viscosity of the linear alternating polyketone polymer is 1.2 to 2.0 dl/g, and the air intake garnish for automobiles have impact strength of 20 $kJ/m^2$ or more and wear resistance Rmax of 1.0 or less.

The present invention provides medical transportation tray manufactured by injection molding a polyketone composition comprising a linear alternating polyketone which is polyketone copolymer comprising repeating units represented by general formula (1) and (2) described above, and has y/x of 0.1 to 0.3, wherein the polyketone composition further comprises at least one kind selected from a group comprising a silicon-based wear resistant agent, a glass fiber and a polytetrafluoroethylene resin, the medical transportation tray has wear amount of 0.015 g or less at a base state, the polyketone has intrinsic viscosity of 1.0 to 2.0 dl/g, and a ligand of a catalyst composition used in polyketone polymerization is ((2,2-dimethyl-1,3-dioxane-5,5-diyl)bis(methylene))bis(bis(2-methoxyphenyl)-phosphine).

The present invention provides medical pipette manufactured by injection molding a polyketone composition comprising a linear alternating polyketone which is polyketone copolymer comprising repeating units represented by general formula (1) and (2) described above, and has y/x of 0.1 to 0.3, wherein the polyketone composition further comprises at least one kind selected from a group comprising a silicon-based wear resistant agent, a glass fiber and a polytetrafluoroethylene resin, the medical pipette has flexural strength of 180 MPa or more and wear amount of 0.015 g or less at a base state, the polyketone has intrinsic viscosity of 1.0 to 2.0 dl/g, and a ligand of a catalyst composition used in polyketone polymerization is ((2,2-dimethyl-1,3-dioxane-5,5-diyl)bis(methylene))bis(bis(2-methoxyphenyl)phosphine).

The present invention provides refrigerator door closure manufactured by injection molding a polyketone composition comprising a linear alternating polyketone which is polyketone copolymer comprising repeating units represented by general formula (1) and (2) described above, and has y/x of 0.1 to 0.3, wherein the polyketone composition further comprises a polytetrafluoroethylene resin and thermoplastic polyurethane rein, the refrigerator door closure has wear amount of 0.015 g or less at a base state, the polyketone has intrinsic viscosity of 1.0 to 2.0 dl/g, and a ligand of a catalyst composition used in polyketone polymerization is ((2,2-dimethyl-1,3-dioxane-5,5-diyl)bis(methylene))bis(bis(2-methoxyphenyl)phosphine).

The present invention provides cellular phone polishing fixture manufactured by injection molding a polyketone composition comprising a linear alternating polyketone which is polyketone copolymer comprising repeating units represented by general formula (1) and (2) described above, and has y/x of 0.1 to 0.3, wherein the polyketone composition further comprises a polytetrafluoroethylene resin, the cellular phone polishing fixture has tensile strength of 30 MPa or more, the polyketone has intrinsic viscosity of 1.0 to 2.0 dl/g, and a ligand of a catalyst composition used in polyketone polymerization is ((2,2-dimethyl-1,3-dioxane-5,5-diyl)bis(methylene))bis(bis(2-methoxyphenyl)phosphine).

Also, the present invention provides a polyketone resin composition for ATM gears, which is comprising 100 parts by weight of polyketone resin which is polyketone copolymer comprising repeating units represented by following general formula (1) and (2), and has y/x of 0.1 to 0.3 and 0.1 to 15 parts by weight of silicone resin relative to 100 parts by weight of the polyketone resin.

$$-(CH_2CH_2-CO)x- \qquad (1)$$

$$-(CH_2CH(CH_3)-CO)y- \qquad (2)$$

(x and y are mole% of each of the general formula (1) and (2) in a polymer)

In this case, the silicone resin is in a form of powder and preferably has a diameter of 1 to 2 μm.
Also, the polyketone resin preferably has intrinsic viscosity of 1.0 to 2.0 dl/g.
Also, the polyketone resin preferably has a molecular weight distribution of 1.5 to 2.5.
Also, wear amount of the polyketone resin composition is 0.020 g or less in a speed of 50 rpm, a load of 150 N and a

wear distance of 3 km under JIS K7218 standard.

## Advantageous Effects

A polyketone resin composition manufactured by a method of the present invention has excellent wear resistance and impact resistance, thereby there is an effect of exhibiting properties suitable for use in gears, microwave containers, cams, helmet gears for electric welding, plastic boards, yarn guides, bedding cleaner cams, office supplies, window drums for automobiles, sun visor retainers, door frame inner covers for automobiles, safety belt jointers for automobiles, auto gear slides for automobiles, door latch housings for automobiles, slide guides for automobiles, switch shafts in heating, ventilation and air conditioning (HAVC) systems for automobiles, actuator gears for automobiles, trim mounting clips for automobiles, cup holders for automobiles, roof racks for automobiles, outside door handles for automobiles, air intake garnishes for automobiles, medical transportation trays, medical pipettes, refrigerator door closures, cellular phone polishing fixtures, ATM gears and etc.

## Brief Description of the Drawings

FIG. 1 is a schematic view of a thrust washer test device for evaluating wear resistance.
FIG. 2 shows a process for producing a plastic gear by blanking a plastic board of the present invention.

## Detailed Description of the Preferred Embodiments

Hereinafter, the present invention is described in more detail based on embodiments. The present invention may be modified in various different ways and may have various embodiments.
A polyketone of the present invention is a linear alternating structure and substantially contains carbon monoxide per one molecule of unsaturated hydrocarbon. Ethylenically unsaturated hydrocarbon suitable for use as precursor of the polyketone has up to 20, preferably up to 10 carbon atoms. Also, ethylenically unsaturated hydrocarbons is ethene, $\alpha$-olefin, aliphatic such as propene, 1-butene, isobutene, 1-hexene and 1-octene or an aryl aliphatic containing an aryl substituent on another aliphatic molecule, particularly containing an aryl substituent on an ethylenically unsaturated carbon atom. Examples of aryl aliphatic hydrocarbons in ethylenically unsaturated hydrocarbons include styrene, p-methyl styrene, p-ethyl styrene and m-isopropyl styrene. A polyketone polymer preferably used in the present invention is a copolymer of carbon monoxide and ethene or a second ethylenically unsaturated hydrocarbon having carbon monoxide, ethene and at least three carbon atoms, in particular terpolymer with $\alpha$-olefins such as propene.
When the polyketone terpolymer is used as a main polymer component of the blend of the present invention, there are at least two units comprising an ethylene part in each unit comprising a second hydrocarbon part in the terpolymer. There is preferably 10 to 100 of units comprising the second hydrocarbon part.
A preferred polymer ring of the polyketone polymer in the present invention is represented by following general formula (1).

[Formula 1]

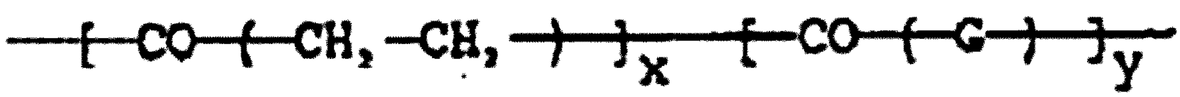

In the above formula (1), G is an ethylenically unsaturated hydrocarbon, particularly a part obtained from an ethylenically unsaturated hydrocarbon having at least three carbon atoms, and x:y is at least 2:1.
In the above, when y is 0, it can be represented by following general formula (2), and it becomes a copolymer and does not contain a second ethylenic unsaturated hydrocarbon.

[Formula 2] $-CO\{CH_2\ CH_2\}$ :-CO{G}

and
Units of formula (2) are randomly applied throughout polymer chain. A preferred y:x ratio is 0.01 to 0.5. A terminal root, or "cap", of a polymeric ring is determined by what material is present during preparation of the polymer,

and whether the polymer is to be purified or how the polymer is to be purified.

Number average molecular weight measured by gel penetration chromatography is preferably 100 to 200,000 and especially polyketone of 20,000 to 90,000 is preferable. Physical properties of polymer are set according to molecular amount, according to whether polymer is copolymer or terpolymer, and in case of terpolymer according to character of the second hydrocarbon. Ordinary melting point of polymer used in the present invention is 175°C to 300°C, and generally 210°C to 270°C. Limiting Viscosity Number (LVN) of polymer measured by standard viscosity measuring device and HFIP (Hexafluoroisopropylalcohol) in 60°C is 0.5 dl/g to 10 dl/g, and preferably 1.0 dl/g to 2.0 dl/g. In this case, if Limiting Viscosity Number is less than 0.5 dl/g, mechanical properties are declined, and if it is more than 10 dl/g, processability is declined.

Polyketone manufacturing method can use liquefied polymerization implemented under alcohol solvent through catalyst composition comprising carbon monoxide, olefin palladium compound, acid of 6 or less pKa, and bidentate ligand compound of phosphorus. Polymerization reaction temperature is preferably 50 to 100°C and reaction pressure is 40 to 60 bar. After polymerization of polymer, through purified process retrieving and remained catalyst composition is removed by solvent such as alcohol or acetone.

In this case, for palladium compound, acetic acid palladium is preferable and the amount is preferably 10-3 to 10-1 1 mole. Examples of acid with less than 6 pKa are trifluoroacetic acid, p-toluenesulfonic acid, sulfuric acid, sulfonic acid, and etc. In the present invention, trifluoroacetic acid is used and the amount is compared to palladium 6 to 20 equivalent weight. Also, bidentate ligand compound of phosphorus is preferably ((2,2-dimethyl-1,3-dioxane-5,5-diyl)bis(methylene))bis(bis(2-methoxyphenyl)phosphine), and the amount is compared to palladium 1 to 1.2 equivalent weight.

The following statement explains polymerization of polyketone in detail.

Carbon monoxide, ethylenically unsaturated compound, and one or more ollefinically unsaturated compound, three or more copolymer, especially monoxide-oriented repeating unit, and ethylenically unsaturated compound-oriented repeating unit, and propylenically unsaturated compound-oriented repeating unit are alternatively connected in structure of polyketone, the polyketone is excellent in mechanical properties and thermal properties and processibility, and having high abrasion resistance, chemical resistance, and gas barrier ability, therefore, useful in a variety of applications. High molecular weight of copolymer, terpolymer, or more copolymerized polyketone has higher processability and thermal properties, and regarded as useful engineering plastic with excellent economic efficiency. Especially, having high wear resistance used as component such as automobile gear, having high chemical resistance used as lining material of chemical transport pipe, and having high gas barrier ability used as light weight gasoline tank. In addition, in case of using ultra high molecular weight polyketone having 2 or more intrinsic viscosity in fiber, elongation of high magnification is possible, fiber having high strength and high elasticity modulus oriented in elongation direction, and it is suitable material in construction material and industrial material such as reinforcement of belt and rubber hose, and reinforcement of tire cord and concrete.

Polyketone manufacturing method under the presence of (a) Group 9, Group 10 or Group 11 transition metal compound, (b) organic metal complex catalyst comprising ligand having Group 15 element in polyketone manufacturing method by terpolymerization of carbon monoxide, ethylenically and propylenically unsaturated compound among liquid medium, the carbon monoxide, ethylene, and propylene liquefied polymerized in mixed solvent of alcohol(for example, methanol) and water, and produces linear terpolymer, and for the mixed solvent, mixture of methanol 100 part by weight and water 2 to 10 part by weight can be used. In mixed solvent if content of water is less than 2 part by weight, ketal is formed and thermal stability can be declined in process, and if content of water is more than 10 part by weight, mechanical properties of product can be declined.

A mixed solvent comprising 70-90 volume% of acetic acid and 10-30 volume% of water is used as a liquid medium, and benzophenone is added during polymerization.

In this case, for liquid medium, methanol, dichloromethane or nitromethane which were mainly used in conventional polyketone manufacturing are not used, and mixed solvent comprising acetic acid and water is used. By using mixed solvent of acetic acid and water for liquid medium in polykeone manufacture, manufacturing cost of polyketone is reduced and catalytic activity is enhanced. In addition, since a use of methanol or dichloromethane solvent forms a mechanism to induce a stopping reaction in polymerization step, a use of acetic acid or water other than methanol or dichloromethane in the solvent does not have an effect of stopping a catalytic activity stochastically, it plays a big role in improving a polymerization activity.

In case of using mixed solvent of acetic acid and water for liquid medium, if concentration of water is less than 10 vol%, it impacts less in catalyst activity, and if concentration is more than 10 vol%, catalyst activity is rapidly increased. Meanwhile, if concentration of water is more than 30 vol%, catalyst activity tends to decrease. Therefore, for liquid medium, mixed solvent comprising acetic acid of 70-90 vol% and water of 10-30 vol% is preferably used.

In this case, catalyst comprises periodic table (IUPAC Inorganic Chemistry Nomenclature revised edition, 1989) (a) Group 9, Group 10 or Group 11 transition metal compound, (b) ligand having Group 15 element.

Among Group 9, Group 10 or Group 11 transition metal compound (a), examples of Group 9 transition metal

compound are cobalt or ruthenium complex, carbon acid salt, phosphate, carbamate, sulfonate, and etc., specific examples are cobalt acetate, cobalt acetylacetate, ruthenium acetate, trifluororuthenium acetate, ruthenium acetylacetate, trifluoro-methane sulfonic acid, and etc.

Examples of Group 10 transition metal compound are nickel or palladium complex, carbon acid salt, phosphate, carbamate, sulfonate, and etc., specific examples are nickel acetate, nickel acetylacetate, palladium acetate, trifluoropalladium acetate, palladium acetylacetate, palladium chloride, bis(N,N-diethyl carbamate)bis(diethylamine)palladium, palladium sulfate, and etc.

Examples of Group 11 transition metal compound are copper or silver complex, carbon acid salt, phosphate, carbamate, sulfonate, and etc., specific examples are copper acetate, trifluoro-copper acetate, copper acetylacetate, silver acetate, trifluoro-silver acetate, silver acetylacetate, trifluoro-methane sulfonic silver, and etc.

Among them transition metal compound (a) preferable in cost and economically are nickel and copper compound, transition metal compound (a) preferable in the yield and molecular weight of polyketone is palladium compound, and in terms of enhancing catalyst activity and intrinsic viscosity using palladium acetate is most preferable.

Examples of ligand having Group 15 atom (b) are nitrogen ligand such as 2,2'-bipyridyl, 4,4'-dimethyl-2,2'-bipyridyl, 2,2'-bi-4-picoline, 2,2'-bikinoline and etc., and phosphorus ligand such as 1,2-bis(diphenylphosphino)ethane, 1,3-bis(diphenylphosphino)propane, 1,4-bis(diphenylphosphino)butane, 1,3-bis[di(2-methyl)phosphino]propane, 1,3-bis[di(2-isopropyl)phosphino]propane, 1,3-bis[di(2-methoxyphenyl)phosphino] propane, 1,3-bis[di-methoxy-4-sodium sulfonate-phenyl)-phosphino]propane, 1,2-bis(diphenylphosphino)cyclohexane,1,2-bis(diphenylphosphino)benzene, 1,2-bis[[diphenylphosphino]methyl]benzene, 1,2-bis[[di(2-methoxyphenyl)phosphino]methyl]benzene, 1,2-bis[[di(2-methoxy-4-sodium sulfonate-phenyl)phosphino]methyl]benzene, 1,1'-bis(diphenylphosphino)ferrocene, 2-hydroxy-1,3-bis[di(2-methoxyphenyl)phosphino] propane, 2,2-dimethyl-1,3-bis[di(2-methoxyphenyl)phosphino]propane, and etc.

Among them preferable ligand having Group 15 element (b) is phosphorous ligand having Group 15 element, especially in terms of the yield of polyketone preferable phosphorous ligand is 1,3-bis[di(2-methoxyphenyl)phosphino]propane, 1,2-bis[[di(2-methoxyphenyl)phosphino]methyl]benzene, in terms of molecular weight of polyketone preferably 2-hydroxy-1,3-bis[di(2-methoxyphenyl)phosphino]propane, in terms of safety and not needing organic solvent preferably water soluble 1,3-bis[di(2-methoxy-4-sodium sulfonate-phenyl)phosphino]propane, 1,2-bis[[di(2-methoxy-4-sodium sulfonate-phenyl)phosphino]methyl]benzene, in terms of economic aspect and easy synthesis preferably 1,3-bis(diphenylphosphino)propane, 1,4-bis(diphenylphosphino)butane. Preferable ligand having Group 15 element (b) is 1,3-bis[di(2-methoxyphenyl)phosphino]propane or 1,3-bis(diphenylphosphino)propane, and most preferably 1,3-bis[di(2-methoxyphenyl)phosphino]propane or ((2,2-dimethyl-1,3-dioxane-5,5-diyl)bis(methylene))bis(bis(2-methoxyphenyl)phosphine).

[formula 3]

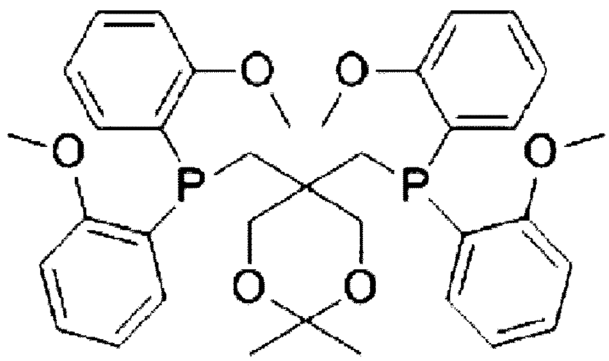

((2,2-dimethyl-1,3-dioxane-5,5-diyl)bis(methylene))bis(bis(2-methoxyphenyl)phosphine) of formula 3 shows the same activity expression as 3,3-bis-[bis-(2-methoxyphenyl)phosphonylmethyl]-1,5-dioxaspiro[5,5]undecane which is known as showing highest activity among polyketone polymerization catalyst introduced until now, and the structure is simpler and molecular weight is lower. Therefore, the present invention obtains highest activity as polymerization catalyst in relevant field, and providing novel polyketone polymerization catalyst with lower manufacturing cost and production cost. Manufacturing method of ligand for polyketone polymerization catalyst is as follows. Using bis(2-methoxyphenyl)phosphine, 5,5-bis(bromomethyl)-2,2-dimethyl-1,3-dioxane and sodium hydride (NaH) and obtaining ((2,2-dimethyl-1,3-dioxane-5,5-diyl)bis(methylene))bis(bis(2-methoxyphenyl)phosphine) features manufacturing method of ligand for polyketone polymerization catalyst. Manufacturing method of ligand for polyketone polymerization catalyst of the present invention is different from conventional synthesis method of 3,3-bis-[bis-(2-methoxyphenyl)phosphonylmethyl]-1,5-dioxaspiro[5,5]undecane as under safe environment not using lithium through simple process, ((2,2-dimethyl-1,3-dioxane-

5,5-diyl)bis(methylene))bis(bis(2-methoxyphenyl)phosphine) can be commercially mass-synthesized.

In a preferred embodiment, manufacturing method of ligand for polyketone polymerization catalyst of the present invention goes through (a) a step of inserting bis(2-methoxyphenyl)phosphine and dimethylsulfoxide (DMSO) to reaction vessel under nitrogen atmosphere and adding sodium hydride in room temperature and stirring; (b) a step of adding 5,5-bis(bromethyl)-2,2-dimethyl-1,3-dioxane and dimethylsulfoxide to obtained mixed solution and stirring and reacting; (c) a step of inserting methanol and stirring after completing reaction; (d) a step of inserting toluene and water after separating layer, cleaning oil layer with water and drying with anhydrous sodium sulfate, pressure filtering and pressure concentration; and (e) a step of recrystallizing residue under methanol and obtaining ((2,2-dimethyl-1,3-dioxane-5,5-diyl)bis(methylene))bis(bis(2-methoxyphenyl)phosphine).

The amount of Group 9, Group 10, or Group 11 transition metal compound (a) differs suitable value according to selected ethylenically and propylenically unsaturated compound type or different polymerization condition, so the range is not uniformly limited, but conventionally capacity of reaction zone is 0.01 to 100 mmol per liter, and preferably 0.01 to 10 mmol. Capacity of reaction zone refers to capacity of liquid phase of reactor. The amount of ligand (b) is not limited, but transition metal compound (a) per 1 mol is conventionally 0.1 to 3 mol and preferably 1 to 3 mol.

Moreover, adding benzophenone could be another feature in polymerization of the polyketone.

In the present invention, in polymerization of polyketone by adding benzophenone, it can achieve effects of enhancing intrinsic viscosity of polyketone. The (a) Group 9, Group 10 or Group 11 transition metal compound and benzophenone molar ratio is 1: 5 to 100, preferably 1: 40 to 60. If transition metal and benzophenone molar ratio is less than 1:5, effects of enhancement in intrinsic viscosity of polyketone is not satisfactory, and if transition metal and benzophenone molar ratio is more than 1:100, catalyst activity of produced polyketone tends to decrease therefore not preferable.

Examples of ethylenically unsaturated compound polymerized with carbon monoxide are ethylene, propylene, 1-butene, 1-hexene, 4-methyl-1-pentene, 1-octene, 1-decene, 1-dodecene, 1-tetradecene, 1-hexadecene, α-olefin such as vinyl cyclohexane; alkenyl aromatic compound such as styrene, α-methyl styrene; cyclopentane, norbornene, 5-methyl norbornene, tetracyclododecene, tricyclo dodecane, tricyclo undecene, pentacyclopentadecene, pentacyclohexadecene, 8-ethyltetracyclododecene; halogenation vinyl such as vinyl chloride; acrylic ester such as ethyl acrylate and methyl acrylate. Among them preferable ethylenically unsaturated compound is α-olefin, and more preferably α-olefin with carbon number of 2 to 4, and most preferably ethylene, in terms of producing terpolymer polyketone inserting 120 mol% of propylene.

In this case, adjusting carbon monoxide and ethylenically unsaturated compound inserting ratio to 1:12 (molar ratio), and adjusting propylene to 1-20 mol% to the total mixed gas are preferable. In case of manufacturing polyketone, it is general to make carbon monoxide and ethylenically unsaturated compound inserting ratio to 1:1, but in the present invention wherein acetate and water is used as mixed solvent for liquid medium and adding benzophenone in polymerization, in the case of making carbon monoxide and ethylenically unsaturated compound inserting ratio to 1:12 and adjusting propylene 1-20 mol% to the total mixed gas not only enhances processability but also simultaneously achieves enhancement in catalyst activity and intrinsic viscosity. In case of the inserting amount of propylene is less than 1 mol%, it can no attain effects of terpolymer to lower melting temperature, and in the case of the inserting amount of propylene is more than 20 mol%, problem occurs such as impediment in enhancing intrinsic viscosity and catalyst activity, so it is preferable to adjust inserting ratio to 1-20 mol%.

Moreover, in the polymerization process, for liquid medium, mixed solvent of acetate and water is used, and adding benzophenone in polymerization, and by inserting carbon monoxide and ethylenically unsaturated compound and one or more olefin-based unsaturated compound not only polyketone catalyst activity and intrinsic viscosity enhance but also it is possible to produce terpolymer polyketone having high intrinsic viscosity by setting polymerization time for 12 hours, and this is different from conventional technology which set polymerization time for at least 10 hours to enhance intrinsic viscosity.

Terpolymer of carbon monoxide, the ethylenically unsaturated compound and propylenically unsaturated compound occurs by organic metal complex catalyst comprising the Group 9, Group 10 or Group 11 transition metal compound (a), ligand having Group 15 element (b), and the catalyst is formed by contacting to the 2 components. Contacting method can be selected arbitrary. In other words, among suitable solvent, solution of mixed 2 components in advance can be used, or each of 2 components can separately be provided to polymerization system and contact in polymerization system.

In the present invention, to improve a processability and physical properties of the polymer, conventionally known additives such as an antioxidant, a stabilizer, a filler, a refractory material, a releasing agent, a coloring agent and other materials may be additionally added.

Examples of a polymerization method include a solution polymerization method using a liquid medium, a suspension polymerization method, a vapor phase polymerization method in which a small amount of a polymer is impregnated with a high concentration catalyst solution, and etc. The polymerization may be either batch or continuous. The reactor used for the polymerization may be a known reactor as it is, or may be used by processing. Polymerization

temperature is not particularly limited, and is generally 40 to 180°C, preferably 50 to 120°C. Polymerization pressure is not particularly limited, but is generally from atmospheric pressure to 20 MPa, preferably from 4 to 15 MPa.

Number average molecular weight measured by gel penetration chromatography is preferably 100 to 200,000 especially 20,000 to 90,000 of polyketone polymer. Physical properties of polymer according to molecular weight, according to whether polymer is copolymer or terpolymer, or in case of terpolymer, it is determined according to properties of the second hydrocarbon portion. Polymer used in the present invention has conventional melting point of 175°C to 300°C, and generally 210°C to 270°C. Limiting Viscosity Number (LVN) of polymer measured by using standard viscosity measuring device with HFIP (Hexafluoroisopropylalcohol) in 60°C is 0.5 dl/g to 10 dl/g, and preferably 1 dl/g to 2 dl/g. In case of limiting viscosity number of polymer is less than 0.5, polyketone mechanical properties and chemical properties decline, and in case of limiting viscosity number of polymer is more than 10, moldability declines.

Meanwhile, polyketone molecular weight distribution is preferably 1.5 to 2.5, more preferably 1.8 to 2.2. If molecular weight distribution is less than 1.5, polymerization transference number declines, and if molecular weight distribution is more than 2.5, moldability declines. In order to adjust the molecular weight distribution adjusting proportionately according to palladium catalyst amount and polymerization temperature is possible. In other words, if palladium catalyst amount increases, or polymerization temperature is more than 100°C, molecular amount distribution increases.

Linear alternative polyketone is formed according to polymerization method stated above.

The wear resistant agent of the present invention is described below.

The polyketone molded component excellent in wear resistance according to the present invention is comprising a polyketone excellent in heat resistance, chemical resistance, fuel permeation resistance, and impact resistance; and a wear resistant agent of a specific material which reduces wear, thereby it is possible to remarkably improve the wear resistance of the polyketone because of the wear resistant agent of the specific material dispersed in the polyketone, especially the wear resistant agent of the specific material dispersed on a surface.

Also, the wear resistant agents of the specific materials added in order to improve friction resistance and wear resistance of polyketone molded components include at least one or more kinds selected from a group comprising silicon, polytetrafluorethylene (PTFE), calcium carbonate ($CaCO_3$), maleic acid, Molybdenum (Mo), glass fiber, magnesium stearate, and etc., more preferably silicon, polytetrafluoroethylene or magnesium stearate, and most preferably, silicon or polytetrafluoroethylene.

More specifically, the silicon may be provided in a form of a gum (product of a liquid type silicon produced by POM master batch) or powder, and after added to the polyketone, it might be dispersed in the polyketone through blending or etc.

The polytetrafluorethylene(PTFE) may be provided in a powder form, and after added to the polyketone, it might be dispersed in the polyketone through blending or etc.

The calcium carbonate ($CaCO_3$) may be provided in a powder form of a product used as a lubricant for engineering plastics, and after added to the polyketone, it might be dispersed in the polyketone through blending or etc.

The maleic acid may be provided in a form of an MA-g-ethylene copolymer substituted with maleic acid by graft polymerization of maleic acid to an ethylene copolymer, and after added to the polyketone, it might be dispersed in the polyketone through blending or etc.

The molybdenum (Mo) may be provided in a powder form of a metallized product family of molybdenum disulfide ($MoS_2$), and after added to the polyketone, it might be dispersed in the polyketone through blending or etc.

The glass fiber preferably has a particle diameter of 10 to 13 $\mu$m. If the particle diameter of the glass fiber is less than 10 $\mu$m, a shape of the glass fiber may be changed and mechanical properties may decline.

The magnesium stearate may be provided in a powder form of a lubricant product, and after added to the polyketone, it might be dispersed in the polyketone through blending or etc.

In the polyketone molded component having excellent wear resistance according to the present invention, a polyketone content based on total composition weight is preferably 80 to 99.9 weight% and wear resistant agent of the specific material is preferably 0.1 to 20 weight%. If the content of the polyketone is less than 80 weight%, mechanical properties, thermal stability and fluidity of the molded component may be reduced. If the content is 99.9 weight% or more, the content of the wear resistant agent of the specific material is relatively small, thereby an effect of improving the wear resistance of the polyketone molded component may not enough.

Also, if content of the wear resistant agent of the specific material is less than 0.1 weight%, an effect of improving the wear resistance of the molded component may be insignificant. If the content is more than 20 weight%, the mechanical properties and thermal stability of the molded component decline, exfoliation may occur on a surface of the molded component.

According to an exemplary embodiment of the present invention, a silicone resin is 0.1 to 15 parts by weight of a silicone resin relative to 100 parts by weight of the polyketone resin. When the content of the silicone resin is less than 0.1, wear resistance is not enough when used as a gear, and when the content is more than 15, mechanical properties inherent to polyketone decline.

The silicone resin used in the present invention is in a powder form and preferably has a diameter of 1 to 2

μm, and if it is out of the range, blending with the polyketone is not smooth.

Also, the polyketone molded component of the present invention can be used in industrial fields requiring wear resistance, and is preferably applicable to wear parts in OA, ATM gear, electric/electronic gear, city gas meter gear or laser printer toner gear, however the present invention is not limited thereto.

Polyketone polymer of the present invention is polyketone copolymer of y/x 0.03 to 0.3, x and y in the following formula indicate each mol% in polymer.

$-(CH_2CH_2-CO)x-$ formula (1)

$-(CH_2CH(CH_3)-CO)y-$ formula (2)

Copolymer comprising repeating unit shown as formula (1) and (2) of the present invention, y/x is preferably 0.03 to 0.3. If the y/x is less than 0.03, there are limits such as melting property and processability decline, and if the y/x is more than 0.3, mechanical properties decline. Moreover, y/x is more preferably 0.03 to 0.1. Also, melting point of polymer can be adjusted by adjusting ratio of ethylene and propylene of polyketone polymer. For example, in case of molar ratio of ethylene:propylene:carbon monoxide is adjusted to 46:4:50, melting point is approximately 220°C, and if molar ratio is adjusted to 47.3:2.7:50, melting point is adjusted to 235°C.

The polyketone of the present invention can be used for industrial parts such as gears, microwave containers, cams, helmet gears for electric welding, plastic boards, yarn guides, bedding cleaner cams, office supplies and etc. Particularly when it is intended to be used as an industrial part, a wear resistant agent may be added to enhance wear resistance and impact resistance of the polyketone. The wear resistant agent is preferably selected from a group comprising ricons, polytetrafluoroethylene, calcium carbonate, maleic acid, molybdenum, glass fibers and magnesium stearate.

The wear resistance agent is preferably 0.1 to 20 parts by weight based on 100 parts by weight of the polyketone polymer based on a weight ratio. If the content of the wear resistant agent is less than 0.1, an effect of improving wear resistance and impact resistance is insignificant. If it is more than 20 parts by weight, mechanical properties, thermal stability and fluidity inherent to the polyketone may decline or exfoliation may occur on a surface.

The present invention also provides a window drum for automobiles, a sun visor retainer for automobiles, a safety belt jointer for automobiles, a auto gear slide for automobiles, a door latch housing for automobiles, a slide guide for automobiles, a switch shaft in heating, ventilation and air conditioning(HAVC) systems for automobiles, an actuator gear for automobiles, a trim mounting clip for automobiles, a cup holder for automobiles, a roof rack for an automobiles, and an air intake garnish for automobiles manufactured by injection molding a polyketone composition comprising a linear alternating polyketone polymer comprising carbon monoxide and at least one kind of olefinically unsaturated hydrocarbon and having a residual amount of palladium catalyst of 5 to 50 ppm and a molecular weight distribution of 1.5 to 3.0.

In this case, the polyketone composition preferably further comprises at least one kind selected from a group comprising a silicon-based wear resistant agent, glass fiber, and polytetrafluoroethylene resin. Here, the silicon-based wear resistant agent is preferably 2 to 20 weight% based on 100 weight% of total polyketone composition.

Also, the present invention provides a method for being manufactured by injection molding a blend comprising 60 to 95 weight% of a linear alternating polyketone polymer comprising carbon monoxide and at least one kind of olefinically unsaturated hydrocarbon and having a residual amount of palladium catalyst of 5 to 50 ppm and a molecular weight distribution of 1.5 to 3.0, and 40 weight% of glass fiber.

In addition, in case of an outside door handle for automobiles, the glass fiber is 5 to 30 weight% based on total blend, and the mineral filler is 10 to 20 weight%.

In this case, the glass fibers preferably have a particle diameter of 10 to 15 μm, but the present invention is not limited thereto. If the particle diameter of the glass fiber is less than 10 μm, a shape of the glass fiber may be changed and mechanical properties may decline.

Also, a composition ratio of the glass fiber to entire composition is preferably 5 to 40 weight%. If the composition ratio of the glass fiber is less than 5 weight%, mechanical stiffness may decline. If it is more than 40 weight%, extrusion and injection processability may decline.

Also, the mineral filler is one selected from a group comprising Talc, Kaolin, Mica, wollastonite, $TiO_2$-coated mica platelets, silica, alumina, borosilicates, and oxides.

In this case, content of the mineral filler is 10 to 20 weight%. If the content of the mineral filler added is less than 10 weight%, dimensional stability declines. If it is more than 20 weight%, injection moldability declines.

Hereinafter, a manufacturing method for manufacturing a window drum for automobiles, a sun visor retainer for automobiles, a door frame inner cover for automobiles, a safety belt jointer for automobiles, an auto gear slide for automobiles, a door latch housing for automobiles, a slide guide for automobiles, a switch shaft in heating, ventilation and air conditioning (HAVC) systems for automobiles, an air intake opening garnish for an automobile, the HAVC switch

and cam shaft, an actuator gear for automobiles, a trim mounting clip for automobiles, a cup holder for automobiles, a roof rack for automobiles, an outside door handle for automobiles, and an air intake garnish for automobiles according to the present invention is described.

The manufacturing method for manufacturing a window drum for automobiles, a sun visor retainer for automobiles, a door frame inner cover for automobiles, a safety belt jointer for automobiles, an auto gear slide for automobiles, a door latch housing for automobiles, a slide guide for automobiles, a switch shaft in heating, ventilation and air conditioning (HAVC) systems for automobiles, an air intake opening garnish for an automobile, the HAVC switch and cam shaft, an actuator gear for automobiles, a trim mounting clip for automobiles, a cup holder for automobiles, a roof rack for automobiles, an outside door handle for automobiles, and an air intake garnish for automobiles according to the present invention is comprising a step of preparing a catalyst composition comprising a palladium compound, an acid having a pKa value of 6 or less, and a phosphorus compound; a step of preparing a mixed solvent(polymerization solvent) comprising an alcohol (e.g., methanol) and water; a step of conducting a polymerization in a presence of the catalyst composition and the mixed solvent to prepare a linear terpolymer of carbon monoxide, ethylene and propylene; a step of removing a remaining catalyst composition from the linear terpolymer with a solvent (e.g., alcohol and acetone) to obtain a polyketone polymer; and a step of injection molding the polyketone composition including the polyketone polymer and a silicon-based wear resistant agent, but the present invention is not limited thereto.

In this case, based on 100 weight% of total polyketone composition, the silicon-based wear resistant agent is preferably 2 to 20 weight%, but is not limited thereto.

For the palladium compound comprising the catalyst composition, palladium acetate can be used, and the amount is preferably $10^{-3}$ to $10^{-1}$ mol, but it is not limited thereto.

In addition, for acid having 6 or less pKa value comprising the catalyst composition is in group of trifluoro acetate, p-toluenesulfonic acid, sulfuric acid, and sulfonic acid, one or more kind selected, preferably trifluoro acetate can be used, and the amount is preferably 6 to 20 mol equivalent to palladium compound.

Moreover, bidentate ligand compound of the phosphorous comprising the catalyst composition is any one selected in group of 1,3-bis[diphenylphosphino]propane, 1,3-bis[di(2-methoxyphenylphosphino)]propane, 1,3-bis[bis[anisyl]phosphinomethyl-1,5-dioxaspiro[5,5]undecane, and ((2,2-dimetyl-1,3-dioxane-5,5-diyl)bis(methylene))bis(bis(2-methoxyphenyl)phosphine), and the amount is preferably 1 to 20 (mol) equivalent to palladium compound.

The carbon monoxide, ethylene and propylene liquefied polymerized in mixed solvent of alcohol (for example, methanol) and water, and produces linear terpolymer, for the mixed solvent, methanol 100 parts by weight and water 2 to 10 parts by weight can be used. If content of water in mixed solvent is less than 2 parts weight, ketal is formed and thermal stability can be declined in process, and if it is more than 10 parts by weight, mechanical properties of product can be declined.

Also, in the polymerization, reaction temperature is preferably 50 to 100°C and reaction pressure is preferably 40 to 60 bar. Produced polymer is retrieved through filtering and cleaning process after polymerization, and remained catalyst composition is removed by solvent such as alcohol or acetone.

In the present invention, it can be manufactured by melt-kneading and extruding inserting a polyketone composition comprising the obtained polyketone polymer; and one or more kinds selected from a group comprising a silicon-based wear resistant agent, glass fiber and polytetrafluoroethylene resin into an extruder using a biaxial screw.

In this case, extrusion temperature is preferably 230 to 260°C, and a screw rotating speed is preferably in a range of 100 to 300 rpm. If the extrusion temperature is less than 230°C, kneading may not occur properly, and if the extrusion temperature is more than 260°C, a problem relating to heat resistance of the resin may occur. If the screw rotating speed is less than 100 rpm, kneading may not be smoothly performed, and if the screw rotating speed is more than 300 rpm, mechanical properties may decline. By manufacturing the blend by the method as above and extrusion molding or injection molding it, a window drum for automobiles, a sun visor retainer for automobiles, a door frame inner cover for automobiles, a safety belt jointer for automobiles, an auto gear slide for automobiles, a door latch housing for automobiles, a slide guide for automobiles, a switch shaft in heating, ventilation and air conditioning (HAVC) systems for automobiles, an air intake opening garnish for an automobile, the HAVC switch and cam shaft, an actuator gear for automobiles, a trim mounting clip for automobiles, a cup holder for automobiles, a roof rack for automobiles, an outside door handle for automobiles, and an air intake garnish for automobiles can be manufactured.

The air intake garnish for automobiles according to the present invention exhibits excellent wear resistance and dimensional stability. Specifically, the air intake garnish for automobiles has an impact strength of 20 $kJ/m^2$ or more and a wear resistance of 1.0 $mm^3/kg/km$ or less in a base state.

In addition, the polyketone medical transportation tray, medical pipette, and refrigerator door closures of the present invention are made of polyketone. In this case, the polyketone medical transportation tray of the present invention may be made of a polyketone composition comprising one or more kinds selected from a group comprising a polyketone; a silicon-based wear resistant agent, glass fiber, and polytetrafluoroethylene resin, but is not limited thereto.

Also, the polyketone cellular phone polishing fixture of the present invention may comprise a polyketone composition comprising a polyketone and a polytetrafluoroethylene resin, but is not limited thereto.

In a method of polymerization of polyketone, wherein monomer units are alternating, thereby the polymer is composed of units of general formula -(CO)-A'-(Here, A' represents a monomer unit derived from the applied monomer A), a high molecular weight linear polymer of one or more olefinically unsaturated compound(simply referred to as A) and carbon monoxide can be manufactured by contacting with a solution of a palladium-comprising catalyst composition in a diluent in which the polymer does not dissolve. During polymerization process, the polymer is obtained in a form of a suspension in the diluent. Polymer manufacture is performed primarily batchwise.

The batchwise manufacture of the polymer is usually carried out by inserting a catalyst into a reactor containing the diluent and the monomer and having a desired temperature and pressure. As the polymerization progresses, the pressure drops, as a concentration of the polymer in the diluent increases, and viscosity of the suspension increases. The polymerization is continued until the viscosity of the suspension reaches a high enough value to cause difficulty associated with heat removal. During batchwise polymer manufacture, if desired, by adding monomers to the reactor during the polymerization, it is possible to maintain the temperature as well as the pressure constant.

Hereinafter, a manufacturing method for manufacturing the polyketone medical transportation tray, the medical pipette, the refrigerator door closure, and the cellular phone polishing fixture is described. A manufacturing method for manufacturing a polyketone medical transportation tray, a medical pipette, a refrigerator door closure and a cellular phone polishing fixture of the present invention comprises: a step of preparing a catalyst composition comprising a palladium compound, an acid having a pKa value of 6 or less, and a bidentate compound of phosphorus; a step of preparing a mixed solvent(polymerization solvent) of acetic acid and water; a step of conducting a polymerization in presence of the catalyst composition and the mixed solvent to prepare a linear terpolymer of carbon monoxide, ethylene and propylene; a step of removing the remaining catalyst composition from the prepared linear terpolymer using a solvent (e.g., alcohol and acetone) to obtain a polyketone resin; and a step of mixing and extruding the polyketone resin.

For the palladium compound comprising the catalyst composition, palladium acetate can be used, and the amount is preferably $10^{-3}$ to $10^{-1}$, but it is not limited thereto.

In addition, for acid having 6 or less pKa value comprising the catalyst composition is in group of trifluoro acetate, p-toluenesulfonic acid, sulfuric acid, and sulfonic acid, one or more kind selected, preferably trifluoro acetate can be used, and the amount is preferably 6 to 20 mol equivalent to palladium compound.

The bidentate compound of phosphorus comprising the catalyst composition is preferably ((2,2-dimethyl-1,3-dioxane-5,5-diyl)bis(methylene))bis(bis(2-methoxyphenyl)phosphine), and amount thereof is preferably 1 to 1.2 (mol) relative to the palladium compound.

The carbon monoxide, ethylene and propylene liquefied polymerized in mixed solvent of alcohol (for example, methanol) and water, and produces linear terpolymer, for the mixed solvent, acetic acid 100 parts by weight and water 2 to 30 parts by weight can be used. If content of water in mixed solvent is less than 2 parts weight, ketal is formed and thermal stability can be declined in process, and if it is more than 30 parts by weight, mechanical properties of product can be declined.

Also, in the polymerization, reaction temperature is preferably 50 to 100°C and reaction pressure is preferably 40 to 60 bar. Produced polymer is retrieved through filtering and cleaning process after polymerization, and remained catalyst composition is removed by solvent such as alcohol or acetone. In the present invention, the obtained polyketone resin is extruded by an extruder to finally obtain a blend composition. The blend is produced by putting into an extruder using a biaxial screw, melt-kneading and extruding.

In this case, extrusion temperature is preferably 230 to 260°C, and screw rotating speed is preferably in a range of 100 to 300 rpm. If the extrusion temperature is less than 230°C, kneading may not occur properly, and if the extrusion temperature is more than 260°C, a problem relating to heat resistance of the resin may occur.

The polyketone medical transportation tray, the medical pipette, the refrigerator door closure, and the cellular phone polishing fixture can be manufactured by manufacturing the resin and injecting it by the above-described method.

The polyketone medical transportation tray, the medical pipette, the refrigerator door closure, and the cellular phone polishing fixture manufactured according to the present invention were found to have excellent wear resistance.

Hereinafter, the present invention is described in more detail with reference to the following examples. However, the following examples are illustrative of the present invention and are not to be construed as limiting the scope of the present invention. The present invention is illustrated in detail by following non-limiting examples.

Example 1

Linear alternating polyketone terpolymers comprising carbon monoxide and ethylene and propene are prepared under presence of catalyst composition formed from palladium acetate, trifluoroacetic acid and ((2,2-dimethyl-1,3-dioxane-5,5-diyl)bis(methylene)bis(2-methoxyphenyl) phosphine). In the prepared polyketone terpolymer, a molar ratio of ethylene to propene was 46:4. Also, a melting point of the polyketone terpolymer was 220°C, LVN measured at 25°C by HFIP (hexa-fluoroisopropanol) was 1.4 dl/g, and MI (Melt Index) was 48 g/10 min. 90 weight% of the polyketone terpolymer prepared above and 10 weight% of silicone were molded into pellets on an extruder using a biaxial screw

having a diameter of 2.5 cm and L/D=32, which was operated at 250 rpm, and then injection molded to produce a specimen for a wear part in OA.

Example 2

The same as Example 1 except that the polyketone composition was composed of 90 weight% of polyketone and 10 weight% of polytetrafluoroethylene.

Example 3

The same as Example 1 except that the polyketone composition was composed of 90 weight% of polyketone and 10 weight% of glass fiber.

Example 4

The same as Example 1 except that the polyketone composition was composed of 97 weight% of polyketone and 3 weight% of silicone.

Comparative Example 1

The same as Example 1 except that 100 weight% of polyketone was used.

Comparative Example 2

The same as Example 1 except that 100 weight% of polyoxymethylene was used.

Comparative Example 3

The same as Example 1 except that 100 weight% of nylon 66 was used.

Comparative Example 4

The same as Example 1 except that 90 weight% of polyoxymethylene and 10 weight% of silicone were used.

Properties Evaluation

wear amount and wear length of the specimens prepared in Examples and Comparative Examples were measured under experimental conditions of a speed of 50 rpm, a load of 150 N and a wear distance of 3 km, and results are shown in Table 1.

[Table 1]

| | Composition ratio | Wear amount (g) | Wear length (mm) |
|---|---|---|---|
| Example 1 | polyketone 90%/silicone 10% | 0.0031 | 0.012 |
| Example 2 | polyketone 90%/ polytetrafluoroethylene 10% | 0.0033 | 0.013 |
| Example 3 | polyketone 90%/glass fiber 10% | 0.0040 | 0.015 |
| Example 4 | polyketone 97%/silicone 3% | 0.0027 | 0.011 |
| Comparative Example 1 | polyketone 100% | 0.0150 | 0.060 |
| Comparative Example 2 | polyoxymethylene 100% | 0.0610 | 0.630 |
| Comparative Example 3 | polyoxymethylene 90%/silicone 10% | 0.0530 | 0.615 |
| Comparative Example 4 | Nylon 66 100% | 0.0070 | 0.025 |

As shown in Table 1, the polyketone molded components produced by blending the polyketone with wear resistant agent such as silicone, polytetrafluoroethylene, glass fiber and silicone exhibited a wear amount of 0.005 g or

less under same test conditions as those of the comparative examples, thereby it is suitable for wear parts in OA, ATM gear, electric/electronic gear, city gas meter gear and laser printer toner gear which are required to have wear resistance.

Example 5

Linear alternating polyketone terpolymers comprising carbon monoxide and ethylene and propene are prepared under presence of catalyst composition formed from palladium acetate, trifluoroacetic acid and ((2,2-dimethyl-1,3-dioxane-5,5-diyl)bis(methylene)bis(2-methoxyphenyl) phosphine). In the above, content of trifluoroacetic acid with respect to palladium is 11 times the molar ratio, and two stages of the first stage at a polymerization temperature of 78°C and the second stage at 84°C are carried out. In the prepared polyketone terpolymer, a molar ratio of ethylene to propene was 46:4. Also, a melting point of the polyketone terpolymer was 220°C, LVN measured at 25°C by HFIP (hexa-fluoroisopropanol) was 1.4 dl/g, MI (Melt Index) was 60 g/10 min, MWD was 2.0, and a residual amount of palladium catalyst was 5 ppm. The polyketone terpolymer prepared above was molded into pellets on an extruder using a biaxial screw having a diameter of 40 mm and L/D=32, which was operated at 250 rpm, and then injection molded to produce a specimen for a microwave container.

Example 6

Linear alternating polyketone terpolymers comprising carbon monoxide and ethylene and propene are prepared under presence of catalyst composition formed from palladium acetate, trifluoroacetic acid and ((2,2-dimethyl-1,3-dioxane-5,5-diyl)bis(methylene)bis(2-methoxyphenyl) phosphine). In the above, content of trifluoroacetic acid with respect to palladium is 10 times the molar ratio, and two stages of the first stage at a polymerization temperature of 74°C and the second stage at 84°C are carried out. In the prepared polyketone terpolymer, a molar ratio of ethylene to propene was 46:4. Also, a melting point of the polyketone terpolymer was 220°C, LVN measured at 25°C by HFIP (hexa-fluoroisopropanol) was 1.6 dl/g, MI (Melt Index) was 60 g/10 min, MWD was 2.0, and a residual amount of palladium catalyst was 5 ppm. The polyketone terpolymer prepared above was molded into pellets on an extruder using a biaxial screw having a diameter of 40 mm and L/D=32, which was operated at 250 rpm, and then injection molded to produce a specimen for a microwave container.

Example 7

Linear alternating polyketone terpolymers comprising carbon monoxide and ethylene and propene are prepared under presence of catalyst composition formed from palladium acetate, trifluoroacetic acid and ((2,2-dimethyl-1,3-dioxane-5,5-diyl)bis(methylene)bis(2-methoxyphenyl) phosphine). In the above, content of trifluoroacetic acid with respect to palladium is 9 times the molar ratio, and two stages of the first stage at a polymerization temperature of 72°C and the second stage at 78°C are carried out. In the prepared polyketone terpolymer, a molar ratio of ethylene to propene was 46:4. Also, a melting point of the polyketone terpolymer was 220°C, LVN measured at 25°C by HFIP (hexa-fluoroisopropanol) was 2.0 dl/g, MI (Melt Index) was 60 g/10 min, MWD was 2.0, and a residual amount of palladium catalyst was 7 ppm. The polyketone terpolymer prepared above was molded into pellets on an extruder using a biaxial screw having a diameter of 40 mm and L/D=32, which was operated at 250 rpm, and then injection molded to produce a specimen for a microwave container.

Comparative Example 5

A specimen of a microwave container was produced with polytetrafluoroethylene resin.

Properties Evaluation

A coefficient of kinetic friction, wear property and impact strength of the specimens respectively prepared in Examples 5 to 7 and Comparative Example 5 were evaluated by following method. Results are shown in Table 2.

The coefficient of kinetic friction is a measure of a degree of kinetic friction, it means that the greater the coefficient of kinetic friction, the greater the frictional force, and the smaller the value, the smaller the frictional force.

The wear property is a value indicating a degree of wear. The larger the wear property, the smaller the wear resistance because wear occurs more easily. Meanwhile, the smaller the wear property, the greater the wear resistance because wear does not occur easily. A wear test was carried out by a pin-on-disk type under a load of 1 kg, a linear velocity of 7 Hz and a test time of 30 minutes.

[Table 2]

| Item | Example 5 | Example 6 | Example 7 | Comparative Example 5 |
|---|---|---|---|---|
| Coefficient of kinetic Friction | 0.10 | 0.15 | 0.12 | 0.40 |
| Wear property (Rmax) | 0.60 | 0.65 | 0.70 | 8.10 |

As can be seen from Table 2, impact strength of the example was improved compared to comparative example, the coefficient of kinetic friction and the wear property were small, thereby the frictional force was small and the wear resistance was improved.

Example 8

Under presence of catalyst composition formed from palladium acetate, trifluoroacetic acid and ((2,2-dimethyl-1,3-dioxane-5,5-diyl)bis(methylene)bis(2-methoxyphenyl) phosphine), linear alternating polyketone terpolymers comprising carbon monoxide and ethylene and propene are prepared. In the above, content of trifluoroacetic acid with respect to palladium is 11 times the molar ratio, and two stages of the first stage at a polymerization temperature of 80°C and the second stage at 84°C are carried out. In the prepared polyketone terpolymer, a molar ratio of ethylene to propene was 46:4. Also, a melting point of the polyketone terpolymer was 220°C, LVN measured at 25°C by HFIP (hexa-fluoroisopropanol) was 1.2 dl/g, MI (Melt Index) was 60 g/10 min, MWD was 2.0.

The polyketone terpolymer prepared above was extruded through melt-kneading on an extruder using a biaxial screw having L/D32 and D 40 at 240°C, which was operated at 250 rpm.

Example 9

Under presence of catalyst composition formed from palladium acetate, trifluoroacetic acid and ((2,2-dimethyl-1,3-dioxane-5,5-diyl)bis(methylene)bis(2-methoxyphenyl) phosphine), linear alternating polyketone terpolymers comprising carbon monoxide and ethylene and propene are prepared. In the above, content of trifluoroacetic acid with respect to palladium is 10 times the molar ratio, and two stages of the first stage at a polymerization temperature of 78°C and the second stage at 84°C are carried out. In the prepared polyketone terpolymer, a molar ratio of ethylene to propene was 46:4. Also, a melting point of the polyketone terpolymer was 220°C, LVN measured at 25°C by HFIP (hexa-fluoroisopropanol) was 1.4dl/g, MI (Melt Index) was 60 g/10 min, MWD was 2.0.

The polyketone terpolymer prepared above was extruded through melt-kneading on an extruder using a biaxial screw having L/D32 and D 40 at 240°C, which was operated at 250 rpm.

Example 10

Under presence of catalyst composition formed from palladium acetate, trifluoroacetic acid and ((2,2-dimethyl-1,3-dioxane-5,5-diyl)bis(methylene)bis(2-methoxyphenyl) phosphine), linear alternating polyketone terpolymers comprising carbon monoxide and ethylene and propene are prepared. In the above, content of trifluoroacetic acid with respect to palladium is 9 times the molar ratio, and two stages of the first stage at a polymerization temperature of 74°C and the second stage at 84°C are carried out. In the prepared polyketone terpolymer, a molar ratio of ethylene to propene was 46:4. Also, a melting point of the polyketone terpolymer was 220°C, LVN measured at 25°C by HFIP (hexa-fluoroisopropanol) was 1.6 dl/g, MI (Melt Index) was 60 g/10 min, MWD was 2.0.

The polyketone terpolymer prepared above was extruded through melt-kneading on an extruder using a biaxial screw having L/D32 and D 40 at 240°C, which was operated at 250 rpm.

Example 11

Under presence of catalyst composition formed from palladium acetate, trifluoroacetic acid and ((2,2-dimethyl-1,3-dioxane-5,5-diyl)bis(methylene)bis(2-methoxyphenyl)phosphine), linear alternating polyketone terpolymers comprising carbon monoxide and ethylene and propene are prepared. In the above, content of trifluoroacetic acid with respect to palladium is 10 times the molar ratio, and two stages of the first stage at a polymerization temperature of 78°C and the second stage at 84°C are carried out. In the prepared polyketone terpolymer, a molar ratio of ethylene to propene was 46:4. Also, a melting point of the polyketone terpolymer was 220°C, LVN measured at 25°C by HFIP (hexa-fluoroisopropanol) was 1.4 dl/g, MI (Melt Index) was 60 g/10 min, MWD was 1.8.

The polyketone terpolymer prepared above was extruded through melt-kneading on an extruder using a biaxial

screw having L/D32 and D 40 at 240°C, which was operated at 250 rpm.

Example 12

Under presence of catalyst composition formed from palladium acetate, trifluoroacetic acid and ((2,2-dimethyl-1,3-dioxane-5,5-diyl)bis(methylene)bis(2-methoxyphenyl) phosphine), linear alternating polyketone terpolymers comprising carbon monoxide and ethylene and propene are prepared. In the above, content of trifluoroacetic acid with respect to palladium is 10 times the molar ratio, and two stages of the first stage at a polymerization temperature of 78°C and the second stage at 84°C are carried out. In the prepared polyketone terpolymer, a molar ratio of ethylene to propene was 46:4. Also, a melting point of the polyketone terpolymer was 220°C, LVN measured at 25°C by HFIP (hexa-fluoroisopropanol) was 1.4 dl/g, MI (Melt Index) was 60 g/10 min, MWD was 2.2.
The polyketone terpolymer prepared above was extruded through melt-kneading on an extruder using a biaxial screw having L/D32 and D 40 at 240°C, which was operated at 250 rpm.

Comparative Example 6

The same as Example 1 except that a polyoxymethylene resin was used in place of the polyketone copolymer.

Properties Evaluation

The prepared pellets of the above examples were injection-molded to produce specimens for chair cams. Specific gravity, wear resistance, and impact resistance were evaluated in following method in comparison with the specimens of the comparative examples, and results are shown in Table 3.

1. Wear resistance evaluation: The moldings prepared in Examples and Comparative Examples were processed into a disk shape (120 mm in diameter and 2 mm in thickness) and left at 25°C for 2 days. Then, a Taber wear tester (manufactured by DAITO ELECTRON CO., LTD., conditions: a load of 1 kg, wear wheel H-22) was used to measure a wear amount according to JIS K-7311.
2. Izod impact strength: Evaluated about ASTM D256, 1/4 inch thick specimens at room temperature.
3. Noise measurement: While using the wear resistance evaluation device of 1., noise was measured by a portable noise measurement device.

[Table 3]

| Item | Example 8 | Example 9 | Example 10 | Example 11 | Example 12 | Comparative Example 6 |
|---|---|---|---|---|---|---|
| Specific gravity | 1.24 | 1.22 | 1.24 | 1.23 | 1.22 | 1.42 |
| Wear resistance wear amount (mg) | 18 | 20 | 19 | 22 | 20 | 44 |
| Izod impact strength (kg·cm/cm) | 42 | 40 | 44 | 38 | 42 | 44 |
| Noise level during wear measurement (dB) | 60 | 62 | 61 | 63 | 65 | 80 |

As shown in Table 1, in Examples 8 to 12, it was evaluated as being superior in light weight, wear resistance, impact resistance and noise generation as compared with Comparative Example 6, thereby it is proved to be suitable for use as a chair cam.
In the present invention, when measured by the above method, the wear amount is 25 mg or less and the noise generation amount is 70 dB or less.

Example 13

Under presence of catalyst composition formed from palladium acetate, trifluoroacetic acid and ((2,2-dimethyl-1,3-dioxane-5,5-diyl)bis(methylene)bis(2-methoxyphenyl) phosphine), linear alternating polyketone terpolymers comprising carbon monoxide and ethylene and propene are prepared. In the above, content of trifluoroacetic acid with respect

to palladium is 11 times the molar ratio, and two stages of the first stage at a polymerization temperature of 80°C and the second stage at 84°C are carried out. In the prepared polyketone terpolymer, a molar ratio of ethylene to propene was 46:4. Also, a melting point of the polyketone terpolymer was 220°C, LVN measured at 25°C by HFIP (hexa-fluoroisopropanol) was 1.2 dl/g, MI (Melt Index) was 60 g/10 min, MWD was 2.0.

The polyketone copolymer prepared above was placed in a mold and injection molded at 70 bar, a temperature of 250°C and a mold temperature of 150°C to produce a gear.

Example 14

Under presence of catalyst composition formed from palladium acetate, trifluoroacetic acid and ((2,2-dimethyl-1,3-dioxane-5,5-diyl)bis(methylene)bis(2-methoxyphenyl) phosphine), linear alternating polyketone terpolymers comprising carbon monoxide and ethylene and propene are prepared. In the above, content of trifluoroacetic acid with respect to palladium is 10 times the molar ratio, and two stages of the first stage at a polymerization temperature of 78°C and the second stage at 84°C are carried out. In the prepared polyketone terpolymer, a molar ratio of ethylene to propene was 46:4. Also, a melting point of the polyketone terpolymer was 220°C, LVN measured at 25°C by HFIP (hexa-fluoroisopropanol) was 1.4 dl/g, MI (Melt Index) was 60 g/10 min, MWD was 2.0.

The polyketone copolymer prepared above was placed in a mold and injection molded at 70 bar, a temperature of 250°C and a mold temperature of 150°C to produce a gear.

Example 15

Under presence of catalyst composition formed from palladium acetate, trifluoroacetic acid and ((2,2-dimethyl-1,3-dioxane-5,5-diyl)bis(methylene)bis(2-methoxyphenyl) phosphine), linear alternating polyketone terpolymers comprising carbon monoxide and ethylene and propene are prepared. In the above, content of trifluoroacetic acid with respect to palladium is 9 times the molar ratio, and two stages of the first stage at a polymerization temperature of 74°C and the second stage at 84°C are carried out. In the prepared polyketone terpolymer, a molar ratio of ethylene to propene was 46:4. Also, a melting point of the polyketone terpolymer was 220°C, LVN measured at 25°C by HFIP (hexa-fluoroisopropanol) was 1.6 dl/g, MI (Melt Index) was 60 g/10 min, MWD was 2.0.

The polyketone copolymer prepared above was placed in a mold and injection molded at 70 bar, a temperature of 250°C and a mold temperature of 150°C to produce a gear.

Example 16

Under presence of catalyst composition formed from palladium acetate, trifluoroacetic acid and ((2,2-dimethyl-1,3-dioxane-5,5-diyl)bis(methylene)bis(2-methoxyphenyl) phosphine), linear alternating polyketone terpolymers comprising carbon monoxide and ethylene and propene are prepared. In the above, content of trifluoroacetic acid with respect to palladium is 10 times the molar ratio, and two stages of the first stage at a polymerization temperature of 78°C and the second stage at 84°C are carried out. In the prepared polyketone terpolymer, a molar ratio of ethylene to propene was 46:4. Also, a melting point of the polyketone terpolymer was 220°C, LVN measured at 25°C by HFIP (hexa-fluoroisopropanol) was 1.4 dl/g, MI (Melt Index) was 60 g/10 min, MWD was 1.8.

The polyketone copolymer prepared above was placed in a mold and injection molded at 70 bar, a temperature of 250°C and a mold temperature of 150°C to produce a gear.

Example 17

Under presence of catalyst composition formed from palladium acetate, trifluoroacetic acid and ((2,2-dimethyl-1,3-dioxane-5,5-diyl)bis(methylene)bis(2-methoxyphenyl) phosphine), linear alternating polyketone terpolymers comprising carbon monoxide and ethylene and propene are prepared. In the above, content of trifluoroacetic acid with respect to palladium is 10 times the molar ratio, and two stages of the first stage at a polymerization temperature of 78°C and the second stage at 84°C are carried out. In the prepared polyketone terpolymer, a molar ratio of ethylene to propene was 46:4. Also, a melting point of the polyketone terpolymer was 220°C, LVN measured at 25°C by HFIP (hexa-fluoroisopropanol) was 1.4 dl/g, MI (Melt Index) was 60 g/10 min, MWD was 2.2.

The polyketone copolymer prepared above was placed in a mold and injection molded at 70 bar, a temperature of 250°C and a mold temperature of 150°C to produce a gear.

Comparative Example 7

The same as Example 13 except that a high impact nylon 66 was used in place of the polyketone copolymer.

Properties Evaluation

1. Dimensional stability evaluation: Product strain rate was evaluated according to MS211-47 for vertical and horizontal directions at a temperature of 50°C and a relative humidity of 90 %.
2. Wear resistance evaluation: Weight loss was calculated at 25°C using a thrust washer test device shown in FIG. 1 for the plastic gear manufactured according to the Examples, and then wear coefficient ($K_{LNP}$) was calculated by a following equation.

$$K_{LNP} = \frac{W}{PVT}$$

(V = velocity (ft./min.), P = pressure (lbs./in2), T = elapsed time (hrs) and W is weight loss divided by density, thereby it means an wear amount per unit volume.)
3. Moisture resistance evaluation: The polyketone copolymer prepared in Example 13 to 17 and a nylon 66 of DuPont were separately inserted into an extruder using a biaxial screw having L/D32 and D 40, and extruded through melt-kneading at 240°C and screw rotating speed of 250 rpm to produce pellets. The produced pellets were left in 85°C/85% thermo-hygrostat for 48 hours, and water content rate for 20 minutes of the pellets was measured using a Mettler HR-83.
4. Driving evaluation: After the helmet gears manufactured in Examples and Comparative examples were mounted on a helmet, an evaluation was made to drive an inside of a face part and a number of times the gears endured was measured.

[Table 4]

| Item | Example 13 | Example 14 | Example 15 | Example 16 | Example 17 | Comparative Example 7 |
|---|---|---|---|---|---|---|
| Product strain rate - vertical (50°C, RH 90 %) | 0.22 | 0.24 | 0.20 | 0.21 | 0.18 | 0.34 |
| Product strain rate - horizontal (50°C, RH 90 %) | 0.08 | 0.12 | 0.10 | 0.08 | 0.10 | 0.25 |
| wear coefficient $K_{LNP}$ | 250 | 240 | 260 | 240 | 250 | 450 |
| Moisture resistance | 0.31 | 0.34 | 0.28 | 0.32 | 0.40 | 3.2 |
| Driving evaluation (number of times) | 300,025 | 298,025 | 300,245 | 299,031 | 300,025 | 150,140 |

From Table 4, it can be seen that the polyketone resin in a base state of the present invention is superior in property maintenance rate, wear resistance (wear coefficient of 200 to 300) and moisture resistance as compared with high impact polyamide. Therefore, the helmet gear for electric welding manufactured from the polyketone resin of the present invention can solve a problem that a face protection cover of the helmet for electric welding can easily flow down, because it is less worn than conventional polyamide gear.

Example 18

Under presence of catalyst composition formed from palladium acetate, trifluoroacetic acid and ((2,2-dimethyl-1,3-dioxane-5,5-diyl)bis(methylene)bis(2-methoxyphenyl) phosphine), linear alternating polyketone terpolymers comprising carbon monoxide and ethylene and propene are prepared. In the above, content of trifluoroacetic acid with respect to palladium is 11 times the molar ratio, and two stages of the first stage at a polymerization temperature of 80°C and the second stage at 84°C are carried out. In the prepared polyketone terpolymer, a molar ratio of ethylene to propene was 46:4. Also, a melting point of the polyketone terpolymer was 220°C, LVN measured at 25°C by HFIP (hexa-fluoroisopropanol) was 1.2 dl/g, MI (Melt Index) was 60 g/10 min, MWD was 2.0.

The polyketone terpolymer was solidified in a plate shape at a room temperature to a thickness corresponding to a gear thickness, and in the solidified plastic board, a gear of a required size were molded by blanking to produce a gear.

Example 19

After produced the polyketone terpolymer in a same method as Example 18, 100 parts by weight of the polyketone terpolymer and 1 part by weight of silicone resin were mixed and solidified in a plate shape at a room temperature to a thickness corresponding to a gear thickness, and in the solidified plastic board, a gear of a required size were molded by blanking to produce a gear.

Example 20

Under presence of catalyst composition formed from palladium acetate, trifluoroacetic acid and ((2,2-dimethyl-1,3-dioxane-5,5-diyl)bis(methylene)bis(2-methoxyphenyl) phosphine), linear alternating polyketone terpolymers comprising carbon monoxide and ethylene and propene are prepared. In the above, content of trifluoroacetic acid with respect to palladium is 11 times the molar ratio, and two stages of the first stage at a polymerization temperature of 80°C and the second stage at 84°C are carried out. In the prepared polyketone terpolymer, a molar ratio of ethylene to propene was 46:4. Also, a melting point of the polyketone terpolymer was 220°C, LVN measured at 25°C by HFIP (hexa-fluoroisopropanol) was 1.4 dl/g, MI (Melt Index) was 60 g/10 min, MWD was 2.0.

The polyketone terpolymer was solidified in a plate shape at a room temperature to a thickness corresponding to a gear thickness, and in the solidified plastic board, a gear of a required size were molded by blanking to produce a gear.

Example 21

Under presence of catalyst composition formed from palladium acetate, trifluoroacetic acid and ((2,2-dimethyl-1,3-dioxane-5,5-diyl)bis(methylene)bis(2-methoxyphenyl) phosphine), linear alternating polyketone terpolymers comprising carbon monoxide and ethylene and propene are prepared. In the above, content of trifluoroacetic acid with respect to palladium is 11 times the molar ratio, and two stages of the first stage at a polymerization temperature of 80°C and the second stage at 84°C are carried out. In the prepared polyketone terpolymer, a molar ratio of ethylene to propene was 46:4. Also, a melting point of the polyketone terpolymer was 220°C, LVN measured at 25°C by HFIP (hexa-fluoroisopropanol) was 2.0 dl/g, MI (Melt Index) was 60 g/10 min, MWD was 2.0.

The polyketone terpolymer was solidified in a plate shape at a room temperature to a thickness corresponding to a gear thickness, and in the solidified plastic board, a gear of a required size were molded by blanking to produce a gear.

Example 22

After produced the polyketone terpolymer in a same method as Example 20, 100 parts by weight of the polyketone terpolymer and 5 parts by weight of silicone resin were mixed and solidified in a plate shape at a room temperature to a thickness corresponding to a gear thickness, and in the solidified plastic board, a gear of a required size were molded by blanking to produce a gear.

Example 23

After produced the polyketone terpolymer in a same method as Example 20, 100 parts by weight of the polyketone terpolymer and 10 parts by weight of silicone resin were mixed and solidified in a plate shape at a room temperature to a thickness corresponding to a gear thickness, and in the solidified plastic board, a gear of a required size were molded by blanking to produce a gear.

Comparative Example 8

The same as Example 18 except that POM was used as a material of Dupont in a base state in place of the polyketone copolymer.

Properties Evaluation

1. Evaluation of dimensional stability, wear resistance, and impact resistance: The plastic panels produced in the above Examples and Comparative Examples were evaluated for dimensional stability, wear resistance and impact resistance by following methods.

1) Dimensional stability evaluation: Product strain rate was evaluated according to MS211-47 for vertical and horizontal directions at a temperature of 50°C and a relative humidity of 90 %.
2) Wear amount evaluation: Evaluation was made using a cylindrical specimen having an inner diameter of 20 mm, an outer diameter of 25 mm and a height of 15 mm and a counter material. Wear loss was measured at a speed of 50 rpm under a JIS K7218 standard, a load of 150 N, and a wear distance of 3 km.
3) Impact resistance evaluation: Evaluated about ASTM D256, 1/4 inch thick specimens at room temperature.

[Table 5]

| Item | Example 18 | Example 19 | Example 20 | Example 21 | Example 22 | Example 23 | Comparative Example 8 |
|---|---|---|---|---|---|---|---|
| Product strain rate - vertical (50°C, RH 90 %) | 0.12 | 0.14 | 0.13 | 0.10 | 0.08 | 0.15 | 0.25 |
| Product strain rate - horizontal (50°C, RH 90 %) | 0.04 | 0.03 | 0.06 | 0.05 | 0.062 | 0.04 | 0.12 |
| Wear amount (g) | 0.018 | 0.015 | 0.013 | 0.010 | 0.003 | 0.002 | 0.083 |
| Izod impact strength (kg·cm/cm) | 33 | 35 | 30 | 60 | 28 | 22 | 22 |

The gears manufactured from the polyketone base or blend composition of the present invention of Example 18 to 23 are superior in product strain rate in vertical and horizontal directions as compared with the gears manufactured by a method of the comparative example and have excellent wear resistance.

Also, the present invention had a wear amount of 0.020 g or less, which is superior, when measured under JIS K7218 at a speed of 50 rpm, a load of 150 N and a wear distance of 3 km.

Example 24

Under presence of catalyst composition formed from palladium acetate, trifluoroacetic acid and ((2,2-dimethyl-1,3-dioxane-5,5-diyl)bis(methylene)bis(2-methoxyphenyl) phosphine), linear alternating polyketone terpolymers comprising carbon monoxide and ethylene and propene are prepared. In the above, content of trifluoroacetic acid with respect to palladium is 11 times the molar ratio, and two stages of the first stage at a polymerization temperature of 80°C and the second stage at 84°C are carried out. In the prepared polyketone terpolymer, a molar ratio of ethylene to propene was 46:4. Also, a melting point of the polyketone terpolymer was 220°C, LVN measured at 25°C by HFIP (hexa-fluoroisopropanol) was 1.2 dl/g, MI (Melt Index) was 60 g/10 min, MWD was 2.0.

The polyketone terpolymer prepared above was extruded through melt-kneading on an extruder using a biaxial screw having L/D32 and D 40 at 240°C and a screw rotating speed of 250 rpm to produce a specimen for manufacturing a yarn guide.

Example 25

Under presence of catalyst composition formed from palladium acetate, trifluoroacetic acid and ((2,2-dimethyl-1,3-dioxane-5,5-diyl)bis(methylene)bis(2-methoxyphenyl) phosphine), linear alternating polyketone terpolymers comprising carbon monoxide and ethylene and propene are prepared. In the above, content of trifluoroacetic acid with respect to palladium is 10 times the molar ratio, and two stages of the first stage at a polymerization temperature of 78°C and the second stage at 84°C are carried out. In the prepared polyketone terpolymer, a molar ratio of ethylene to propene was 46:4. Also, a melting point of the polyketone terpolymer was 220°C, LVN measured at 25°C by HFIP (hexa-fluoroisopropanol) was 1.4 dl/g, MI (Melt Index) was 60 g/10 min, MWD was 2.0.

The polyketone terpolymer prepared above was extruded through melt-kneading on an extruder using a biaxial screw having L/D32 and D 40 at 240°C and a screw rotating speed of 250 rpm to produce a specimen for manufacturing a yarn guide.

Example 26

Under presence of catalyst composition formed from palladium acetate, trifluoroacetic acid and ((2,2-dimethyl-1,3-dioxane-5,5-diyl)bis(methylene)bis(2-methoxyphenyl) phosphine), linear alternating polyketone terpolymers comprising carbon monoxide and ethylene and propene are prepared. In the above, content of trifluoroacetic acid with respect to palladium is 9 times the molar ratio, and two stages of the first stage at a polymerization temperature of 74°C and the second stage at 84°C are carried out. In the prepared polyketone terpolymer, a molar ratio of ethylene to propene was 46:4. Also, a melting point of the polyketone terpolymer was 220°C, LVN measured at 25°C by HFIP (hexa-fluoroisopropanol) was 1.6 dl/g, MI (Melt Index) was 60 g/10 min, MWD was 2.0.

The polyketone terpolymer prepared above was extruded through melt-kneading on an extruder using a biaxial screw having L/D32 and D 40 at 240°C and a screw rotating speed of 250 rpm to produce a specimen for manufacturing a yarn guide.

Example 27

Under presence of catalyst composition formed from palladium acetate, trifluoroacetic acid and ((2,2-dimethyl-1,3-dioxane-5,5-diyl)bis(methylene)bis(2-methoxyphenyl) phosphine), linear alternating polyketone terpolymers comprising carbon monoxide and ethylene and propene are prepared. In the above, content of trifluoroacetic acid with respect to palladium is 10 times the molar ratio, and two stages of the first stage at a polymerization temperature of 78°C and the second stage at 84°C are carried out. In the prepared polyketone terpolymer, a molar ratio of ethylene to propene was 46:4. Also, a melting point of the polyketone terpolymer was 220°C, LVN measured at 25°C by HFIP (hexa-fluoroisopropanol) was 1.4 dl/g, MI (Melt Index) was 60 g/10 min, MWD was 1.8.

The polyketone terpolymer prepared above was extruded through melt-kneading on an extruder using a biaxial screw having L/D32 and D 40 at 240°C and a screw rotating speed of 250 rpm to produce a specimen for manufacturing a yarn guide.

Experimental Example 1

Specific gravity, wear resistance, chemical resistance and corrosion resistance of the specimens for the yarn guide produced by methods of Example 24 to 27 were measured and compared with physical properties of aluminum, and results are shown in Table 6. Physical properties are evaluated as follows.

Properties Evaluation

1. Wear resistance evaluation: Specimens from the Examples were fabricated in a form of a disk (120 mm in diameter and 2 mm in thickness) and ceramic and aluminum specimens of a same type were produced and left at 25°C for 2 days respectively, and then wear amount was measured using a Taber wear tester (DAITO ELECTRON CO., LTD., conditions: a load of 1 kg, a wear wheel H-22) according to JIS K-7311.

2. Chemical resistance evaluation: The specimens prepared from the Examples were cut into a size of 195 mm x 19 mm x 3 mm, and ceramic and aluminum specimens of a same size were prepared. The specimens were fixed on a jig for each strain, immersed in cyclopentane for 2 minutes, taken out and left for a minute, and JIG value of the strain without cracking was measured.

3. Corrosion resistance evaluation: Aqueous spray test was conducted by a method specified in ASTM B117 and then evaluated according to following criteria.
Excellent: no white rust after 120 hours
Good: less than 5 % of white rust area after 120 hours
Insufficient: 5 % or more, less than 50 % of white rust area after 120 hours
Defective: 50 % or more of white rust area after 120 hours

4. Full winding rate evaluation: Full winding rate was measured by a conventional method.

5. Frequency of occurrence of hairness evaluation: For a polyethylene multifilament, frequency of occurrence was evaluated according to a number of hairness per 100,000 m.

[Table 6]

| Item | Example 24 | Example 25 | Example 26 | Example 27 | Aluminum |
|---|---|---|---|---|---|
| Specific gravity (g/cm$^3$) | 1.24 | 1.22 | 1.20 | 1.26 | 2.7 |
| Wear resistance wear amount (mg) | 18 | 20 | 19 | 22 | 68 |
| Chemical resistance (JIG value) | 3.5 | 3.2 | 3.4 | 3.5 | 1.8 |
| Corrosion resistance | Excellent | Excellent | Excellent | Excellent | Insufficient |
| Full winding rate (%) | 98 | 99 | 98 | 99 | 95 |
| Number of hairness (pieces/100000 m) | 1 | 1 | 1 | 1 | 4 |

Table 6 shows that the polyketone copolymer of the present invention is lighter than ceramic and aluminum and is suitable for being used as a material for a yarn guide because of its excellent wear resistance, chemical resistance, corrosion resistance, full winding rate and low frequency of occurrence of hairness.

Example 28

Linear alternating polyketone terpolymers comprising carbon monoxide and ethylene and propene are prepared under presence of catalyst composition formed from palladium acetate, trifluoroacetic acid and ((2,2-dimethyl-1,3-dioxane-5,5-diyl)bis(methylene)bis(2-methoxyphenyl) phosphine). In the above, content of trifluoroacetic acid with respect to palladium is 11 times the molar ratio, and two stages of the first stage at a polymerization temperature of 80°C and the second stage at 84°C are carried out. In the prepared polyketone terpolymer, a molar ratio of ethylene to propene was 46:4. Also, a melting point of the polyketone terpolymer was 220°C, LVN measured at 25°C by HFIP (hexa-fluoroisopropanol) was 1.2 dl/g, MI (Melt Index) was 60 g/10 min, MWD was 2.0.

The polyketone terpolymers prepared above were molded into pellets on an extruder using a biaxial screw having a diameter of 40 mm and L/D=32, which was operated at 250 rpm, and then injection molded to produce a specimen for a microwave component.

Example 29

Linear alternating polyketone terpolymers comprising carbon monoxide and ethylene and propene are prepared under presence of catalyst composition formed from palladium acetate, trifluoroacetic acid and ((2,2-dimethyl-1,3-dioxane-5,5-diyl)bis(methylene)bis(2-methoxyphenyl) phosphine). In the above, content of trifluoroacetic acid with respect to palladium is 10 times the molar ratio, and two stages of the first stage at a polymerization temperature of 78°C and the second stage at 84°C are carried out. In the prepared polyketone terpolymer, a molar ratio of ethylene to propene was 46:4. Also, a melting point of the polyketone terpolymer was 220°C, LVN measured at 25°C by HFIP (hexa-fluoroisopropanol) was 1.4 dl/g, MI (Melt Index) was 60 g/10 min, MWD was 2.0.

The polyketone terpolymers prepared above were molded into pellets on an extruder using a biaxial screw having a diameter of 40 mm and L/D=32, which was operated at 250 rpm, and then injection molded to produce a specimen for a microwave component.

Example 30

Linear alternating polyketone terpolymers comprising carbon monoxide and ethylene and propene are prepared under presence of catalyst composition formed from palladium acetate, trifluoroacetic acid and ((2,2-dimethyl-1,3-dioxane-5,5-diyl)bis(methylene)bis(2-methoxyphenyl) phosphine). In the above, content of trifluoroacetic acid with respect to palladium is 9 times the molar ratio, and two stages of the first stage at a polymerization temperature of 74°C and the second stage at 84°C are carried out. In the prepared polyketone terpolymer, a molar ratio of ethylene to propene was 46:4. Also, a melting point of the polyketone terpolymer was 220°C, LVN measured at 25°C by HFIP (hexa-fluoroisopropanol) was 1.6 dl/g, MI (Melt Index) was 60 g/10 min, MWD was 2.0.

The polyketone terpolymer prepared above were molded into pellets on an extruder using a biaxial screw having a diameter of 40 mm and L/D=32, which was operated at 250 rpm, and then injection molded to produce a specimen for a microwave component.

Example 31

Linear alternating polyketone terpolymers comprising carbon monoxide and ethylene and propene are prepared under presence of catalyst composition formed from palladium acetate, trifluoroacetic acid and ((2,2-dimethyl-1,3-dioxane-5,5-diyl)bis(methylene)bis(2-methoxyphenyl) phosphine). In the above, content of trifluoroacetic acid with respect to palladium is 10 times the molar ratio, and two stages of the first stage at a polymerization temperature of 78°C and the second stage at 84°C are carried out. In the prepared polyketone terpolymer, a molar ratio of ethylene to propene was 46:4. Also, a melting point of the polyketone terpolymer was 220°C, LVN measured at 25°C by HFIP (hexa-fluoroisopropanol) was 1.4 dl/g, MI (Melt Index) was 60 g/10 min, MWD was 1.8.

The polyketone terpolymers prepared above were molded into pellets on an extruder using a biaxial screw having a diameter of 40 mm and L/D=32, which was operated at 250 rpm, and then injection molded to produce a specimen for a microwave component.

Example 32

Linear alternating polyketone terpolymers comprising carbon monoxide and ethylene and propene are prepared under presence of catalyst composition formed from palladium acetate, trifluoroacetic acid and ((2,2-dimethyl-1,3-dioxane-5,5-diyl)bis(methylene)bis(2-methoxyphenyl) phosphine). In the above, content of trifluoroacetic acid with respect to palladium is 10 times the molar ratio, and two stages of the first stage at a polymerization temperature of 78°C and the second stage at 84°C are carried out. In the prepared polyketone terpolymer, a molar ratio of ethylene to propene was 46:4. Also, a melting point of the polyketone terpolymer was 220°C, LVN measured at 25°C by HFIP (hexa-fluoroisopropanol) was 1.4 dl/g, MI (Melt Index) was 60 g/10 min, MWD was 2.2.

The polyketone terpolymers prepared above were molded into pellets on an extruder using a biaxial screw having a diameter of 40 mm and L/D=32, which was operated at 250 rpm, and then injection molded to produce a specimen for a microwave component.

Comparative Example 9

Specimens for microwave components were produced using PTFE, which was conventionally used as a material for microwave components.

Properties Evaluation

The prepared pellets of the above Examples were injection-molded to prepare specimens for microwave components. Properties of the specimens were evaluated in following method in comparison with the specimens of the comparative examples, and results are shown in Table 1.

1. Izod impact strength evaluation: Performed according to ASTM D256.
2. Water absorption rate evaluation: Measurement of water content after treatment for 24 hours at a temperature of 50°C and a relative humidity of 90 %
3. Friction-wear resistance (Ring-on-Ring Type, large resin): A through-type test piece having an outer diameter of 25.6 mm, an inner diameter of 20 mm and a height of 15 mm is injection-molded, fixed to a test apparatus, and subjected to a test under a driving condition of a pressure load of 6.6 kgf and a linear velocity of 10 cm/s. In this case, non-wear amount was calculated using following formula to evaluate friction-wear resistance. The smaller the amount of non-wear obtained, the better the friction-wear resistance.

$$\text{Non-wear amount} = \text{wear weight(mg)}/[\text{density(mg/mm3)} \times \text{pressure load(kgf)} \times \text{travel distance(km)}]$$

*test apparatus: Trust type friction-wear tester

4. Cycly time(injection moldability evaluation sec): Time taken for the pellets prepared in Examples or Comparative Examples to crystallize after melting was measured in sec.

Properties of Examples and Comparative Examples were as shown in Table 7.

[Table 7]

| | Example 28 | Example 29 | Example 30 | Example 31 | Example 32 | Comparative Example 9 |
|---|---|---|---|---|---|---|
| Properties | IV : 1.2 MWD : 2.0 | IV : 1.4 MWD : 2.0 | IV : 1.6 MWD : 2.0 | IV : 1.4 MWD : 1.8 | IV : 1.4 MWD : 2.2 | PTFE |
| Izod impact strength ($kJ/m^2$) | 14 | 12 | 11 | 13 | 15 | 6 |
| Product water absorption rate (%, 50°C/90 % RH) | 0.8 | 0.9 | 0.9 | 1.0 | 0.8 | 5.0 |
| wear resistance ($mm^3/kg/km$) | 0.62 | 0.60 | 0.55 | 0.60 | 0.62 | 5.10 |
| Injection cycle (sec) | 17 | 17 | 17 | 17 | 17 | 23 |

As shown in Table 7, in the examples, the water absorption rate was lower than that of the comparative example, and the wear amount was also evaluated to be very low, thereby wear resistance was excellent. In addition, the injection cycle was short, thereby the injection moldability was excellent. Therefore, the polyketone microwave components manufactured by the examples of the present invention are well suited for application to microwave components having excellent impact resistance, wear resistance, moisture resistance, and injection moldability.

Example 33

Under presence of catalyst composition formed from palladium acetate, trifluoroacetic acid and ((2,2-dimethyl-1,3-dioxane-5,5-diyl)bis(methylene)bis(2-methoxyphenyl) phosphine), linear alternating polyketone terpolymers comprising carbon monoxide and ethylene and propene are prepared. In the above, content of trifluoroacetic acid with respect to palladium is 11 times the molar ratio, and two stages of the first stage at a polymerization temperature of 80°C and the second stage at 84°C are carried out. In the prepared polyketone terpolymer, a molar ratio of ethylene to propene was 46:4. Also, a melting point of the polyketone terpolymer was 220°C, LVN measured at 25°C by HFIP (hexafluoroisopropanol) was 1.2 dl/g, MI (Melt Index) was 60 g/10 min, MWD was 2.0.

The polyketone terpolymer prepared above was extruded through melt-kneading on an extruder using a biaxial screw having L/D32 and D 40 at 240°C, which was operated at 250 rpm.

Example 34

Under presence of catalyst composition formed from palladium acetate, trifluoroacetic acid and ((2,2-dimethyl-1,3-dioxane-5,5-diyl)bis(methylene)bis(2-methoxyphenyl) phosphine), linear alternating polyketone terpolymers comprising carbon monoxide and ethylene and propene are prepared. In the above, content of trifluoroacetic acid with respect to palladium is 10 times the molar ratio, and two stages of the first stage at a polymerization temperature of 78°C and the second stage at 84°C are carried out. In the prepared polyketone terpolymer, a molar ratio of ethylene to propene was 46:4. Also, a melting point of the polyketone terpolymer was 220°C, LVN measured at 25°C by HFIP (hexafluoroisopropanol) was 1.4 dl/g, MI (Melt Index) was 60 g/10 min, MWD was 2.0.

The polyketone terpolymer prepared above was extruded through melt-kneading on an extruder using a biaxial screw having L/D32 and D 40 at 240°C, which was operated at 250 rpm.

Example 35

Under presence of catalyst composition formed from palladium acetate, trifluoroacetic acid and ((2,2-dimethyl-1,3-dioxane-5,5-diyl)bis(methylene)bis(2-methoxyphenyl) phosphine), linear alternating polyketone terpolymers comprising carbon monoxide and ethylene and propene are prepared. In the above, content of trifluoroacetic acid with respect to palladium is 9 times the molar ratio, and two stages of the first stage at a polymerization temperature of 74°C and the second stage at 84°C are carried out. In the prepared polyketone terpolymer, a molar ratio of ethylene to propene was 46:4. Also, a melting point of the polyketone terpolymer was 220°C, LVN measured at 25°C by HFIP (hexafluoroisopropanol) was 1.6 dl/g, MI (Melt Index) was 60 g/10 min, MWD was 2.0.

The polyketone terpolymer prepared above was extruded through melt-kneading on an extruder using a biaxial screw having L/D32 and D 40 at 240°C, which was operated at 250 rpm.

Example 36

Under presence of catalyst composition formed from palladium acetate, trifluoroacetic acid and ((2,2-dimethyl-1,3-dioxane-5,5-diyl)bis(methylene)bis(2-methoxyphenyl) phosphine), linear alternating polyketone terpolymers comprising carbon monoxide and ethylene and propene are prepared. In the above, content of trifluoroacetic acid with respect to palladium is 10 times the molar ratio, and two stages of the first stage at a polymerization temperature of 78°C and the second stage at 84°C are carried out. In the prepared polyketone terpolymer, a molar ratio of ethylene to propene was 46:4. Also, a melting point of the polyketone terpolymer was 220°C, LVN measured at 25°C by HFIP (hexafluoroisopropanol) was 1.4 dl/g, MI (Melt Index) was 60 g/10 min, MWD was 1.8.

The polyketone terpolymer prepared above was extruded through melt-kneading on an extruder using a biaxial screw having L/D32 and D 40 at 240°C, which was operated at 250 rpm.

Example 37

Under presence of catalyst composition formed from palladium acetate, trifluoroacetic acid and ((2,2-dimethyl-1,3-dioxane-5,5-diyl)bis(methylene)bis(2-methoxyphenyl) phosphine), linear alternating polyketone terpolymers comprising carbon monoxide and ethylene and propene are prepared. In the above, content of trifluoroacetic acid with respect to palladium is 10 times the molar ratio, and two stages of the first stage at a polymerization temperature of 78°C and the second stage at 84°C are carried out. In the prepared polyketone terpolymer, a molar ratio of ethylene to propene was 46:4. Also, a melting point of the polyketone terpolymer was 220°C, LVN measured at 25°C by HFIP (hexafluoroisopropanol) was 1.4 dl/g, MI (Melt Index) was 60 g/10 min, MWD was 2.2.

The polyketone terpolymer prepared above was mixed and extruded through melt-kneading on an extruder using a biaxial screw having L/D32 and D 40 at 240°C, which was operated at 250 rpm.

Example 38

The same as Example 34 except that intrinsic viscosity of the polyketone was adjusted to 2.0 dl/g.

Comparative Example 10

The same as Example 33 except that a polyoxymethylene resin was used in place of the polyketone copolymer.

Properties Evaluation

The prepared pellets of the above Examples were injection-molded to prepare specimens for bedding cleaner. Scratch resistance and impact resistance of the specimens were evaluated by following method in comparison with the specimens of the comparative example. Results are shown in Table 8.

1. Wear resistance evaluation: The moldings prepared in Examples and Comparative Examples were processed into a disk shape (120 mm in diameter and 2 mm in thickness) and left at 25°C for 2 days. Then, a Taber wear tester (manufactured by DAITO ELECTRON CO., LTD., conditions: a load of 1 kg, wear wheel H-22) was used to measure a wear amount according to JIS K-7311.
2. Scratch resistance evaluation: Evaluated according to JIS K 5600-5-4 pencil scratch hardness measurement method
3. Izod impact strength: Evaluated about ASTM D256, 1/4 inch thick specimens at room temperature.

[Table 8]

| Item | Example 33 | Example 34 | Example 35 | Example 36 | Example 37 | Example 38 | Comparative Example 10 |
|---|---|---|---|---|---|---|---|
| Wear resistance wear amount (mg) | 18 | 20 | 19 | 22 | 20 | 16 | 44 |
| Scratch resistance | 3H | 3H | 3H | 2H | 3H | 3H | 2H |

(continued)

| Item | Example 33 | Example 34 | Example 35 | Example 36 | Example 37 | Example 38 | Comparative Example 10 |
|---|---|---|---|---|---|---|---|
| Izod impact strength (kg·cm/cm) | 42 | 40 | 44 | 38 | 42 | 45 | 44 |

As shown in Table 8, in Example 33 to 38, wear resistance, scratch resistance and impact strength were evaluated to be superior to the comparative examples, and it was proved to be suitable for use as a bedding cleaner cam.
The polyketone bedding cleaner cam of the present invention has a wear amount of 25 mg or less, which is excellent.

Example 39

Linear alternating polyketone terpolymers comprising carbon monoxide and ethylene and propene are prepared under presence of catalyst composition formed from palladium acetate, trifluoroacetic acid and ((2,2-dimethyl-1,3-dioxane-5,5-diyl)bis(methylene)bis(2-methoxyphenyl) phosphine). In the prepared polyketone terpolymer, a molar ratio of ethylene to propene was 46:4. Also, a melting point of the polyketone terpolymer was 220°C, LVN measured at 25°C by HFIP (hexa-fluoroisopropanol) was 1.4 dl/g, MI (Melt Index) was 48 g/10 min, residual amount of palladium catalyst was 10 ppm, and a molecular weight distribution was 2.0. The polyketone terpolymer prepared above was molded into pellets on an extruder using a biaxial screw having a diameter of 2.5 cm and L/D=32, which was operated at 250 rpm, and then injection molded to produce a specimen for a polyketone office supply.
Apart from the above specimen, a polyketone composition was prepared by incorporating a silicone oil, which is a wear resistant agent, into the polyketone, wherein the content of the silicone oil was 1.0 weight% based on total composition. After produced the polyketone composition, it was produced into pellets in same method as a pellet preparation described above to prepare a specimen for a polyketone office component.

Comparative Example 11

A polyoxymethylene resin was produced into pellets on an extruder using a biaxial screw having a diameter of 2.5 cm and L/D=32, which was operated at 250 rpm to prepare a specimen for a polyketone office component. And apart from that, a specimen for a polyketone office component by adding same amount of silicone oil as Example 1.

Properties Evaluation

Wear resistance and impact resistance of each of the specimens for office components prepared in Example 39 and Comparative Example 11 were evaluated in case of a base state and a case where a wear resistance agent was added, respectively, and results are shown in Table 9.

1. Friction-wear resistance (Ring-on-Ring type, large resin): A through-type test piece having an outer diameter of 25.6 mm, an inner diameter of 20 mm and a height of 15 mm is injection-molded, fixed to a test apparatus, and subjected to a test under a driving condition of a pressure load of 6.6 kgf and a linear velocity of 10 cm/s. Wear resistance was evaluated by evaluating a non-wear amount. The smaller the amount of non-wear obtained, the better the friction-wear resistance.
2. Izod impact strength: Performed according to ASTM D256.

[Table 9]

<table>
<tr><th></th><th>Grade</th><th>specific gravity</th><th>Cycle time(s)</th><th>Coefficient of friction</th><th>wear amount (mm³/kg/km)</th><th>noise</th><th>impact strength (kJ/m²)</th></tr>
<tr><td rowspan="2">Example 39</td><td>Base</td><td rowspan="2">1.24</td><td rowspan="2">17</td><td>0.34</td><td>0.60</td><td>NO (60)</td><td rowspan="2">10</td></tr>
<tr><td>Wear resistant agent added</td><td>0.10</td><td>0.01</td><td>NO (60)</td></tr>
<tr><td rowspan="2">Comparative Example 11</td><td>Base</td><td rowspan="2">1.42</td><td rowspan="2">22</td><td>0.40</td><td>8.10</td><td>squeak noise (80)</td><td rowspan="2">6.5</td></tr>
<tr><td>Wear resistant agent added</td><td>0.13</td><td>0.10</td><td>NO(60)</td></tr>
</table>

As shown in Table 9, it was evaluated that the wear resistance and the impact resistance in polyketone base state were superior to those in POM base state, and the wear resistance and the impact resistance were also excellent in a case where wear resistant agent was added, thereby the polyketone office components produced by the example of the present invention had excellent impact resistance and wear resistance and was very suitable for application to polyketone office components.

Example 40

Linear alternating polyketone terpolymers comprising carbon monoxide and ethylene and propene are prepared under presence of catalyst composition formed from palladium acetate, trifluoroacetic acid and ((2,2-dimethyl-1,3-dioxane-5,5-diyl)bis(methylene)bis(2-methoxyphenyl) phosphine). In the above, content of trifluoroacetic acid with respect to palladium is 11 times the molar ratio, and two stages of the first stage at a polymerization temperature of 78°C and the second stage at 84°C are carried out. In the prepared polyketone terpolymer, a molar ratio of ethylene to propene was 85:15. Also, a melting point of the polyketone terpolymer was 220°C, LVN measured at 25°C by HFIP (hexafluoroisopropanol) was 1.4 dl/g, and MWD was 2.0.

The polyketone terpolymer prepared above was molded into pellets on an extruder using a biaxial screw having a diameter of 40 mm and L/D=32, which was operated at 250 rpm, and then injection molded to produce a specimen for a window drum for automobiles.

Example 41

Linear alternating polyketone terpolymers comprising carbon monoxide and ethylene and propene are prepared under presence of catalyst composition formed from palladium acetate, trifluoroacetic acid and ((2,2-dimethyl-1,3-dioxane-5,5-diyl)bis(methylene)bis(2-methoxyphenyl) phosphine). In the above, content of trifluoroacetic acid with respect to palladium is 10 times the molar ratio, and two stages of the first stage at a polymerization temperature of 74°C and the second stage at 84°C are carried out. In the prepared polyketone terpolymer, a molar ratio of ethylene to propene was 85:15. Also, a melting point of the polyketone terpolymer was 220°C, LVN measured at 25°C by HFIP (hexafluoroisopropanol) was 1.6 dl/g, and MWD was 2.0.

The polyketone terpolymer prepared above was molded into pellets on an extruder using a biaxial screw having a diameter of 40 mm and L/D=32, which was operated at 250 rpm, and then injection molded to produce a specimen for a window drum for automobiles.

Example 42

Linear alternating polyketone terpolymers comprising carbon monoxide and ethylene and propene are prepared under presence of catalyst composition formed from palladium acetate, trifluoroacetic acid and ((2,2-dimethyl-1,3-dioxane-5,5-diyl)bis(methylene)bis(2-methoxyphenyl) phosphine). In the above, content of trifluoroacetic acid with respect to palladium is 9 times the molar ratio, and two stages of the first stage at a polymerization temperature of 72°C and the

second stage at 78°C are carried out. In the prepared polyketone terpolymer, a molar ratio of ethylene to propene was 85:15. Also, a melting point of the polyketone terpolymer was 220°C, LVN measured at 25°C by HFIP (hexa-fluoroisopropanol) was 2.0 dl/g, and MWD was 2.0.

The polyketone terpolymer prepared above was molded into pellets on an extruder using a biaxial screw having a diameter of 40 mm and L/D=32, which was operated at 250 rpm, and then injection molded to produce a specimen for a window drum for automobiles.

Example 43

Linear alternating polyketone terpolymers comprising carbon monoxide and ethylene and propene are prepared under presence of catalyst composition formed from palladium acetate, trifluoroacetic acid and ((2,2-dimethyl-1,3-dioxane-5,5-diyl)bis(methylene)bis(2-methoxyphenyl) phosphine). In the above, content of trifluoroacetic acid with respect to palladium is 11 times the molar ratio, and two stages of the first stage at a polymerization temperature of 78°C and the second stage at 84°C are carried out. In the prepared polyketone terpolymer, a molar ratio of ethylene to propene was 85:15. Also, a melting point of the polyketone terpolymer was 220°C, LVN measured at 25°C by HFIP (hexa-fluoroisopropanol) was 1.4 dl/g, and MWD was 2.0.

The polyketone terpolymer prepared above and a silicon-based wear resistant agent were molded into pellets on an extruder using a biaxial screw having a diameter of 40 mm and L/D=32, which was operated at 250 rpm, and then injection molded to produce a specimen for a window drum for automobiles.

Comparative Example 12

A polyoxymethylene resin was molded into pellets on an extruder using a biaxial screw having a diameter of 40 cm and L/D=32, which was operated at 250 rpm, and then injection molded into a specimen of a window drum for automobiles.

Properties Evaluation

Properties of the specimens prepared in Examples 40 to 43 and Comparative Example 11 were evaluated in following method, and results are shown in Table 10.

1. Izod impact strength evaluation: Performed according to ASTM D256.
2. Friction coefficient: Coefficient of kinetic friction is a value that can represent a degree of kinetic friction. The larger the coefficient of kinetic friction means the greater the frictional force, and the smaller the value, the smaller the frictional force.
3. Wear property evaluation: Performed according to JIS K7218 (test condition: 3 km wear at 50 rmp and 150 N)

[Table 10]

| Item | Example 40 | Example 41 | Example 42 | Example 43 | Comparative Example 12 |
|---|---|---|---|---|---|
| impact strength ($kJ/m^2$) | 11 | 12 | 11 | 10 | 6 |
| friction coefficient | 0.34 | 0.33 | 0.35 | 0.14 | 0.15 |
| Wear property (g) | 0.009 | 0.008 | 0.010 | 0.004 | 0.191 |

As can be seen in Table 10, the impact strength of the Examples was improved compared to Comparative Example 11. Further, in case of Examples, the coefficient of kinetic friction and wear property were smaller than those of Comparative Example 12, thereby the frictional force was small and the wear resistance was improved. Therefore, a window drum manufactured by the examples of the present invention exhibits better impact resistance and wear resistance than the comparative example used as a conventional window drum material, and thus is more suitable for application as a window drum for automobiles.

Example 44

Linear alternating polyketone terpolymers comprising carbon monoxide and ethylene and propene are prepared under presence of catalyst composition formed from palladium acetate, trifluoroacetic acid and ((2,2-dimethyl-1,3-dioxane-5,5-diyl)bis(methylene)bis(2-methoxyphenyl) phosphine). In the above, content of trifluoroacetic acid with respect to palladium is 11 times the molar ratio, and two stages of the first stage at a polymerization temperature of 78°C and the second stage at 84°C are carried out. In the prepared polyketone terpolymer, a molar ratio of ethylene to propene was 85:15. Also, a melting point of the polyketone terpolymer was 220°C, LVN measured at 25°C by HFIP (hexa-fluoroisopropanol) was 1.4 dl/g, and MWD was 2.0.

The polyketone terpolymer prepared above was molded into pellets on an extruder using a biaxial screw having a diameter of 40 mm and L/D=32, which was operated at 250 rpm, and then injection molded to produce a specimen for a sun visor retainer for automobiles.

Example 45

Linear alternating polyketone terpolymers comprising carbon monoxide and ethylene and propene are prepared under presence of catalyst composition formed from palladium acetate, trifluoroacetic acid and ((2,2-dimethyl-1,3-dioxane-5,5-diyl)bis(methylene)bis(2-methoxyphenyl) phosphine). In the above, content of trifluoroacetic acid with respect to palladium is 10 times the molar ratio, and two stages of the first stage at a polymerization temperature of 74°C and the second stage at 84°C are carried out. In the prepared polyketone terpolymer, a molar ratio of ethylene to propene was 85:15. Also, a melting point of the polyketone terpolymer was 220°C, LVN measured at 25°C by HFIP (hexa-fluoroisopropanol) was 1.6 dl/g, and MWD was 2.0.

The polyketone terpolymer prepared above was molded into pellets on an extruder using a biaxial screw having a diameter of 40 mm and L/D=32, which was operated at 250 rpm, and then injection molded to produce a specimen for a sun visor retainer for automobiles.

Example 46

Linear alternating polyketone terpolymers comprising carbon monoxide and ethylene and propene are prepared under presence of catalyst composition formed from palladium acetate, trifluoroacetic acid and ((2,2-dimethyl-1,3-dioxane-5,5-diyl)bis(methylene)bis(2-methoxyphenyl) phosphine). In the above, content of trifluoroacetic acid with respect to palladium is 9 times the molar ratio, and two stages of the first stage at a polymerization temperature of 72°C and the second stage at 78°C are carried out. In the prepared polyketone terpolymer, a molar ratio of ethylene to propene was 85:15. Also, a melting point of the polyketone terpolymer was 220°C, LVN measured at 25°C by HFIP (hexa-fluoroisopropanol) was 2.0 dl/g, and MWD was 2.0.

The polyketone terpolymer prepared above was molded into pellets on an extruder using a biaxial screw having a diameter of 40 mm and L/D=32, which was operated at 250 rpm, and then injection molded to produce a specimen for a sun visor retainer for automobiles.

Comparative Example 13

Nylon 6 resin was molded into pellets on an extruder using a biaxial screw having a diameter of 40 mm and L/D=32, which was operated at 250 rpm, and then injection molded to produce a specimen for a sun visor retainer for automobiles.

Properties Evaluation

Coefficient of kinetic friction, wear property and impact strength of the specimens prepared in Example 44 to 46 and Comparative Example 13 respectively were evaluated by following method. Results are shown in Table 11.

Coefficient of kinetic friction is a value of a degree of kinetic friction. The larger the coefficient of kinetic friction, the greater the frictional force. The smaller the value, the smaller the frictional force.

Wear property is a value indicating a degree of wear. The larger the wear property, the smaller the wear resistance because wear occurs more easily. Meanwhile, the smaller the value, the greater the wear resistance. Wear property test was carried out in a pin-on-disk type under conditions of a load of 1 kg, a linear velocity of 7 Hz and a test time of 30 minutes.

In addition, a dimensional change rate was measured according to MS211-47 in vertical and horizontal directions at a temperature of 50°C and a relative humidity of 90%.

[Table 11]

| Item | Example 44 | Example 45 | Example 46 | Comparative Example 13 |
|---|---|---|---|---|
| Coefficient of kinetic friction | 0.10 | 0.15 | 0.12 | 0.40 |

(continued)

| Item | Example 44 | Example 45 | Example 46 | Comparative Example 13 |
|---|---|---|---|---|
| Wear property (Rmax) | 0.60 | 0.65 | 0.70 | 8.10 |
| Dimensional change rate (%) | 1.3 | 1.1 | 1.2 | 5.2 |

As can be seen in Table 11, the impact strength of the example was improved compared to the comparative example, and the coefficient of kinetic friction and the wear property were small, thereby the frictional force was small and the wear resistance was improved. Therefore, the sun visor retainer for automobiles manufactured by the examples of the present invention exhibits superior wear resistance and dimensional stability as compared with the comparative example used as a conventional sun visor retainer material for automobiles, thereby it is suitable for application as a sun visor retainer for automobiles.

Example 47

Linear alternating polyketone terpolymers comprising carbon monoxide and ethylene and propene are prepared under presence of catalyst composition formed from palladium acetate, trifluoroacetic acid and ((2,2-dimethyl-1,3-dioxane-5,5-diyl)bis(methylene)bis(2-methoxyphenyl) phosphine). In the above, content of trifluoroacetic acid with respect to palladium is 10 times the molar ratio, and two stages of the first stage at a polymerization temperature of 78°C and the second stage at 84°C are carried out. In the polyketone terpolymer prepared above, carbon monoxide was 50 mol%, ethylene was 46 mol%, and propylene was 4 mol%. Also, a melting point of the polyketone terpolymer was 220°C, LVN measured at 25°C by HFIP (hexa-fluoroisopropanol) was 1.4 dl/g, and MWD was 2.0.

70 weight% of the polyketone terpolymer prepared above and 30 weight% of glass fiber were inserted to produce a composition and the produced composition was molded into pellets on an extruder using a biaxial screw having a diameter of 40 cm and L/D=32, which was operated at 250 rpm, and then injection molded to produce a specimen for a door frame inner cover for automobiles.

Example 48

75 weight% of the polyketone terpolymer prepared in same method as Example 47 and 25 weight% of glass fiber were inserted to produce a composition and the produced composition was molded into pellets on an extruder using a biaxial screw having a diameter of 40cm and L/D=32, which was operated at 250 rpm, and then injection molded to produce a specimen for a door frame inner cover for automobiles.

Example 49

80 weight% of the polyketone terpolymer prepared in same method as Example 47 and 20 weight% of glass fiber were inserted to produce a composition and the produced composition was molded into pellets on an extruder using a biaxial screw having a diameter of 40 cm and L/D=32, which was operated at 250 rpm, and then injection molded to produce a specimen for a door frame inner cover for automobiles.

Example 50

85 weight% of the polyketone terpolymer prepared in same method as Example 47 and 15 weight% of glass fiber were inserted to produce a composition and the produced composition was molded into pellets on an extruder using a biaxial screw having a diameter of 40 cm and L/D=32, which was operated at 250 rpm, and then injection molded to produce a specimen for a door frame inner cover for automobiles.

Comparative Example 14

A nylon 66 resin and 30 weight% of glass fiber were inserted to produce a composition and the produced composition was molded into pellets on an extruder using a biaxial screw having a diameter of 40 cm and L/D=32, which was operated at 250 rpm, and then injection molded to produce a specimen for a door frame inner cover for automobiles.

Properties Evaluation

Properties of the specimens prepared in Example 47 to 50 and Comparative Example 14 were evaluated in following method. Results are shown in Table 12.

1. Izod impact strength evaluation: Performed according to ASTM D256.
2. Dimensional change rate evaluation: Evaluation was made according to MS211-47 for vertical and horizontal directions at a temperature of 50°C and a relative humidity of 90 %.

[Table 12]

| Item | Example 47 | Example 48 | Example 49 | Example 50 | Comparative Example 14 |
|---|---|---|---|---|---|
| impact strength (kJ/$m^2$) | 25 | 23 | 28 | 27 | 18 |
| Dimensional change rate (%) | 1.3 | 1.1 | 1.2 | 1.5 | 5.2 |

As can be seen in Table 12, the impact strength and dimensional stability of the Examples were improved compared with Comparative Examples. Therefore, the door frame inner cover for automobiles manufactured by the examples of the present invention shows better dimensional stability than a conventional material of door frame inner cover for automobiles, thereby it is suitable for application as a door frame inner cover for automobiles.

Example 51

Linear alternating polyketone terpolymers comprising carbon monoxide and ethylene and propene are prepared under presence of catalyst composition formed from palladium acetate, trifluoroacetic acid and ((2,2-dimethyl-1,3-dioxane-5,5-diyl)bis(methylene)bis(2-methoxyphenyl) phosphine). In the above, content of trifluoroacetic acid with respect to palladium is 11 times the molar ratio, and two stages of the first stage at a polymerization temperature of 78°C and the second stage at 84°C are carried out. In the prepared polyketone terpolymer, a molar ratio of ethylene to propene was 85:15. Also, a melting point of the polyketone terpolymer was 220°C, LVN measured at 25°C by HFIP (hexafluoroisopropanol) was 1.4 dl/g, and MWD was 2.0.

The polyketone terpolymer prepared above was molded into pellets on an extruder using a biaxial screw having a diameter of 40 mm and L/D=32, which was operated at 250 rpm, and then injection molded to produce a specimen for a safety belt jointer for automobiles.

Example 52

Linear alternating polyketone terpolymers comprising carbon monoxide and ethylene and propene are prepared under presence of catalyst composition formed from palladium acetate, trifluoroacetic acid and ((2,2-dimethyl-1,3-dioxane-5,5-diyl)bis(methylene)bis(2-methoxyphenyl) phosphine). In the above, content of trifluoroacetic acid with respect to palladium is 11 times the molar ratio, and two stages of the first stage at a polymerization temperature of 78°C and the second stage at 84°C are carried out. In the prepared polyketone terpolymer, a molar ratio of ethylene to propene was 85:15. Also, a melting point of the polyketone terpolymer was 220°C, LVN measured at 25°C by HFIP (hexafluoroisopropanol) was 1.4 dl/g, and MWD was 2.0.

The polyketone terpolymer prepared above and a silicon-based wear resistant agent were inserted to produce a composition and the produced composition was molded into pellets on an extruder using a biaxial screw having a diameter of 40 mm and L/D=32, which was operated at 250 rpm, and then injection molded to produce a specimen for a safety belt jointer for automobiles.

Example 53

Linear alternating polyketone terpolymers comprising carbon monoxide and ethylene and propene are prepared under presence of catalyst composition formed from palladium acetate, trifluoroacetic acid and ((2,2-dimethyl-1,3-dioxane-5,5-diyl)bis(methylene)bis(2-methoxyphenyl) phosphine). In the above, content of trifluoroacetic acid with respect to palladium is 11 times the molar ratio, and two stages of the first stage at a polymerization temperature of 78°C and the second stage at 84°C are carried out. In the prepared polyketone terpolymer, a molar ratio of ethylene to propene was 85:15. Also, a melting point of the polyketone terpolymer was 220°C, LVN measured at 25°C by HFIP (hexa-fluoroisopropanol) was 1.4 dl/g, and MWD was 2.0.

The polyketone terpolymer prepared above and a glass fiber were inserted to produce a composition and the produced composition was molded into pellets on an extruder using a biaxial screw having a diameter of 40 mm and L/D=32, which was operated at 250 rpm, and then injection molded to produce a specimen for a safety belt jointer for automobiles.

Example 54

Linear alternating polyketone terpolymers comprising carbon monoxide and ethylene and propene are prepared under presence of catalyst composition formed from palladium acetate, trifluoroacetic acid and ((2,2-dimethyl-1,3-dioxane-5,5-diyl)bis(methylene)bis(2-methoxyphenyl) phosphine). In the above, content of trifluoroacetic acid with respect to palladium is 11 times the molar ratio, and two stages of the first stage at a polymerization temperature of 78°C and the second stage at 84°C are carried out. In the prepared polyketone terpolymer, a molar ratio of ethylene to propene was 85:15. Also, a melting point of the polyketone terpolymer was 220°C, LVN measured at 25°C by HFIP (hexafluoroisopropanol) was 1.4 dl/g, and MWD was 2.0.

The polyketone terpolymer prepared above, a glass fiber and a polytetrafluoroethylene resin were inserted to produce a composition and the produced composition was molded into pellets on an extruder using a biaxial screw having a diameter of 40 mm and L/D=32, which was operated at 250 rpm, and then injection molded to produce a specimen for a safety belt jointer for automobiles.

Comparative Example 15

A nylon 66 resin was molded into pellets on an extruder using a biaxial screw having a diameter of 40 mm and L/D=32, which was operated at 250 rpm, and then injection molded to produce a specimen for a safety belt jointer for automobiles.

Properties Evaluation

Coefficient of kinetic friction, wear property and impact strength of the specimens prepared in Example 51 to 54 and Comparative Example 15 respectively were evaluated by following method. Results are shown in Table 13.

1. Izod impact strength evaluation: Performed according to ASTM D256.
2. Friction coefficient: Coefficient of kinetic friction is a value that can represent a degree of kinetic friction. The larger the coefficient of kinetic friction means the greater the frictional force, and the smaller the value, the smaller the frictional force.
3. Wear property evaluation: Performed according to JIS K7218 (test condition: 3 km wear at 50 rmp and 150 N)

[Table 13]

| Item | Example 51 | Example 52 | Example 53 | Example 54 | Comparative Example 15 |
|---|---|---|---|---|---|
| impact strength ($kJ/m^2$) | 11 | 10 | 14 | 14 | 5 |
| Friction coefficient | 0.34 | 0.14 | 0.3 | 0.25 | 0.35 |
| Wear property (g) | 0.009 | 0.004 | 0.006 | 0.003 | 0.007 |

As can be seen in Table 13, the impact strength of the example was improved compared to the comparative example, and the coefficient of kinetic friction and the wear property were small, thereby the frictional force was small and the wear resistance was improved. Therefore, the safety belt jointer for automobiles manufactured by the examples of the present invention exhibits superior wear resistance and impact strength as compared with the comparative example used as a conventional material for a safety belt jointer for automobiles, thereby it is suitable for application as a safety belt jointer for automobiles.

Example 55

Linear alternating polyketone terpolymers comprising carbon monoxide and ethylene and propene are prepared under presence of catalyst composition formed from palladium acetate, trifluoroacetic acid and ((2,2-dimethyl-1,3-dioxane-5,5-diyl)bis(methylene)bis(2-methoxyphenyl) phosphine). In the above, content of trifluoroacetic acid with respect to palladium is 11 times the molar ratio, and two stages of the first stage at a polymerization temperature of 78°C and the second stage at 84°C are carried out. In the prepared polyketone terpolymer, a molar ratio of ethylene to propene was 85:15. Also, a melting point of the polyketone terpolymer was 220°C, LVN measured at 25°C by HFIP (hexafluoroisopropanol) was 1.4 dl/g, and MWD was 2.0.

The polyketone terpolymer prepared above was molded into pellets on an extruder using a biaxial screw having a diameter of 40 mm and L/D=32, which was operated at 250 rpm, and then injection molded to produce a specimen for an auto gear slide for automobiles.

Example 56

Linear alternating polyketone terpolymers comprising carbon monoxide and ethylene and propene are prepared under presence of catalyst composition formed from palladium acetate, trifluoroacetic acid and ((2,2-dimethyl-1,3-dioxane-5,5-diyl)bis(methylene)bis(2-methoxyphenyl) phosphine). In the above, content of trifluoroacetic acid with respect to palladium is 11 times the molar ratio, and two stages of the first stage at a polymerization temperature of 78°C and the second stage at 84°C are carried out. In the prepared polyketone terpolymer, a molar ratio of ethylene to propene was 85:15. Also, a melting point of the polyketone terpolymer was 220°C, LVN measured at 25°C by HFIP (hexafluoroisopropanol) was 1.4 dl/g, and MWD was 2.0.

The polyketone terpolymer prepared above and a silicon-based wear resistant agent were inserted to produce a composition and the produced composition was molded into pellets on an extruder using a biaxial screw having a diameter of 40 mm and L/D=32, which was operated at 250 rpm, and then injection molded to produce a specimen for an auto gear slide for automobiles.

Example 57

Linear alternating polyketone terpolymers comprising carbon monoxide and ethylene and propene are prepared under presence of catalyst composition formed from palladium acetate, trifluoroacetic acid and ((2,2-dimethyl-1,3-dioxane-5,5-diyl)bis(methylene)bis(2-methoxyphenyl) phosphine). In the above, content of trifluoroacetic acid with respect to palladium is 11 times the molar ratio, and two stages of the first stage at a polymerization temperature of 78°C and the second stage at 84°C are carried out. In the prepared polyketone terpolymer, a molar ratio of ethylene to propene was 85:15. Also, a melting point of the polyketone terpolymer was 220°C, LVN measured at 25°C by HFIP (hexafluoroisopropanol) was 1.4 dl/g, and MWD was 2.0.

The polyketone terpolymer prepared above and a glass fiber were inserted to produce a composition and the produced composition was molded into pellets on an extruder using a biaxial screw having a diameter of 40 mm and L/D=32, which was operated at 250 rpm, and then injection molded to produce a specimen for an auto gear slide for automobiles.

Example 58

Linear alternating polyketone terpolymers comprising carbon monoxide and ethylene and propene are prepared under presence of catalyst composition formed from palladium acetate, trifluoroacetic acid and ((2,2-dimethyl-1,3-dioxane-5,5-diyl)bis(methylene)bis(2-methoxyphenyl) phosphine). In the above, content of trifluoroacetic acid with respect to palladium is 11 times the molar ratio, and two stages of the first stage at a polymerization temperature of 78°C and the second stage at 84°C are carried out. In the prepared polyketone terpolymer, a molar ratio of ethylene to propene was 85:15. Also, a melting point of the polyketone terpolymer was 220°C, LVN measured at 25°C by HFIP (hexafluoroisopropanol) was 1.4 dl/g, and MWD was 2.0.

The polyketone terpolymer prepared above, a glass fiber and a polytetrafluoroethylene resin were inserted to produce a composition and the produced composition was molded into pellets on an extruder using a biaxial screw having a diameter of 40 mm and L/D=32, which was operated at 250 rpm, and then injection molded to produce a specimen for an auto gear slide for automobiles.

Comparative Example 16

A polyester elastomer (PEL) was molded into pellets on an extruder using a biaxial screw having a diameter of 40 cm and L/D=32, which was operated at 250 rpm, and then injection molded to produce a specimen for an auto gear slide for automobiles.

Properties Evaluation

Properties of the specimens prepared in Example 55 to 58 and Comparative Example 16 were evaluated in following method and results are shown in Table 6.

1. Friction coefficient: Coefficient of kinetic friction is a value that can represent a degree of kinetic friction. The larger the coefficient of kinetic friction means the greater the frictional force, and the smaller the value, the smaller the frictional force.
2. Wear property evaluation: Performed according to JIS K7218 (test condition: 3 km wear at 50 rmp and 150 N)

[Table 14]

| Item | Example 55 | Example 56 | Example 57 | Example 58 | Comparative Example 16 |
|---|---|---|---|---|---|
| Coefficient of kinetic friction | 0.34 | 0.14 | 0.3 | 0.25 | 0.4 |
| Wear property (g) | 0.009 | 0.004 | 0.006 | 0.003 | 0.081 |

As can be seen in Table 14, the coefficient of kinetic friction and the wear property of the Examples were smaller than those of the Comparative Example, thereby the frictional force was small and the wear resistance was improved. Therefore, the auto gear slide for automobiles manufactured by the examples of the present invention shows better wear resistance than the comparative example used for a conventional material of auto gear slide for automobiles.

Example 59

Linear alternating polyketone terpolymers comprising carbon monoxide and ethylene and propene are prepared under presence of catalyst composition formed from palladium acetate, trifluoroacetic acid and ((2,2-dimethyl-1,3-dioxane-5,5-diyl)bis(methylene)bis(2-methoxyphenyl) phosphine). In the above, content of trifluoroacetic acid with respect to palladium is 11 times the molar ratio, and two stages of the first stage at a polymerization temperature of 78°C and the second stage at 84°C are carried out. In the prepared polyketone terpolymer, a molar ratio of ethylene to propene was 85:15. Also, a melting point of the polyketone terpolymer was 220°C, LVN measured at 25°C by HFIP (hexa-fluoroisopropanol) was 1.4 dl/g, and MWD was 2.0.

The polyketone terpolymer prepared above was molded into pellets on an extruder using a biaxial screw having a diameter of 40 mm and L/D=32, which was operated at 250 rpm, and then injection molded to produce a specimen for a door latch housing for automobiles.

Example 60

Linear alternating polyketone terpolymers comprising carbon monoxide and ethylene and propene are prepared under presence of catalyst composition formed from palladium acetate, trifluoroacetic acid and ((2,2-dimethyl-1,3-dioxane-5,5-diyl)bis(methylene)bis(2-methoxyphenyl) phosphine). In the above, content of trifluoroacetic acid with respect to palladium is 10 times the molar ratio, and two stages of the first stage at a polymerization temperature of 74°C and the second stage at 84°C are carried out. In the prepared polyketone terpolymer, a molar ratio of ethylene to propene was 85:15. Also, a melting point of the polyketone terpolymer was 220°C, LVN measured at 25°C by HFIP (hexa-fluoroisopropanol) was 1.6 dl/g, and MWD was 2.0.

The polyketone terpolymer prepared above was molded into pellets on an extruder using a biaxial screw having a diameter of 40 mm and L/D=32, which was operated at 250 rpm, and then injection molded to produce a specimen for a door latch housing for automobiles.

Example 61

Linear alternating polyketone terpolymers comprising carbon monoxide and ethylene and propene are prepared under presence of catalyst composition formed from palladium acetate, trifluoroacetic acid and ((2,2-dimethyl-1,3-dioxane-5,5-diyl)bis(methylene)bis(2-methoxyphenyl) phosphine). In the above, content of trifluoroacetic acid with respect to palladium is 9 times the molar ratio, and two stages of the first stage at a polymerization temperature of 72°C and the second stage at 78°C are carried out. In the prepared polyketone terpolymer, a molar ratio of ethylene to propene was 85:15. Also, a melting point of the polyketone terpolymer was 220°C, LVN measured at 25°C by HFIP (hexa-fluoroisopropanol) was 2.0 dl/g, and MWD was 2.0.

The polyketone terpolymer prepared above was molded into pellets on an extruder using a biaxial screw having a diameter of 40 mm and L/D=32, which was operated at 250 rpm, and then injection molded to produce a specimen for a door latch housing for automobiles.

Example 62

Linear alternating polyketone terpolymers comprising carbon monoxide and ethylene and propene are prepared under presence of catalyst composition formed from palladium acetate, trifluoroacetic acid and ((2,2-dimethyl-1,3-dioxane-5,5-diyl)bis(methylene)bis(2-methoxyphenyl) phosphine). In the above, content of trifluoroacetic acid with respect to palladium is 11 times the molar ratio, and two stages of the first stage at a polymerization temperature of 78°C and the second stage at 84°C are carried out. In the prepared polyketone terpolymer, a molar ratio of ethylene to propene was 85:15. Also, a melting point of the polyketone terpolymer was 220°C, LVN measured at 25°C by HFIP (hexafluoroisopropanol) was 1.4 dl/g, and MWD was 2.0.

The polyketone terpolymer prepared above and a silicon-based wear resistant agent were molded into pellets on an extruder using a biaxial screw having a diameter of 40 mm and L/D=32, which was operated at 250 rpm, and then injection molded to produce a specimen for a door latch housing for automobiles.

Comparative Example 17

A polyoxymethylene resin was molded into pellets on an extruder using a biaxial screw having a diameter of 40 cm and L/D=32, which was operated at 250 rpm, and then injection molded to produce a specimen for a door latch housing for automobiles.

Properties Evaluation

Properties of the specimens prepared in Example 59 to 62 and Comparative Example 17 were evaluated in following method and results are shown in Table 15.

1. Izod impact strength evaluation: Performed according to ASTM D256.
2. Friction coefficient: Coefficient of kinetic friction is a value that can represent a degree of kinetic friction. The larger the coefficient of kinetic friction means the greater the frictional force, and the smaller the value, the smaller the frictional force.
3. Wear property evaluation: Performed according to JIS K7218 (test condition: 3 km wear at 50 rmp and 150 N)

[Table 15]

| Item | Example 59 | Example 60 | Example 61 | Example 62 | Comparative Example 17 |
|---|---|---|---|---|---|
| impact strength ($kJ/m^2$) | 11 | 12 | 11 | 10 | 6 |
| Friction coefficient | 0.34 | 0.33 | 0.35 | 0.14 | 0.15 |
| Wear property (g) | 0.009 | 0.008 | 0.010 | 0.004 | 0.191 |

As can be seen in Table 15, the impact strength of the Examples was improved compared to Comparative Example 16. Also, the coefficient of kinetic friction and the wear property of the Examples were smaller than those of the Comparative Example 17, thereby the frictional force was small and the wear resistance was improved. Therefore, the door latch housing for automobiles manufactured by the examples of the present invention shows better impact resistance and wear resistance than the comparative example used for a conventional material of door latch housing for automobiles, thereby it is suitable for application as a door latch housing for automobiles.

Example 63

Linear alternating polyketone terpolymers comprising carbon monoxide and ethylene and propene are prepared under presence of catalyst composition formed from palladium acetate, trifluoroacetic acid and ((2,2-dimethyl-1,3-dioxane-5,5-diyl)bis(methylene)bis(2-methoxyphenyl) phosphine). In the above, content of trifluoroacetic acid with respect to palladium is 11 times the molar ratio, and two stages of the first stage at a polymerization temperature of 78°C and

the second stage at 84°C are carried out. In the prepared polyketone terpolymer, a molar ratio of ethylene to propene was 85:15. Also, a melting point of the polyketone terpolymer was 220°C, LVN measured at 25°C by HFIP (hexa-fluoroisopropanol) was 1.4 dl/g, and MWD was 2.0.

The polyketone terpolymer prepared above was molded into pellets on an extruder using a biaxial screw having a diameter of 40 mm and L/D=32, which was operated at 250 rpm, and then injection molded to produce a specimen for a slide guide for automobiles.

Example 64

Linear alternating polyketone terpolymers comprising carbon monoxide and ethylene and propene are prepared under presence of catalyst composition formed from palladium acetate, trifluoroacetic acid and ((2,2-dimethyl-1,3-dioxane-5,5-diyl)bis(methylene)bis(2-methoxyphenyl) phosphine). In the above, content of trifluoroacetic acid with respect to palladium is 10 times the molar ratio, and two stages of the first stage at a polymerization temperature of 74°C and the second stage at 84°C are carried out. In the prepared polyketone terpolymer, a molar ratio of ethylene to propene was 85:15. Also, a melting point of the polyketone terpolymer was 220°C, LVN measured at 25°C by HFIP (hexa-fluoroisopropanol) was 1.6 dl/g, and MWD was 2.0.

The polyketone terpolymer prepared above was molded into pellets on an extruder using a biaxial screw having a diameter of 40 mm and L/D=32, which was operated at 250 rpm, and then injection molded to produce a specimen for a slide guide for automobiles.

Example 65

Linear alternating polyketone terpolymers comprising carbon monoxide and ethylene and propene are prepared under presence of catalyst composition formed from palladium acetate, trifluoroacetic acid and ((2,2-dimethyl-1,3-dioxane-5,5-diyl)bis(methylene)bis(2-methoxyphenyl) phosphine). In the above, content of trifluoroacetic acid with respect to palladium is 9 times the molar ratio, and two stages of the first stage at a polymerization temperature of 72°C and the second stage at 78°C are carried out. In the prepared polyketone terpolymer, a molar ratio of ethylene to propene was 85:15. Also, a melting point of the polyketone terpolymer was 220°C, LVN measured at 25°C by HFIP (hexa-fluoroisopropanol) was 2.0 dl/g, and MWD was 2.0.

The polyketone terpolymer prepared above was molded into pellets on an extruder using a biaxial screw having a diameter of 40 mm and L/D=32, which was operated at 250 rpm, and then injection molded to produce a specimen for a slide guide for automobiles.

Example 66

Linear alternating polyketone terpolymers comprising carbon monoxide and ethylene and propene are prepared under presence of catalyst composition formed from palladium acetate, trifluoroacetic acid and ((2,2-dimethyl-1,3-dioxane-5,5-diyl)bis(methylene)bis(2-methoxyphenyl) phosphine). In the above, content of trifluoroacetic acid with respect to palladium is 11 times the molar ratio, and two stages of the first stage at a polymerization temperature of 78°C and the second stage at 84°C are carried out. In the prepared polyketone terpolymer, a molar ratio of ethylene to propene was 85:15. Also, a melting point of the polyketone terpolymer was 220°C, LVN measured at 25°C by HFIP (hexa-fluoroisopropanol) was 1.4 dl/g, and MWD was 2.0.

The polyketone terpolymer prepared above and a silicon-based wear resistant agent were molded into pellets on an extruder using a biaxial screw having a diameter of 40 mm and L/D=32, which was operated at 250 rpm, and then injection molded to produce a specimen for a slide guide for automobiles.

Comparative Example 18

A polyoxymethylene resin was molded into pellets on an extruder using a biaxial screw having a diameter of 40 cm and L/D=32, which was operated at 250 rpm, and then injection molded to produce a specimen for a slide guide for automobiles.

Properties Evaluation

Properties of the specimens prepared in Example 63 to 66 and Comparative Example 18 were evaluated in following method and results are shown in Table 16.

1. Izod impact strength evaluation: Performed according to ASTM D256.

2. Friction coefficient: Coefficient of kinetic friction is a value that can represent a degree of kinetic friction. The larger the coefficient of kinetic friction means the greater the frictional force, and the smaller the value, the smaller the frictional force.
3. Wear property evaluation: Performed according to JIS K7218 (test condition: 3 km wear at 50 rmp and 150 N)

[Table 16]

| Item | Example 63 | Example 64 | Example 65 | Example 66 | Comparative Example 18 |
|---|---|---|---|---|---|
| impact strength (kJ/$m^2$) | 11 | 12 | 11 | 10 | 6 |
| Friction coefficient | 0.34 | 0.33 | 0.35 | 0.14 | 0.15 |
| Wear property (g) | 0.009 | 0.008 | 0.010 | 0.004 | 0.191 |

As can be seen in Table 16, the impact strength of the Examples was improved compared to Comparative Example 18. Also, the coefficient of kinetic friction and the wear property of the Examples were smaller than those of the Comparative Example 18, thereby the frictional force was small and the wear resistance was improved. Therefore, the slide guide for automobiles manufactured by the examples of the present invention shows better impact resistance and wear resistance than the comparative example used for a conventional material of slide guide for automobiles, thereby it is suitable for application as a slide guide for automobiles.

Example 67

Linear alternating polyketone terpolymers comprising carbon monoxide and ethylene and propene are prepared under presence of catalyst composition formed from palladium acetate, trifluoroacetic acid and ((2,2-dimethyl-1,3-dioxane-5,5-diyl)bis(methylene)bis(2-methoxyphenyl) phosphine). In the above, content of trifluoroacetic acid with respect to palladium is 11 times the molar ratio, and two stages of the first stage at a polymerization temperature of 78°C and the second stage at 84°C are carried out. In the prepared polyketone terpolymer, a molar ratio of ethylene to propene was 85:15. Also, a melting point of the polyketone terpolymer was 220°C, LVN measured at 25°C by HFIP (hexafluoroisopropanol) was 1.4 dl/g, and MWD was 2.0.
The polyketone terpolymer prepared above was molded into pellets on an extruder using a biaxial screw having a diameter of 40 mm and L/D=32, which was operated at 250 rpm, and then injection molded to produce a specimen for a switch shaft in heating, ventilation and air conditioning (HAVC) systems for automobiles.

Example 68

Linear alternating polyketone terpolymers comprising carbon monoxide and ethylene and propene are prepared under presence of catalyst composition formed from palladium acetate, trifluoroacetic acid and ((2,2-dimethyl-1,3-dioxane-5,5-diyl)bis(methylene)bis(2-methoxyphenyl) phosphine). In the above, content of trifluoroacetic acid with respect to palladium is 11 times the molar ratio, and two stages of the first stage at a polymerization temperature of 78°C and the second stage at 84°C are carried out. In the prepared polyketone terpolymer, a molar ratio of ethylene to propene was 85:15. Also, a melting point of the polyketone terpolymer was 220°C, LVN measured at 25°C by HFIP (hexafluoroisopropanol) was 1.4 dl/g, and MWD was 2.0.
The polyketone terpolymer prepared above and a silicon-based wear resistant agent were inserted to produce a composition and the produced composition was molded into pellets on an extruder using a biaxial screw having a diameter of 40 mm and L/D=32, which was operated at 250 rpm, and then injection molded to produce a specimen for a switch shaft in heating, ventilation and air conditioning (HAVC) systems for automobiles.

Example 69

Linear alternating polyketone terpolymers comprising carbon monoxide and ethylene and propene are prepared under presence of catalyst composition formed from palladium acetate, trifluoroacetic acid and ((2,2-dimethyl-1,3-dioxane-5,5-diyl)bis(methylene)bis(2-methoxyphenyl) phosphine). In the above, content of trifluoroacetic acid with respect to palladium is 11 times the molar ratio, and two stages of the first stage at a polymerization temperature of 78°C and the second stage at 84°C are carried out. In the prepared polyketone terpolymer, a molar ratio of ethylene to propene was 85:15. Also, a melting point of the polyketone terpolymer was 220°C, LVN measured at 25°C by HFIP (hexafluoroisopropanol) was 1.4 dl/g, and MWD was 2.0.

The polyketone terpolymer prepared above, a glass fiber were inserted to produce a composition and the produced composition was molded into pellets on an extruder using a biaxial screw having a diameter of 40 mm and L/D=32, which was operated at 250 rpm, and then injection molded to produce a specimen for a switch shaft in heating, ventilation and air conditioning (HAVC) systems for automobiles.

Example 70

Linear alternating polyketone terpolymers comprising carbon monoxide and ethylene and propene are prepared under presence of catalyst composition formed from palladium acetate, trifluoroacetic acid and ((2,2-dimethyl-1,3-dioxane-5,5-diyl)bis(methylene)bis(2-methoxyphenyl) phosphine). In the above, content of trifluoroacetic acid with respect to palladium is 11 times the molar ratio, and two stages of the first stage at a polymerization temperature of 78°C and the second stage at 84°C are carried out. In the prepared polyketone terpolymer, a molar ratio of ethylene to propene was 85:15. Also, a melting point of the polyketone terpolymer was 220°C, LVN measured at 25°C by HFIP (hexafluoroisopropanol) was 1.4 dl/g, and MWD was 2.0.

The polyketone terpolymer prepared above, a glass fiber and a polytetrafluoroethylene resin were inserted to produce a composition and the produced composition was molded into pellets on an extruder using a biaxial screw having a diameter of 40 mm and L/D=32, which was operated at 250 rpm, and then injection molded to produce a specimen for a switch shaft in heating, ventilation and air conditioning (HAVC) systems for automobiles.

Comparative Example 19

A polyoxymethylene resin was molded into pellets on an extruder using a biaxial screw having a diameter of 40 cm and L/D=32, which was operated at 250 rpm, and then injection molded to produce a specimen for a switch shaft in heating, ventilation and air conditioning (HAVC) systems for automobiles.

Properties Evaluation

Properties of the specimens prepared in Example 67 to 70 and Comparative Example 19 were evaluated in following method and results are shown in Table 17.

1. Izod impact strength evaluation: Performed according to ASTM D256.
2. Friction coefficient: Coefficient of kinetic friction is a value that can represent a degree of kinetic friction. The larger the coefficient of kinetic friction means the greater the frictional force, and the smaller the value, the smaller the frictional force.
3. Wear property evaluation: Performed according to JIS K7218(test condition: 3km wear at 50rmp and 150N)

[Table 17]

| Item | Example 67 | Example 68 | Example 69 | Example 70 | Comparative Example 19 |
|---|---|---|---|---|---|
| impact strength ($kJ/m^2$) | 11 | 10 | 14 | 14 | 6 |
| Friction coefficient | 0.34 | 0.14 | 0.3 | 0.25 | 0.15 |
| Wear property (g) | 0.009 | 0.004 | 0.006 | 0.003 | 0.191 |

As can be seen in Table 17, the impact strength of the Examples was improved compared to Comparative Example 19. Also, the coefficient of kinetic friction and the wear property of the Examples were smaller than those of the Comparative Example 19, thereby the frictional force was small and the wear resistance was improved. Therefore, the switch shaft in heating, ventilation and air conditioning (HAVC) systems for automobiles manufactured by the examples of the present invention shows better impact resistance and wear resistance than the comparative example used for a conventional material of switch shaft in heating, ventilation and air conditioning (HAVC) systems for automobiles, thereby it is suitable for application as a switch shaft in heating, ventilation and air conditioning (HAVC) systems for automobiles.

Example 71

Linear alternating polyketone terpolymers comprising carbon monoxide and ethylene and propene are prepared under presence of catalyst composition formed from palladium acetate, trifluoroacetic acid and ((2,2-dimethyl-1,3-dioxane-5,5-diyl)bis(methylene)bis(2-methoxyphenyl) phosphine). In the above, content of trifluoroacetic acid with respect to palladium is 11 times the molar ratio, and two stages of the first stage at a polymerization temperature of 78°C and the second stage at 84°C are carried out. In the prepared polyketone terpolymer, a molar ratio of ethylene to propene was 85:15. Also, a melting point of the polyketone terpolymer was 220°C, LVN measured at 25°C by HFIP (hexafluoroisopropanol) was 1.4 dl/g, and MWD was 2.0.

The polyketone terpolymer prepared above was molded into pellets on an extruder using a biaxial screw having a diameter of 40 mm and L/D=32, which was operated at 250 rpm, and then injection molded to produce a specimen for an actuator gear for automobiles.

Example 72

Linear alternating polyketone terpolymers comprising carbon monoxide and ethylene and propene are prepared under presence of catalyst composition formed from palladium acetate, trifluoroacetic acid and ((2,2-dimethyl-1,3-dioxane-5,5-diyl)bis(methylene)bis(2-methoxyphenyl) phosphine). In the above, content of trifluoroacetic acid with respect to palladium is 11 times the molar ratio, and two stages of the first stage at a polymerization temperature of 78°C and the second stage at 84°C are carried out. In the prepared polyketone terpolymer, a molar ratio of ethylene to propene was 85:15. Also, a melting point of the polyketone terpolymer was 220°C, LVN measured at 25°C by HFIP (hexafluoroisopropanol) was 1.4 dl/g, and MWD was 2.0.

The polyketone terpolymer prepared above and a silicon-based wear resistant agent were inserted to produce a composition and the produced composition was molded into pellets on an extruder using a biaxial screw having a diameter of 40 mm and L/D=32, which was operated at 250 rpm, and then injection molded to produce a specimen for an actuator gear for automobiles.

Example 73

Linear alternating polyketone terpolymers comprising carbon monoxide and ethylene and propene are prepared under presence of catalyst composition formed from palladium acetate, trifluoroacetic acid and ((2,2-dimethyl-1,3-dioxane-5,5-diyl)bis(methylene)bis(2-methoxyphenyl) phosphine). In the above, content of trifluoroacetic acid with respect to palladium is 11 times the molar ratio, and two stages of the first stage at a polymerization temperature of 78°C and the second stage at 84°C are carried out. In the prepared polyketone terpolymer, a molar ratio of ethylene to propene was 85:15. Also, a melting point of the polyketone terpolymer was 220°C, LVN measured at 25°C by HFIP (hexafluoroisopropanol) was 1.4 dl/g, and MWD was 2.0.

The polyketone terpolymer prepared above and a glass fiber were inserted to produce a composition and the produced composition was molded into pellets on an extruder using a biaxial screw having a diameter of 40 mm and L/D=32, which was operated at 250 rpm, and then injection molded to produce a specimen for an actuator gear for automobiles.

Example 74

Linear alternating polyketone terpolymers comprising carbon monoxide and ethylene and propene are prepared under presence of catalyst composition formed from palladium acetate, trifluoroacetic acid and ((2,2-dimethyl-1,3-dioxane-5,5-diyl)bis(methylene)bis(2-methoxyphenyl) phosphine). In the above, content of trifluoroacetic acid with respect to palladium is 11 times the molar ratio, and two stages of the first stage at a polymerization temperature of 78°C and the second stage at 84°C are carried out. In the prepared polyketone terpolymer, a molar ratio of ethylene to propene was 85:15. Also, a melting point of the polyketone terpolymer was 220°C, LVN measured at 25°C by HFIP (hexafluoroisopropanol) was 1.4 dl/g, and MWD was 2.0.

The polyketone terpolymer prepared above, a glass fiber and a polytetrafluoroethylene resin were inserted to produce a composition and the produced composition was molded into pellets on an extruder using a biaxial screw having a diameter of 40 mm and L/D=32, which was operated at 250 rpm, and then injection molded to produce a specimen for an actuator gear for automobiles.

Comparative Example 20

A polyoxymethylene resin was molded into pellets on an extruder using a biaxial screw having a diameter of 40 cm and L/D=32, which was operated at 250 rpm, and then injection molded to produce a specimen for an actuator gear for automobiles.

Properties Evaluation

Properties of the specimens prepared in Example 71 to 74 and Comparative Example 20 were evaluated in following method and results are shown in Table 18.

1. Izod impact strength evaluation: Performed according to ASTM D256.
2. Friction coefficient: Coefficient of kinetic friction is a value that can represent a degree of kinetic friction. The larger the coefficient of kinetic friction means the greater the frictional force, and the smaller the value, the smaller the frictional force.
3. Wear property evaluation: Performed according to JIS K7218 (test condition: 3 km wear at 50 rmp and 150 N)

[Table 18]

| Item | Example 71 | Example 72 | Example 73 | Example 74 | Comparative Example 20 |
|---|---|---|---|---|---|
| impact strength (kJ/m$^2$) | 11 | 10 | 14 | 14 | 6 |
| Friction coefficient | 0.34 | 0.14 | 0.3 | 0.25 | 0.15 |
| Wear property (g) | 0.009 | 0.004 | 0.006 | 0.003 | 0.191 |

As can be seen in Table 18, the impact strength of the Examples was improved compared to Comparative Example 20. Also, the coefficient of kinetic friction and the wear property of the Examples were smaller than those of the Comparative Example 20, thereby the frictional force was small and the wear resistance was improved. Therefore, the actuator gear for automobiles manufactured by the examples of the present invention shows better impact resistance and wear resistance than the comparative example used for a conventional material of actuator gear for automobiles, thereby it is suitable for application as an actuator gear for automobiles.

Example 75

Linear alternating polyketone terpolymers comprising carbon monoxide and ethylene and propene are prepared under presence of catalyst composition formed from palladium acetate, trifluoroacetic acid and ((2,2-dimethyl-1,3-dioxane-5,5-diyl)bis(methylene)bis(2-methoxyphenyl) phosphine). In the above, content of trifluoroacetic acid with respect to palladium is 11 times the molar ratio, and two stages of the first stage at a polymerization temperature of 78°C and the second stage at 84°C are carried out. In the prepared polyketone terpolymer, a molar ratio of ethylene to propene was 85:15. Also, a melting point of the polyketone terpolymer was 220°C, LVN measured at 25°C by HFIP (hexafluoroisopropanol) was 1.4 dl/g, and MWD was 2.0.

The polyketone terpolymer prepared above was molded into pellets on an extruder using a biaxial screw having a diameter of 40 mm and L/D=32, which was operated at 250 rpm, and then injection molded to produce a specimen for a trim mounting clip for automobiles.

Example 76

Linear alternating polyketone terpolymers comprising carbon monoxide and ethylene and propene are prepared under presence of catalyst composition formed from palladium acetate, trifluoroacetic acid and ((2,2-dimethyl-1,3-dioxane-5,5-diyl)bis(methylene)bis(2-methoxyphenyl) phosphine). In the above, content of trifluoroacetic acid with respect to palladium is 11 times the molar ratio, and two stages of the first stage at a polymerization temperature of 78°C and the second stage at 84°C are carried out. In the prepared polyketone terpolymer, a molar ratio of ethylene to propene was 85:15. Also, a melting point of the polyketone terpolymer was 220°C, LVN measured at 25°C by HFIP (hexafluoroisopropanol) was 1.4 dl/g, and MWD was 2.0.

The polyketone terpolymer prepared above and a silicon-based wear resistant agent were inserted to produce a composition and the produced composition was molded into pellets on an extruder using a biaxial screw having a diameter of 40 mm and L/D=32, which was operated at 250 rpm, and then injection molded to produce a specimen for a trim mounting clip for automobiles.

Example 77

Linear alternating polyketone terpolymers comprising carbon monoxide and ethylene and propene are prepared

under presence of catalyst composition formed from palladium acetate, trifluoroacetic acid and ((2,2-dimethyl-1,3-dioxane-5,5-diyl)bis(methylene)bis(2-methoxyphenyl) phosphine). In the above, content of trifluoroacetic acid with respect to palladium is 11 times the molar ratio, and two stages of the first stage at a polymerization temperature of 78°C and the second stage at 84°C are carried out. In the prepared polyketone terpolymer, a molar ratio of ethylene to propene was 85:15. Also, a melting point of the polyketone terpolymer was 220°C, LVN measured at 25°C by HFIP (hexafluoroisopropanol) was 1.4 dl/g, and MWD was 2.0.

The polyketone terpolymer prepared above and a glass fiber were inserted to produce a composition and the produced composition was molded into pellets on an extruder using a biaxial screw having a diameter of 40 mm and L/D=32, which was operated at 250 rpm, and then injection molded to produce a specimen for a trim mounting clip for automobiles.

Example 78

Linear alternating polyketone terpolymers comprising carbon monoxide and ethylene and propene are prepared under presence of catalyst composition formed from palladium acetate, trifluoroacetic acid and ((2,2-dimethyl-1,3-dioxane-5,5-diyl)bis(methylene)bis(2-methoxyphenyl) phosphine). In the above, content of trifluoroacetic acid with respect to palladium is 11 times the molar ratio, and two stages of the first stage at a polymerization temperature of 78°C and the second stage at 84°C are carried out. In the prepared polyketone terpolymer, a molar ratio of ethylene to propene was 85:15. Also, a melting point of the polyketone terpolymer was 220°C, LVN measured at 25°C by HFIP (hexafluoroisopropanol) was 1.4 dl/g, and MWD was 2.0.

The polyketone terpolymer prepared above, a glass fiber and a polytetrafluoroethylene resin were inserted to produce a composition and the produced composition was molded into pellets on an extruder using a biaxial screw having a diameter of 40 mm and L/D=32, which was operated at 250 rpm, and then injection molded to produce a specimen for a trim mounting clip for automobiles.

Comparative Example 21

A polyoxymethylene resin was molded into pellets on an extruder using a biaxial screw having a diameter of 40 cm and L/D=32, which was operated at 250 rpm, and then injection molded to produce a specimen for a trim mounting clip for automobiles.

Properties Evaluation

Properties of the specimens prepared in Example 75 to 78 and Comparative Example 21 were evaluated in following method and results are shown in Table 19.

1. Izod impact strength evaluation: Performed according to ASTM D256.
2. Friction coefficient: Coefficient of kinetic friction is a value that can represent a degree of kinetic friction. The larger the coefficient of kinetic friction means the greater the frictional force, and the smaller the value, the smaller the frictional force.
3. Wear property evaluation: Performed according to JIS K7218 (test conditions: 3 km wear at 50 rmp and 150 N)

[Table 19]

| Item | Example 75 | Example 76 | Example 77 | Example 78 | Comparative Example 21 |
|---|---|---|---|---|---|
| impact strength (kJ/$m^2$) | 11 | 10 | 14 | 14 | 6 |
| Friction coefficient | 0.34 | 0.14 | 0.3 | 0.25 | 0.15 |
| Wear property (g) | 0.009 | 0.004 | 0.006 | 0.003 | 0.191 |

As can be seen in Table 19, the impact strength of the Examples was improved compared to Comparative Example 21. Also, the coefficient of kinetic friction and the wear property of the Examples were smaller than those of the Comparative Example 21, thereby the frictional force was small and the wear resistance was improved. Therefore, the trim mounting clip for automobiles manufactured by the examples of the present invention shows better impact resistance and wear resistance than the comparative example used for a conventional material of trim mounting clip for automobiles, thereby it is suitable for application as a trim mounting clip for automobiles.

Example 79

Linear alternating polyketone terpolymers comprising carbon monoxide and ethylene and propene are prepared under presence of catalyst composition formed from palladium acetate, trifluoroacetic acid and ((2,2-dimethyl-1,3-dioxane-5,5-diyl)bis(methylene)bis(2-methoxyphenyl) phosphine). In the above, content of trifluoroacetic acid with respect to palladium is 11 times the molar ratio, and two stages of the first stage at a polymerization temperature of 78°C and the second stage at 84°C are carried out. In the prepared polyketone terpolymer, a molar ratio of ethylene to propene was 85:15. Also, a melting point of the polyketone terpolymer was 220°C, LVN measured at 25°C by HFIP (hexafluoroisopropanol) was 1.4 dl/g, and MWD was 2.0.

The polyketone terpolymer prepared above was molded into pellets on an extruder using a biaxial screw having a diameter of 40 mm and L/D=32, which was operated at 250 rpm, and then injection molded to produce a specimen for a cup holder for automobiles.

Example 80

Linear alternating polyketone terpolymers comprising carbon monoxide and ethylene and propene are prepared under presence of catalyst composition formed from palladium acetate, trifluoroacetic acid and ((2,2-dimethyl-1,3-dioxane-5,5-diyl)bis(methylene)bis(2-methoxyphenyl) phosphine). In the above, content of trifluoroacetic acid with respect to palladium is 11 times the molar ratio, and two stages of the first stage at a polymerization temperature of 78°C and the second stage at 84°C are carried out. In the prepared polyketone terpolymer, a molar ratio of ethylene to propene was 85:15. Also, a melting point of the polyketone terpolymer was 220°C, LVN measured at 25°C by HFIP (hexafluoroisopropanol) was 1.4 dl/g, and MWD was 2.0.

The polyketone terpolymer prepared above and a silicon-based wear resistant agent were inserted to produce a composition and the produced composition was molded into pellets on an extruder using a biaxial screw having a diameter of 40 mm and L/D=32, which was operated at 250 rpm, and then injection molded to produce a specimen for a cup holder for automobiles.

Example 81

Linear alternating polyketone terpolymers comprising carbon monoxide and ethylene and propene are prepared under presence of catalyst composition formed from palladium acetate, trifluoroacetic acid and ((2,2-dimethyl-1,3-dioxane-5,5-diyl)bis(methylene)bis(2-methoxyphenyl) phosphine). In the above, content of trifluoroacetic acid with respect to palladium is 11 times the molar ratio, and two stages of the first stage at a polymerization temperature of 78°C and the second stage at 84°C are carried out. In the prepared polyketone terpolymer, a molar ratio of ethylene to propene was 85:15. Also, a melting point of the polyketone terpolymer was 220°C, LVN measured at 25°C by HFIP (hexafluoroisopropanol) was 1.4 dl/g, and MWD was 2.0.

The polyketone terpolymer prepared above and a glass fiber were inserted to produce a composition and the produced composition was molded into pellets on an extruder using a biaxial screw having a diameter of 40 mm and L/D=32, which was operated at 250 rpm, and then injection molded to produce a specimen for a cup holder for automobiles.

Example 82

Linear alternating polyketone terpolymers comprising carbon monoxide and ethylene and propene are prepared under presence of catalyst composition formed from palladium acetate, trifluoroacetic acid and ((2,2-dimethyl-1,3-dioxane-5,5-diyl)bis(methylene)bis(2-methoxyphenyl) phosphine). In the above, content of trifluoroacetic acid with respect to palladium is 11 times the molar ratio, and two stages of the first stage at a polymerization temperature of 78°C and the second stage at 84°C are carried out. In the prepared polyketone terpolymer, a molar ratio of ethylene to propene was 85:15. Also, a melting point of the polyketone terpolymer was 220°C, LVN measured at 25°C by HFIP (hexafluoroisopropanol) was 1.4 dl/g, and MWD was 2.0.

The polyketone terpolymer prepared above, a glass fiber and a polytetrafluoroethylene resin were inserted to produce a composition and the produced composition was molded into pellets on an extruder using a biaxial screw having a diameter of 40 mm and L/D=32, which was operated at 250 rpm, and then injection molded to produce a specimen for a cup holder for automobiles.

Comparative Example 22

A polyoxymethylene resin was molded into pellets on an extruder using a biaxial screw having a diameter of 40 cm and L/D=32, which was operated at 250 rpm, and then injection molded to produce a specimen for a cup holder

for automobiles.

Properties Evaluation

Properties of the specimens prepared in Example 79 to 82 and Comparative Example 22 were evaluated in following method and results are shown in Table 20.

1. Izod impact strength evaluation: Performed according to ASTM D256.
2. Friction coefficient: Coefficient of kinetic friction is a value that can represent a degree of kinetic friction. The larger the coefficient of kinetic friction means the greater the frictional force, and the smaller the value, the smaller the frictional force.
3. Wear property evaluation: Performed according to JIS K7218 (test condition: 3 km wear at 50 rmp and 150 N)

[Table 20]

| Item | Example 79 | Example 80 | Example 81 | Example 82 | Comparative Example 22 |
|---|---|---|---|---|---|
| impact strength (kJ/m$^2$) | 11 | 10 | 14 | 14 | 6 |
| Friction coefficient | 0.34 | 0.14 | 0.3 | 0.25 | 0.15 |
| Wear property (g) | 0.009 | 0.004 | 0.006 | 0.003 | 0.191 |

As can be seen in Table 20, the impact strength of the Examples was improved compared to Comparative Example 22. Also, the coefficient of kinetic friction and the wear property of the Examples were smaller than those of the Comparative Example 22, thereby the frictional force was small and the wear resistance was improved. Therefore, the cup holder for automobiles manufactured by the examples of the present invention shows better impact resistance and wear resistance than the comparative example used for a conventional material of cup holder for automobiles, thereby it is suitable for application as a cup holder for automobiles.

Example 83

Linear alternating polyketone terpolymers comprising carbon monoxide and ethylene and propene are prepared under presence of catalyst composition formed from palladium acetate, trifluoroacetic acid and ((2,2-dimethyl-1,3-dioxane-5,5-diyl)bis(methylene)bis(2-methoxyphenyl) phosphine). In the above, content of trifluoroacetic acid with respect to palladium is 10 times the molar ratio, and two stages of the first stage at a polymerization temperature of 78°C and the second stage at 84°C are carried out. In the polyketone terpolymer prepared above, carbon monoxide was 50 mol%, ethylene was 46 mol%, and propylene was 4 mol%. Also, a melting point of the polyketone terpolymer was 220°C, LVN measured at 25°C by HFIP (hexa-fluoroisopropanol) was 1.4ldl/g, and MWD was 2.0.
90 weight% of the polyketone terpolymer prepared above and 10 weight% of glass fiber were inserted to produce a composition and the produced composition was molded into pellets on an extruder using a biaxial screw having a diameter of 40 cm and L/D=32, which was operated at 250 rpm, and then injection molded to produce a specimen for a roof rack for automobiles.

Example 84

The same as Example 83 except that the polyketone content was 80 weight% and the glass fiber content was 20 weight%.

Example 85

The same as Example 83 except that the polyketone content was 70 weight% and the glass fiber content was 30 weight%.

Example 86

The same as Example 83 except that the polyketone content was 60 weight% and the glass fiber content was 40 weight%.

Comparative Example 23

70 weight% of nylon66 resin, 15 weight% of glass fiber and 15 weight% of mineral filler were inserted to produce a composition and the produced composition was molded into pellets on an extruder using a biaxial screw having a diameter of 40 cm and L/D=32, which was operated at 250 rpm, and then injection molded to produce a specimen for a roof rack for automobiles.

Properties Evaluation

Properties of the specimens prepared in Example 83 to 86 and Comparative Example 23 were evaluated in following method and results are shown in Table 21.

1. Izod impact strength evaluation: Performed according to ASTM D256.
2. Scratch resistance evaluation: Evaluated according to JIS K 5600-5-4 pencil scratch hardness measurement method.

[Table 21]

| Item | Example 83 | Example 84 | Example 85 | Example 86 | Comparative Example 23 |
|---|---|---|---|---|---|
| impact strength ($kJ/m^2$) | 10 | 10 | 13 | 15 | 7 |
| Scratch resistance | 3H | 3H | 3H | 3H | 2H |

As can be seen in Table 21, the scratch resistance and the impact strength of the examples were evaluated to be superior to those of the comparative example, thereby they were found to have properties suitable for use in roof racks for automobiles.

Example 87

Linear alternating polyketone terpolymers comprising carbon monoxide and ethylene and propene are prepared under presence of catalyst composition formed from palladium acetate, trifluoroacetic acid and ((2,2-dimethyl-1,3-dioxane-5,5-diyl)bis(methylene)bis(2-methoxyphenyl) phosphine). In the above, content of trifluoroacetic acid with respect to palladium is 10 times the molar ratio, and two stages of the first stage at a polymerization temperature of 78°C and the second stage at 84°C are carried out. In the prepared polyketone terpolymer, a molar ratio of ethylene to propene was 46:4. Also, a melting point of the polyketone terpolymer was 220°C, LVN measured at 25°C by HFIP (hexa-fluoroisopropano) was 1.4 dl/g, and MWD was 2.0.

75 weight% of the polyketone terpolymer prepared above, 10 weight% of glass fiber and 15 weight% of mineral filler which was mixed with 60 to 75 weight% of silica and 25 to 40 weight% of magnesium oxide were mixed and molded into pellets on an extruder using a biaxial screw having a diameter of 40 mm and L/D=32, which was operated at 250 rpm, and then injection molded to produce a specimen for an outside door handle for automobiles and its properties were evaluated.

Example 88

The same as Example 87 except that the glass fiber content was 5 weight% and the mineral filler content was 10 weight%.

Example 89

The same as Example 87 except that the glass fiber content was 20 weight% and the mineral filler content was 20 weight%.

Comparative Example 24

70 weight% of nylon 66 resin, 15 weight% of glass fiber and 15 weight% of mineral filler were inserted to produce a composition and the produced composition was molded into pellets on an extruder using a biaxial screw having a diameter of 40 cm and L/D=32, which was operated at 250 rpm, and then injection molded to produce a specimen for

an outside door handle for automobiles.

Properties Evaluation

Properties of the specimens prepared in Example 87 to 89 and Comparative Example 24 were evaluated in following method and results are shown in Table 22.

1. Izod impact strength evaluation: Performed according to ASTM D256.
2. Scratch resistance evaluation: Evaluated according to JIS K 5600-5-4 pencil scratch hardness measurement method.
3. Dimensional change rate evaluation: Evaluation was made according to MS211-47 for vertical and horizontal directions at a temperature of 50°C and a relative humidity of 90 %.

[Table 22]

| Item | Example 87 | Example 88 | Example 89 | Comparative Example 24 |
|---|---|---|---|---|
| impact strength (kJ/m$^2$) | 12 | 13 | 14 | 7 |
| Scratch resistance | 3H | 3H | 3H | 2H |
| Dimensional change rate (%) | 1.1 | 1.3 | 1.5 | 5.2 |

As can be seen in Table 22, the scratch resistance and the dimensional stability of the examples were evaluated to be superior to those of the comparative example, thereby they were found to have properties suitable for use in outside door handles for automobiles.

Example 90

Linear alternating polyketone terpolymers comprising carbon monoxide and ethylene and propene are prepared under presence of catalyst composition formed from palladium acetate, trifluoroacetic acid and ((2,2-dimethyl-1,3-dioxane-5,5-diyl)bis(methylene)bis(2-methoxyphenyl) phosphine). In the above, content of trifluoroacetic acid with respect to palladium is 10 times the molar ratio, and two stages of the first stage at a polymerization temperature of 78°C and the second stage at 84°C are carried out. In the polyketone terpolymer prepared above, carbon monoxide was 50 mol%, ethylene was 46 mol%, and propylene was 4 mol%. Also, a melting point of the polyketone terpolymer was 220°C, LVN measured at 25°C by HFIP (hexa-fluoroisopropanol) was 1.4 dl/g, and MWD was 2.0.

70 weight% of the polyketone terpolymer prepared above and 30 weight% of glass fiber were inserted to produce a composition and the produced composition was molded into pellets on an extruder using a biaxial screw having a diameter of 40 cm and L/D=32, which was operated at 250 rpm, and then injection molded to produce a specimen for an air intake garnish for automobiles.

Example 91

75 weight% of the polyketone terpolymer prepared in same method as Example 90 and 25 weight% of glass fiber were inserted to produce a composition and the produced composition was molded into pellets on an extruder using a biaxial screw having a diameter of 40 cm and L/D=32, which was operated at 250 rpm, and then injection molded to produce a specimen for an air intake garnish for automobiles.

Example 92

80 weight% of the polyketone terpolymer prepared in same method as Example 90 and 20 weight% of glass fiber were inserted to produce a composition and the produced composition was molded into pellets on an extruder using a biaxial screw having a diameter of 40 cm and L/D=32, which was operated at 250 rpm, and then injection molded to produce a specimen for an air intake garnish for automobiles.

Example 93

85 weight% of the polyketone terpolymer prepared in same method as Example 90 and 15 weight% of glass

fiber were inserted to produce a composition and the produced composition was molded into pellets on an extruder using a biaxial screw having a diameter of 40 cm and L/D=32, which was operated at 250 rpm, and then injection molded to produce a specimen for an air intake garnish for automobiles.

Comparative Example 25

A Nylon 66 resin and 30 weight% of glass fiber were inserted to produce a composition and the produced composition was molded into pellets on an extruder using a biaxial screw having a diameter of 40 cm and L/D=32, which was operated at 250 rpm, and then injection molded to produce a specimen for an air intake garnish for automobiles.

Properties Evaluation

Properties of the specimens prepared in Example 90 to 93 and Comparative Example 25 were evaluated in following method and results are shown in Table 23.

1. Izod impact strength evaluation: Performed according to ASTM D256.
2. Wear property: Wear property is a value indicating a degree of wear. The larger the wear property, the smaller the wear resistance because wear occurs more easily. Meanwhile, the smaller the value, the greater the wear resistance because wear doesn't occur easily. Wear property test was carried out in a pin-on-disk type under conditions of a load of 1 kg, a linear velocity of 7 Hz and a test time of 30 minutes.
3. Dimensional change rate evaluation: Evaluation was made according to MS211-47 for vertical and horizontal directions at a temperature of 50°C and a relative humidity of 90 %.

[Table 23]

| Item | Example 90 | Example 91 | Example 92 | Example 93 | Comparative Example 25 |
|---|---|---|---|---|---|
| impact strength (kJ/$m^2$) | 25 | 23 | 21 | 22 | 18 |
| Wear property (Rmax) | 0.60 | 0.70 | 0.75 | 0.82 | 8.10 |
| Dimensional change rate (%) | 1.3 | 1.4 | 1.5 | 1.5 | 5.2 |

As can be seen in Table 23, the impact strength and the dimensional stability of the Examples was improved compared to Comparative Example. Also, the coefficient of kinetic friction and the wear property of the Examples were small, thereby the frictional force was small and the wear resistance was improved. Therefore, the air intake garnish for automobiles manufactured by the examples of the present invention shows better wear resistance and dimensional stability than the comparative example used for a conventional material of air intake garnish for automobiles, thereby it is suitable for application as an air intake garnish for automobiles.

Example 94

Linear alternating polyketone terpolymers comprising carbon monoxide and ethylene and propene are prepared under presence of catalyst composition formed from palladium acetate, trifluoroacetic acid and ((2,2-dimethyl-1,3-dioxane-5,5-diyl)bis(methylene)bis(2-methoxyphenyl) phosphine). In the above, content of trifluoroacetic acid with respect to palladium is 11 times the molar ratio, and two stages of the first stage at a polymerization temperature of 78°C and the second stage at 84°C are carried out. In the prepared polyketone terpolymer, a molar ratio of ethylene to propene was 85:15. Also, a melting point of the polyketone terpolymer was 220°C, LVN measured at 25°C by HFIP (hexafluoroisopropanol) was 1.4 dl/g, and MWD was 2.0. The polyketone terpolymer prepared above was molded into pellets on an extruder using a biaxial screw having a diameter of 40 mm and L/D=32, which was operated at 250 rpm, and then injection molded to produce a specimen for a medical transportation tray.

Example 95

Linear alternating polyketone terpolymers comprising carbon monoxide and ethylene and propene are prepared under presence of catalyst composition formed from palladium acetate, trifluoroacetic acid and ((2,2-dimethyl-1,3-dioxane-5,5-diyl)bis(methylene)bis(2-methoxyphenyl) phosphine). In the above, content of trifluoroacetic acid with respect

to palladium is 11 times the molar ratio, and two stages of the first stage at a polymerization temperature of 78°C and the second stage at 84°C are carried out. In the prepared polyketone terpolymer, a molar ratio of ethylene to propene was 85:15. Also, a melting point of the polyketone terpolymer was 220°C, LVN measured at 25°C by HFIP (hexafluoroisopropanol) was 1.4 dl/g, and MWD was 2.0.

The polyketone terpolymer prepared above and a silicon-based wear resistant agent were inserted to produce a composition and the produced composition was molded into pellets on an extruder using a biaxial screw having a diameter of 40 mm and L/D=32, which was operated at 250 rpm, and then injection molded to produce a specimen for a medical transportation tray.

Example 96

Linear alternating polyketone terpolymers comprising carbon monoxide and ethylene and propene are prepared under presence of catalyst composition formed from palladium acetate, trifluoroacetic acid and ((2,2-dimethyl-1,3-dioxane-5,5-diyl)bis(methylene)bis(2-methoxyphenyl) phosphine). In the above, content of trifluoroacetic acid with respect to palladium is 11 times the molar ratio, and two stages of the first stage at a polymerization temperature of 78°C and the second stage at 84°C are carried out. In the prepared polyketone terpolymer, a molar ratio of ethylene to propene was 85:15. Also, a melting point of the polyketone terpolymer was 220°C, LVN measured at 25°C by HFIP (hexafluoroisopropanol) was 1.4 dl/g, and MWD was 2.0. The polyketone terpolymer prepared above and a glass fiber were inserted to produce a composition and the produced composition was molded into pellets on an extruder using a biaxial screw having a diameter of 40 mm and L/D=32, which was operated at 250 rpm, and then injection molded to produce a specimen for a medical transportation tray.

Example 96

Linear alternating polyketone terpolymers comprising carbon monoxide and ethylene and propene are prepared under presence of catalyst composition formed from palladium acetate, trifluoroacetic acid and ((2,2-dimethyl-1,3-dioxane-5,5-diyl)bis(methylene)bis(2-methoxyphenyl) phosphine). In the above, content of trifluoroacetic acid with respect to palladium is 11 times the molar ratio, and two stages of the first stage at a polymerization temperature of 78°C and the second stage at 84°C are carried out. In the prepared polyketone terpolymer, a molar ratio of ethylene to propene was 85:15. Also, a melting point of the polyketone terpolymer was 220°C, LVN measured at 25°C by HFIP (hexafluoroisopropanol) was 1.4 dl/g, and MWD was 2.0. The polyketone terpolymer prepared above, a glass fiber and a polytetrafluoroethylene resin were inserted to produce a composition and the produced composition was molded into pellets on an extruder using a biaxial screw having a diameter of 40 mm and L/D=32, which was operated at 250 rpm, and then injection molded to produce a specimen for a medical transportation tray.

Comparative Example 26

A specimen for a medical transportation tray was prepared in same method as Example 94 by using polycarbonate which was conventionally used as a material for a medical transportation tray cover.

Properties Evaluation

The prepared pellets of the above Examples were injection-molded to prepare specimens for medical transportation tray. The properties of the specimens were evaluated by following method in comparison with the specimen of the comparative example, and results are shown in Table 24.

1. Friction coefficient: Coefficient of kinetic friction is a value that can represent a degree of kinetic friction. The larger the coefficient of kinetic friction means the greater the frictional force, and the smaller the value, the smaller the frictional force.
2. Wear property evaluation: Performed according to JIS K7218 (test condition: 3 km wear at 50 rmp and 150 N)

[Table 24]

| Item | Example 94 | Example 95 | Example 96 | Example 97 | Comparative Example 256 |
|---|---|---|---|---|---|
| Friction coefficient | 0.34 | 0.14 | 0.3 | 0.25 | 0.15 |
| Wear property (g) | 0.009 | 0.004 | 0.006 | 0.003 | 0.191 |

As can be seen in Table 24, the coefficient of kinetic friction and the wear property of the Examples were smaller than those of the Comparative example 26, thereby the frictional force was small and the wear resistance was improved. Therefore, the medical transportation tray manufactured by the examples of the present invention shows better wear resistance than the comparative example used for a conventional material of medical transportation tray, thereby it is suitable for application as a medical transportation tray.

Example 98

Linear alternating polyketone terpolymers comprising carbon monoxide and ethylene and propene are prepared under presence of catalyst composition formed from palladium acetate, trifluoroacetic acid and ((2,2-dimethyl-1,3-dioxane-5,5-diyl)bis(methylene)bis(2-methoxyphenyl) phosphine). In the above, content of trifluoroacetic acid with respect to palladium is 11 times the molar ratio, and two stages of the first stage at a polymerization temperature of 80°C and the second stage at 84°C are carried out. In the prepared polyketone terpolymer, a molar ratio of ethylene to propene was 85:15. Also, a melting point of the polyketone terpolymer was 220°C, LVN measured at 25°C by HFIP (hexafluoroisopropanol) was 1.2 dl/g, and MWD was 2.0.

The polyketone terpolymer prepared above was molded into pellets on an extruder using a biaxial screw having a diameter of 40 mm and L/D=32, which was operated at 250 rpm, and then injection molded to produce a specimen for a medical pipette.

Example 99

Linear alternating polyketone terpolymers comprising carbon monoxide and ethylene and propene are prepared under presence of catalyst composition formed from palladium acetate, trifluoroacetic acid and ((2,2-dimethyl-1,3-dioxane-5,5-diyl)bis(methylene)bis(2-methoxyphenyl) phosphine). In the above, content of trifluoroacetic acid with respect to palladium is 11 times the molar ratio, and two stages of the first stage at a polymerization temperature of 78°C and the second stage at 84°C are carried out. In the prepared polyketone terpolymer, a molar ratio of ethylene to propene was 85:15. Also, a melting point of the polyketone terpolymer was 220°C, LVN measured at 25°C by HFIP (hexafluoroisopropanol) was 1.4 dl/g, and MWD was 2.0.

The polyketone terpolymer prepared above and a silicon-based wear resistant agent were inserted to produce a composition and the produced composition was molded into pellets on an extruder using a biaxial screw having a diameter of 40 mm and L/D=32, which was operated at 250 rpm, and then injection molded to produce a specimen for a medical pipette.

Example 100

Linear alternating polyketone terpolymers comprising carbon monoxide and ethylene and propene are prepared under presence of catalyst composition formed from palladium acetate, trifluoroacetic acid and ((2,2-dimethyl-1,3-dioxane-5,5-diyl)bis(methylene)bis(2-methoxyphenyl) phosphine). In the above, content of trifluoroacetic acid with respect to palladium is 11 times the molar ratio, and two stages of the first stage at a polymerization temperature of 78°C and the second stage at 84°C are carried out. In the prepared polyketone terpolymer, a molar ratio of ethylene to propene was 85:15. Also, a melting point of the polyketone terpolymer was 220°C, LVN measured at 25°C by HFIP (hexafluoroisopropanol) was 1.4 dl/g, and MWD was 2.0.

The polyketone terpolymer prepared above and a glass fiber were inserted to produce a composition and the produced composition was molded into pellets on an extruder using a biaxial screw having a diameter of 40 mm and L/D=32, which was operated at 250 rpm, and then injection molded to produce a specimen for a medical pipette.

Example 101

Linear alternating polyketone terpolymers comprising carbon monoxide and ethylene and propene are prepared under presence of catalyst composition formed from palladium acetate, trifluoroacetic acid and ((2,2-dimethyl-1,3-dioxane-5,5-diyl)bis(methylene)bis(2-methoxyphenyl) phosphine). In the above, content of trifluoroacetic acid with respect to palladium is 11 times the molar ratio, and two stages of the first stage at a polymerization temperature of 78°C and the second stage at 84°C are carried out. In the prepared polyketone terpolymer, a molar ratio of ethylene to propene was 85:15. Also, a melting point of the polyketone terpolymer was 220°C, LVN measured at 25°C by HFIP (hexafluoroisopropanol) was 1.4 dl/g, and MWD was 2.0. The polyketone terpolymer prepared above, a glass fiber and a polytetrafluoroethylene resin were inserted to produce a composition and the produced composition was molded into pellets on an extruder using a biaxial screw having a diameter of 40 mm and L/D=32, which was operated at 250 rpm, and then injection molded to produce a specimen for a medical pipette.

Comparative Example 27

A specimen for a medical pipette was prepared in same method as Example 98 by using polycarbonate which was conventionally used as a material for a medical pipette.

Properties Evaluation

The prepared pellets of the above Examples were injection-molded to prepare specimens for medical pipette. The properties of the specimens were evaluated by following method in comparison with the specimen of the comparative example, and results are shown in Table 25.

1. Friction coefficient: Coefficient of kinetic friction is a value that can represent a degree of kinetic friction. The larger the coefficient of kinetic friction means the greater the frictional force, and the smaller the value, the smaller the frictional force.
2. Wear property evaluation: Performed according to JIS K7218 (test condition: 3 km wear at 50 rmp and 150 N)
3. Flexural strength evaluation: Performed according to ASTM D790.

[Table 25]

| Item | Example 98 | Example 99 | Example 100 | Example 101 | Comparative Example 27 |
|---|---|---|---|---|---|
| Friction coefficient | 0.34 | 0.14 | 0.3 | 0.25 | 0.15 |
| Wear property (g) | 0.009 | 0.004 | 0.006 | 0.003 | 0.191 |
| Flexural strength (MPa) | 60 | 60 | 200 | 185 | 160 |

As can be seen in Table 25, the coefficient of kinetic friction and the wear property of the Examples were smaller than those of the Comparative example 27, thereby the frictional force was small and the wear resistance and the flexural strength were improved. Therefore, the medical pipette manufactured by the examples of the present invention shows better wear resistance and flexural strength than the comparative example used for a conventional material of medical pipette, thereby it is suitable for application as a medical pipette.

Example 102

Linear alternating polyketone terpolymers comprising carbon monoxide and ethylene and propene are prepared under presence of catalyst composition formed from palladium acetate, trifluoroacetic acid and ((2,2-dimethyl-1,3-dioxane-5,5-diyl)bis(methylene)bis(2-methoxyphenyl) phosphine). In the above, content of trifluoroacetic acid with respect to palladium is 11 times the molar ratio, and two stages of the first stage at a polymerization temperature of 78°C and the second stage at 84°C are carried out. In the prepared polyketone terpolymer, a molar ratio of ethylene to propene was 85:15. Also, a melting point of the polyketone terpolymer was 220°C, LVN measured at 25°C by HFIP (hexafluoroisopropanol) was 1.4 dl/g, and MWD was 2.0.

The polyketone terpolymer prepared above was molded into pellets on an extruder using a biaxial screw having a diameter of 40 mm and L/D=32, which was operated at 250 rpm, and then injection molded to produce a specimen for a refrigerator door closure.

Example 103

Linear alternating polyketone terpolymers comprising carbon monoxide and ethylene and propene are prepared under presence of catalyst composition formed from palladium acetate, trifluoroacetic acid and ((2,2-dimethyl-1,3-dioxane-5,5-diyl)bis(methylene)bis(2-methoxyphenyl) phosphine). In the above, content of trifluoroacetic acid with respect to palladium is 11 times the molar ratio, and two stages of the first stage at a polymerization temperature of 78°C and the second stage at 84°C are carried out. In the prepared polyketone terpolymer, a molar ratio of ethylene to propene was 85:15. Also, a melting point of the polyketone terpolymer was 220°C, LVN measured at 25°C by HFIP (hexafluoroisopropanol) was 1.4 dl/g, and MWD was 2.0.

The polyketone terpolymer prepared above and a polytetrafluoroethylene resin were inserted to produce a composition and the produced composition was molded into pellets on an extruder using a biaxial screw having a diameter of 40 mm and L/D=32, which was operated at 250 rpm, and then injection molded to produce a specimen for

a refrigerator door closure.

Example 104

Linear alternating polyketone terpolymers comprising carbon monoxide and ethylene and propene are prepared under presence of catalyst composition formed from palladium acetate, trifluoroacetic acid and ((2,2-dimethyl-1,3-dioxane-5,5-diyl)bis(methylene)bis(2-methoxyphenyl) phosphine). In the above, content of trifluoroacetic acid with respect to palladium is 11 times the molar ratio, and two stages of the first stage at a polymerization temperature of 78°C and the second stage at 84°C are carried out. In the prepared polyketone terpolymer, a molar ratio of ethylene to propene was 85:15. Also, a melting point of the polyketone terpolymer was 220°C, LVN measured at 25°C by HFIP (hexafluoroisopropanol) was 1.4 dl/g, and MWD was 2.0.

The polyketone terpolymer prepared above and a thermoplastic polyurethane resin were inserted to produce a composition and the produced composition was molded into pellets on an extruder using a biaxial screw having a diameter of 40 mm and L/D=32, which was operated at 250 rpm, and then injection molded to produce a specimen for a refrigerator door closure.

Comparative Example 28

A specimen for a refrigerator door closure was prepared in same method as Example 102 by using polyoxymethylene resin which was conventionally used as a material for a refrigerator door closure.

Properties Evaluation

The prepared pellets of the above Examples were injection-molded to prepare specimens for refrigerator door closure. The properties of the specimens were evaluated by following method in comparison with the specimen of the comparative example, and results are shown in Table 26.

1. Friction coefficient: Coefficient of kinetic friction is a value that can represent a degree of kinetic friction. The larger the coefficient of kinetic friction means the greater the frictional force, and the smaller the value, the smaller the frictional force.
2. Wear property evaluation: Performed according to JIS K7218 (test condition: 3 km wear at 50 rmp and 150 N)
3. Noise evaluation: After noise was measured, it was indicated by ○ when it was 40 dB or more, and when it was less than 40 dB, it was indicated by x.

[Table 26]

| Item | Example 102 | Example 103 | Example 104 | Comparative Example 28 |
|---|---|---|---|---|
| Friction coefficient | 0.34 | 0.13 | 0.14 | 0.18 |
| Wear property (g) | 0.02 | 0.001 | 0.006 | 0.007 |
| Noise generation | ○ | × | × | ○ |

As can be seen in Table 26, the coefficient of kinetic friction and the wear property of the Examples were smaller than those of the Comparative example 28, thereby the frictional force was small and the wear resistance was improved. Therefore, the refrigerator door closure manufactured by the examples of the present invention shows better wear resistance and has less noise than the comparative example used for a conventional material of refrigerator door closure, thereby it is suitable for application as a refrigerator door closure.

Example 105

Linear alternating polyketone terpolymers comprising carbon monoxide and ethylene and propene are prepared under presence of catalyst composition formed from palladium acetate, trifluoroacetic acid and ((2,2-dimethyl-1,3-dioxane-5,5-diyl)bis(methylene)bis(2-methoxyphenyl) phosphine). In the above, content of trifluoroacetic acid with respect to palladium is 11 times the molar ratio, and two stages of the first stage at a polymerization temperature of 78°C and the second stage at 84°C are carried out. In the prepared polyketone terpolymer, a molar ratio of ethylene to propene was 85:15. Also, a melting point of the polyketone terpolymer was 220°C, LVN measured at 25°C by HFIP (hexa-fluoroisopropanol) was 1.4 dl/g, and MWD was 2.0.

The polyketone terpolymer prepared above was molded into pellets on an extruder using a biaxial screw having a diameter of 40 mm and L/D=32, which was operated at 250 rpm, and then injection molded to produce a specimen for a cellular phone polishing fixture.

Example 106

Linear alternating polyketone terpolymers comprising carbon monoxide and ethylene and propene are prepared under presence of catalyst composition formed from palladium acetate, trifluoroacetic acid and ((2,2-dimethyl-1,3-dioxane-5,5-diyl)bis(methylene)bis(2-methoxyphenyl) phosphine). In the above, content of trifluoroacetic acid with respect to palladium is 11 times the molar ratio, and two stages of the first stage at a polymerization temperature of 78°C and the second stage at 84°C are carried out. In the prepared polyketone terpolymer, a molar ratio of ethylene to propene was 85:15. Also, a melting point of the polyketone terpolymer was 220°C, LVN measured at 25°C by HFIP (hexafluoroisopropanol) was 1.4 dl/g, and MWD was 2.0.

The polyketone terpolymer prepared above and a polytetrafluoroethylene resin were inserted to produce a composition and the produced composition was molded into pellets on an extruder using a biaxial screw having a diameter of 40 mm and L/D=32, which was operated at 250 rpm, and then injection molded to produce a specimen for a cellular phone polishing fixture.

Comparative Example 29

A specimen for a cellular phone polishing fixture was prepared in same method as Example 102 by using unsaturated polyester resin which was conventionally used as a material for a cellular phone polishing fixture.

Properties Evaluation

The prepared pellets of the above Examples were injection-molded to prepare specimens for cellular phone polishing fixture. The properties of the specimens were evaluated by following method in comparison with the specimen of the comparative example, and results are shown in Table 27.

1. Tensile strength evaluation: Performed according to ASTM D638.
2. Wear life evaluation: After a product was mounted on the manufactured specimen for cellular phone polishing fixture, time taken for the polishing fixture to wear by performing polishing was measured.

[Table 27]

| Item | Example 105 | Example 106 | Comparative Example 29 |
|---|---|---|---|
| Tensile strength (MPa) | 60 | 50 | 21 |
| Wear life (day) | 20 | 40 | 30 |

As can be seen in Table 27, the tensile strength of the Examples was improved compared with the comparative example, and the wear life of the Example 106 were longer than that of the Comparative example, thereby the wear resistance was improved. Therefore, the cellular phone polishing fixture manufactured by the examples of the present invention shows better wear resistance and tensile strength than the comparative example used for a conventional material of cellular phone polishing fixture, thereby it is suitable for application as a cellular phone polishing fixture.

Example 107

Linear alternating polyketone terpolymers comprising carbon monoxide and ethylene and propene are prepared under presence of catalyst composition formed from palladium acetate, trifluoroacetic acid and ((2,2-dimethyl-1,3-dioxane-5,5-diyl)bis(methylene)bis(2-methoxyphenyl) phosphine). In the above, content of trifluoroacetic acid with respect to palladium is 11 times the molar ratio, and two stages of the first stage at a polymerization temperature of 80°C and the second stage at 84°C are carried out. In the prepared polyketone terpolymer, a molar ratio of ethylene to propene was 46:4. Also, a melting point of the polyketone terpolymer was 220°C, LVN measured at 25°C by HFIP (hexa-fluoroisopropanol) was 1.2 dl/g, MI (Melt Index) was 60 g/10 min and MWD was 2.0.

100 parts by weight of the polyketone terpolymer prepared above and 1 part by weight of silicone resin were

mixed and extruded through melt-kneading on an extruder using a biaxial screw having L/D32 and D 40 at 240°C, which was operated at 250 rpm.

Example 108

Linear alternating polyketone terpolymers comprising carbon monoxide and ethylene and propene are prepared under presence of catalyst composition formed from palladium acetate, trifluoroacetic acid and ((2,2-dimethyl-1,3-dioxane-5,5-diyl)bis(methylene)bis(2-methoxyphenyl) phosphine). In the above, content of trifluoroacetic acid with respect to palladium is 10 times the molar ratio, and two stages of the first stage at a polymerization temperature of 78°C and the second stage at 84°C are carried out. In the prepared polyketone terpolymer, a molar ratio of ethylene to propene was 46:4. Also, a melting point of the polyketone terpolymer was 220°C, LVN measured at 25°C by HFIP (hexa-fluoroisopropanol) was 1.4 dl/g, MI (Melt Index) was 60 g/10 min and MWD was 2.0.

100 parts by weight of the polyketone terpolymer prepared above and 1 part by weight of silicone resin were mixed and extruded through melt-kneading on an extruder using a biaxial screw having L/D32 and D 40 at 240°C, which was operated at 250 rpm.

Example 109

Linear alternating polyketone terpolymers comprising carbon monoxide and ethylene and propene are prepared under presence of catalyst composition formed from palladium acetate, trifluoroacetic acid and ((2,2-dimethyl-1,3-dioxane-5,5-diyl)bis(methylene)bis(2-methoxyphenyl) phosphine). In the above, content of trifluoroacetic acid with respect to palladium is 9 times the molar ratio, and two stages of the first stage at a polymerization temperature of 74°C and the second stage at 84°C are carried out. In the prepared polyketone terpolymer, a molar ratio of ethylene to propene was 46:4. Also, a melting point of the polyketone terpolymer was 220°C, LVN measured at 25°C by HFIP (hexa-fluoroisopropanol) was 1.6 dl/g, MI (Melt Index) was 60 g/10 min and MWD was 2.0.

100 parts by weight of the polyketone terpolymer prepared above and 1 part by weight of silicone resin were mixed and extruded through melt-kneading on an extruder using a biaxial screw having L/D32 and D 40 at 240°C, which was operated at 250 rpm.

Example 110

Linear alternating polyketone terpolymers comprising carbon monoxide and ethylene and propene are prepared under presence of catalyst composition formed from palladium acetate, trifluoroacetic acid and ((2,2-dimethyl-1,3-dioxane-5,5-diyl)bis(methylene)bis(2-methoxyphenyl) phosphine). In the above, content of trifluoroacetic acid with respect to palladium is 10 times the molar ratio, and two stages of the first stage at a polymerization temperature of 78°C and the second stage at 84°C are carried out. In the prepared polyketone terpolymer, a molar ratio of ethylene to propene was 46:4. Also, a melting point of the polyketone terpolymer was 220°C, LVN measured at 25°C by HFIP (hexa-fluoroisopropanol) was 1.4 dl/g, MI (Melt Index) was 60 g/10 min and MWD was 1.8.

100 parts by weight of the polyketone terpolymer prepared above and 1 part by weight of silicone resin were mixed and extruded through melt-kneading on an extruder using a biaxial screw having L/D32 and D 40 at 240°C, which was operated at 250 rpm.

Example 111

Linear alternating polyketone terpolymers comprising carbon monoxide and ethylene and propene are prepared under presence of catalyst composition formed from palladium acetate, trifluoroacetic acid and ((2,2-dimethyl-1,3-dioxane-5,5-diyl)bis(methylene)bis(2-methoxyphenyl) phosphine). In the above, content of trifluoroacetic acid with respect to palladium is 10 times the molar ratio, and two stages of the first stage at a polymerization temperature of 78°C and the second stage at 84°C are carried out. In the prepared polyketone terpolymer, a molar ratio of ethylene to propene was 46:4. Also, a melting point of the polyketone terpolymer was 220°C, LVN measured at 25°C by HFIP (hexa-fluoroisopropanol) was 1.4 dl/g, MI (Melt Index) was 60 g/10 min and MWD was 2.2.

100 parts by weight of the polyketone terpolymer prepared above and 1 part by weight of silicone resin were mixed and extruded through melt-kneading on an extruder using a biaxial screw having L/D32 and D 40 at 240°C, which was operated at 250 rpm.

Comparative Example 30

The same as Example 107 except that POM was used as a material of DuPont in a base state.

Properties Evaluation

The prepared pellets of the above Examples were injection-molded to prepare specimens for ATM gear. The properties of the specimens were evaluated by following method in comparison with the specimen of the comparative example, and results are shown in Table 28.

1. Product strain rate evaluation: It was evaluated according to MS211-47 for vertical and horizontal directions at a temperature of 50°C and a relative humidity of 90 %.
2. Product weight change rate evaluation: It was evaluated according to MS211-47 at a temperature of 50°C and a relative humidity of 90 %.
3. Water resistance property maintenance rate: After 24 hours treatment at 50°C and 90 % relative humidity, impact strength was measured and compared.
4. Wear amount evaluation: It was evaluated using a cylindrical specimen having an inner diameter of 20 mm, an outer diameter of 25 mm and a height of 15 mm and a counter material. Wear amount was measured at a speed of 50 rpm, a load of 150 N, and a wear distance of 3 km under JIS K7218 standard.

[Table 28]

| Item | Example 107 | Example 108 | Example 109 | Example 110 | Example 111 | Comparative Example 30 |
|---|---|---|---|---|---|---|
| Product strain rate - vertical (50°C, RH 90 %) | 0.05 | 0.05 | 0.04 | 0.05 | 0.05 | 0.15 |
| Product strain rate - horizontal (50°C, RH 90 %) | 0.02 | 0.02 | 0.02 | 0.02 | 0.02 | 0.08 |
| Product weight change rate (50°C, RH 90 %) | 0.95 | 0.85 | 0.88 | 0.92 | 0.94 | 3.42 |
| property maintenance rate (50°C, RH 90 %) | 90 | 88 | 92 | 86 | 88 | 48 |
| Wear amount (g) | 0.005 | 0.003 | 0.004 | 0.003 | 0.004 | 0.083 |

As shown in Table 28, the product strain rate in vertical and horizontal directions was lower than that of the comparative example, and the product weight change rate was also lower than that of the comparative example, and it was excellent in water resistance property maintenance rate and wear resistance.

Also, the present invention had a wear amount of 0.020 g or less, which is superior, at a speed of 50 rpm, a load of 150 N, a wear distance of 3 km under JIS K7218 standard.

Accordingly, it is proved that the polyketone resin composition produced by the examples of the present invention can be utilized as an ATM gear and etc. which requires excellent wear resistance.

## Claims

**1.** A polyketone composition with excellent wear resistance, comprising repeating units represented by following general formula (1) and (2), wherein y/x is 0.03 to 0.3.

$$-(CH_2CH_2\text{-}CO)x\text{-} \qquad (1)$$

$$-(CH_2CH(CH_3)\text{-}CO)y\text{-} \qquad (2)$$

(x and y are mole% of each of the general formula (1) and (2) in a polymer)

**2.** A polyketone molded component manufactured by injection molding a polyketone composition manufactured by blending a linear alternating polyketone comprising carbon monoxide and at least one kind of olefinically unsaturated hydrocarbon; and an wear resistant agent comprising a silicone, a polytetrafluoroethylene, a calcium carbonate, a maleic acid, a molybdenum, a glass fiber and a magnesium stearate, and having an wear amount of 0.005 g or less

measured at a speed of 50 rpm, a load of 150 N and a wear distance of 3 km.

**3.** The polyketone molded component of claim 2, wherein the linear alternating polyketone has a molar ratio of ethylene to propylene was 9 to 24:1.

**4.** The polyketone molded component of claim 2, wherein a content of the polyketone is 80 to 99.9 weight% based on a weight of entire polyketone composition and a content of the wear resistant agent is 0.1 to 20 weight%.

**5.** The polyketone molded component of claim 2, wherein intrinsic viscosity of the linear alternating polyketone is 1.0 to 2.0 dl/g and a molecular weight distribution is 1.5 to 2.5.

**6.** The polyketone molded component of claim 2, wherein the molded component is one kind selected from a group comprising a wear part in OA, an ATM gear, an electric/electronic gear, a city gas meter gear and razor printer toner gear.

**7.** A microwave container manufactured by injection molding a linear alternating polyketone polymer comprising carbon monoxide and at least one kind of olefinically unsaturated hydrocarbon, and having a residual amount of palladium catalyst of 50 ppm or less and a molecular weight distribution of 1.5 to 2.5.

**8.** The microwave container of claim 7, wherein the linear alternating polyketone has a molar ratio of ethylene to propylene was 9 to 24:1.

**9.** The microwave container of claim 7, wherein intrinsic viscosity of the linear alternating polyketone is 1.0 to 2.0 dl/g.

**10.** The microwave container of claim 7, wherein an wear amount is 1.0 $mm^3$/kg/km or less in a base state.

**11.** A polyketone cam manufactured with a polyketone copolymer comprising repeating units represented by following general formula (1) and (2).

$$-(CH_2CH_2\text{-}CO)x- \qquad (1)$$

$$-(CH_2CH(CH_3)\text{-}CO)y- \qquad (2)$$

(x and y are mole% of each of the general formula (1) and (2) in a polymer, and y/x is 0.03 to 0.3)

**12.** The polyketone cam of claim 11, wherein intrinsic viscosity of the polyketone copolymer is 1.0 to 2.0 dl/g.

**13.** The polyketone cam of claim 11, wherein a degree of noise generation when measuring wear resistance is 70 dB or less.

**14.** The polyketone cam of claim 11, wherein an wear amount measured using a Taber wear tester (manufactured by DAITO ELECTRON CO., LTD., condition: a load of 1 kg and wear wheel H-22) according to JIS K-7311 after being left at 25°C for 2 days is 25 mg or less.

**15.** A helmet gear for electric welding comprising, as a material, a polyketone resin comprising repeating units represented by following general formula (1) and (2).

$$-(CH_2CH_2\text{-}CO)x- \qquad (1)$$

$$-(CH_2CH(CH_3)\text{-}CO)y- \qquad (2)$$

(x and y are mole% of each of the general formula (1) and (2) in a polymer, and y/x is 0.03 to 0.3)

**16.** The helmet gear for electric welding of claim 15, wherein intrinsic viscosity of the polyketone resin is 1.0 to 2.0 dl/g.

**17.** The helmet gear for electric welding of claim 15, wherein a molecular weight distribution of the polyketone resin is 1.5 to 2.5.

**18.** The helmet gear for electric welding of claim 15, wherein an wear coefficient ($K_{LNP}$) measured at 25°C using a thrust washer test device is 200 to 300.

**19.** A polyketone plastic board for blanking molding plastic gear, which is a polyketone copolymer comprising repeating units represented by following general formula (1) and (2), and has an wear amount of 0.020 g or less measured at a speed of 50 rpm, a load of 150 N and a wear distance of 3 km under JIS K7218.

$$-(CH_2CH_2\text{-}CO)x- \quad (1)$$

$$-(CH_2CH(CH_3)\text{-}CO)y- \quad (2)$$

(x and y are mole% of each of the general formula (1) and (2) in a polymer, and y/x is 0.03 to 0.3)

**20.** The polyketone plastic board of claim 19, wherein a wear resistant agent is added and the wear resistant agent is a silicone resin in a powder form.

**21.** The polyketone plastic board of claim 20, wherein the wear resistant agent is 0.1 to 15 parts by weight relative to 100 parts by weight of the polyketone copolymer.

**22.** The polyketone plastic board of claim 19, wherein intrinsic viscosity of the polyketone copolymer is 1.0 to 2.0 dl/g.

**23.** The polyketone plastic board of claim 19, wherein a molecular weight distribution of the polyketone copolymer is 1.5 to 2.5.

**24.** A polyketone yarn guide which guides a yarn wound on a bobbin to a needle, comprising a yarn guide hole through which the yarn passes; a yarn path for guiding the yarn passing through the yarn guide hole to the needles; a guide member and a roller, and manufactured with a polyketone copolymer comprising repeating units represented by general formula (1) and (2).

$$-(CH_2CH_2\text{-}CO)x- \quad (1)$$

$$-(CH_2CH(CH_3)\text{-}CO)y- \quad (2)$$

(x and y are mole% of each of the general formula (1) and (2) in a polymer, and y/x is 0.03 to 0.3)

**25.** The polyketone yarn guide of claim 24, wherein intrinsic viscosity of the polyketone copolymer is 1.0 to 2.0 dl/g.

**26.** The polyketone yarn guide of claim 24, wherein ligand of catalyst composition used in polymerization of the polyketone copolymer is ((2,2-dimethyl-1,3-dioxane,5,5-diyl)bis(methylene))bis(bis(2-methoxyphenyl)phosphine).

**27.** The polyketone yarn guide of claim 24, wherein a molecular weight distribution of the polyketone copolymer is 1.5 to 2.5.

**28.** A polyketone microwave component manufactured by injection molding a linear alternating polyketone which is a polyketone copolymer comprising repeating units represented by general formula (1) and (2) and wherein y/x is 0.03 to 0.3, wherein intrinsic viscosity of the polyketone is 1.0 to 2.0 dl/g and an wear amount of the microwave component manufactured by injection molding is 1.0 $mm^3$/kg/km or less.

$-(CH_2CH_2-CO)x-$ (1)

$-(CH_2CH(CH_3)-CO)y-$ (2)

(x and y are mole% of each of the general formula (1) and (2) in a polymer)

**29.** The polyketone microwave component of claim 28, wherein the microwave component is a turntable roller or a turntable bracket.

**30.** The polyketone microwave component of claim 28, wherein a thermal strain temperature is 130°C or higher.

**31.** The polyketone microwave component of claim 28, wherein an injection cycle is less than 20 sec.

**32.** A cam for bedding cleaner which is fixed to a rotating shaft of a vibrator for vibrating a suction mechanism of a bedding cleaner and linearly reciprocates connecting road, wherein the cam is manufactured with polyketone copolymer comprising repeating units represented by general formula (1) and (2).

$-(CH_2CH_2-CO)x-$ (1)

$-(CH_2CH(CH_3)-CO)y-$ (2)

(x and y are mole% of each of the general formula (1) and (2) in a polymer, and y/x is 0.03 to 0.3)

**33.** The cam for bedding cleaner of claim 32,
wherein the polyketone copolymer is manufactured in sequence by
a step of preparing a catalyst composition comprising a palladium compound, an acid having a pKa value of 6 or less, and a bidentate compound of phosphorus;
a step of preparing a mixed solvent comprising methanol and water;
a step of conducting polymerization in presence of the catalyst composition and the mixed solvent to prepare a linear terpolymer of carbon monoxide, ethylene and propene;
a step of obtaining polyketone resin removing a remaining catalyst composition in the prepared linear terpolymer with an alcohol solvent.

**34.** The cam for bedding cleaner of any one among claim 32 to 33, wherein intrinsic viscosity of the polyketone copolymer measured at 25°C by HFIP (hexa-fluoroisopropanol) is 1.0 to 2.0 dl/g.

**35.** The cam for bedding cleaner of claim 34, wherein an wear amount of cam is 25 mg or less.

**36.** A polyketone office component manufactured by injection molding a linear alternating polyketone comprising carbon monoxide and at least one kind of olefinically unsaturated hydrocarbon and having a molecular weight distribution of 1.5 to 2.5 and a residual amount of palladium catalyst of 20 ppm or less, wherein impact strength is 8 to 15 $kJ/m^2$.

**37.** The polyketone office component of claim 36, wherein ligand of a catalyst composition in polymerization of the linear alternating polyketone is (2,2-dimethyl-1,3-dioxane-5,5-diyl)bis(methylene))bis(bis(2-methoxyphenyl)phosphine).

**38.** The polyketone office component of claim 36, wherein an wear amount of the linear alternating polyketone is 1.0 $mm^3/kg/km$ or less in a base state.

**39.** The polyketone office component of claim 36, wherein intrinsic viscosity of the linear alternating polyketone copolymer is 1.0 to 2.0 dl/g.

**40.** A polyketone office component manufactured by injection molding a polyketone composition produced by blending

a wear resistant agent and the linear alternating polyketone comprising carbon monoxide and at least one kind of olefinically unsaturated hydrocarbon and having a molecular weight distribution of 1.5 to 2.5 and a residual amount of palladium catalyst of 20 ppm or less, wherein an wear amount is 0.1 $mm^3$/kg/km or less.

**41.** The polyketone office component of claim 40,
wherein content of the wear resistant agent is 0.5 to 2.0 weight% based on total polyketone composition, and the wear resistant agent is a silicone oil or silicone gum.

**42.** A window drum for automobiles manufactured by injection molding a polyketone composition comprising a linear alternating polyketone polymer comprising carbon monoxide and at least one kind of olefinically unsaturated hydrocarbon and having a residual amount of a palladium catalyst of 5 to 50 ppm and a molecular weight distribution of 1.5 to 3.0.

**43.** The window drum for automobiles of claim 42,
wherein the polyketone composition further comprises a silicon-based wear resistant agent.

**44.** The window drum for automobiles of claim 43,
wherein content of the silicon-based wear resistant agent is 2 to 20 weight% based on 100 weight% of the entire polyketone composition.

**45.** The window drum for automobiles of claim 42,
wherein a molar ratio of ethylene to propylene of the linear alternating polyketone is 99:1 to 85:15.

**46.** The window drum for automobiles of claim 42,
wherein intrinsic viscosity of the linear alternating polyketone polymer is 1.2 to 2.0 dl/g.

**47.** The window drum for automobiles of claim 42,
wherein impact strength is 10 kJ/$m^2$ or more and wear resistance in the base state is 0.015 g or less.

**48.** A sun visor retainer manufactured by injection molding a linear alternating polyketone polymer comprising carbon monoxide and at least one kind of olefinically unsaturated hydrocarbon and having a residual amount of palladium catalyst of 5 to 50 ppm and a molecular weight distribution of 1.5 to 3.0.

**49.** The sun visor retainer of claim 48,
wherein a molar ratio of ethylene to propylene of the linear alternating polyketone is 99:1 to 85:15.

**50.** The sun visor retainer of claim 48,
wherein intrinsic viscosity of the linear alternating polyketone polymer is 1.2 to 2.0 dl/g.

**51.** The sun visor retainer of claim 48,
wherein wear resistance Rmax is 1.0 or less.

**52.** A door frame inner cover for automobiles manufactured by injection molding a blend comprising 60 to 85 weight% of a linear alternating polyketone polymer comprising carbon monoxide and at least one kind of olefinically unsaturated hydrocarbon and having a residual amount of palladium catalyst of 5 to 50 ppm and a molecular weight distribution of 1.5 to 3.0, and 15 to 40 weight% of glass fiber.

**53.** The door frame inner cover for automobiles of claim 52,
wherein a molar ratio of ethylene to propylene of the linear alternating polyketone is 99:1 to 85:15.

**54.** The door frame inner cover for automobiles of claim 52,
wherein intrinsic viscosity of the linear alternating polyketone polymer is 1.2 to 2.0 dl/g.

**55.** The door frame inner cover for automobiles of claim 52,
wherein impact strength is 20 kJ/$m^2$ or more and a dimensional change rate is 2% or less.

**56.** A safety belt jointer for automobiles manufactured by injection molding a polyketone composition comprising a linear alternating polyketone polymer comprising carbon monoxide and at least one kind of olefinically unsaturated hydrocarbon and having a residual amount of palladium catalyst of 5 to 50 ppm and a molecular weight distribution of 1.5 to 3.0.

**57.** The safety belt jointer for automobiles of claim 56,
wherein the polyketone composition further comprises at least one kind selected from a group comprising a silicon-based wear resistant agent, a glass fiber and a polytetrafluoroethylene resin.

**58.** The safety belt jointer for automobiles of claim 56,
wherein a molar ratio of ethylene to propylene of the linear alternating polyketone is 99:1 to 85:15.

**59.** The safety belt jointer for automobiles of claim 56,
wherein intrinsic viscosity of the linear alternating polyketone polymer is 1.2 to 2.0 dl/g.

**60.** The safety belt jointer for automobiles of claim 56,
wherein impact strength is 10 kJ/$m^2$ or more and wear amount is 0.015 g or less in a base state.

**61.** An auto gear slide for automobiles manufactured by injection molding a polyketone composition comprising a linear alternating polyketone polymer comprising carbon monoxide and at least one kind of olefinically unsaturated hydrocarbon and having a residual amount of palladium catalyst of 5 to 50 ppm and a molecular weight distribution of 1.5 to 3.0.

**62.** The auto gear slide for automobiles of claim 61,
wherein the polyketone composition further comprises at least one kind selected from a group comprising a silicon-based wear resistant agent, a glass fiber and a polytetrafluoroethylene resin.

**63.** The auto gear slide for automobiles of claim 61,
wherein a molar ratio of ethylene to propylene of the linear alternating polyketone is 99:1 to 85:15.

**64.** The auto gear slide for automobiles of claim 61,
wherein intrinsic viscosity of the linear alternating polyketone polymer is 1.2 to 2.0 dl/g.

**65.** The auto gear slide for automobiles of claim 61,
wherein wear amount is 0.015 g or less in a base state.

**66.** A door latch housing for automobiles manufactured by injection molding a polyketone composition comprising a linear alternating polyketone polymer comprising carbon monoxide and at least one kind of olefinically unsaturated hydrocarbon and having a residual amount of palladium catalyst of 5 to 50 ppm and a molecular weight distribution of 1.5 to 3.0.

**67.** The door latch housing for automobiles of claim 66,
wherein the polyketone composition further comprises a silicon-based wear resistant agent.

**68.** The door latch housing for automobiles of claim 67,
wherein content of the silicon-based wear resistant agent is 2 to 20 weight% based on 100 weight% of the entire polyketone composition.

**69.** The door latch housing for automobiles of claim 66,
wherein a molar ratio of ethylene to propylene of the linear alternating polyketone is 99:1 to 85:15.

**70.** The door latch housing for automobiles of claim 66,
wherein intrinsic viscosity of the linear alternating polyketone polymer is 1.2 to 2.0 dl/g.

**71.** The door latch housing for automobiles of claim 66,
wherein impact strength is 10 kJ/$m^2$ or more and wear resistance in the base state is 0.015 g or less.

**72.** A slide guide for automobiles manufactured by injection molding a polyketone composition comprising a linear alternating polyketone polymer comprising carbon monoxide and at least one kind of olefinically unsaturated hydrocarbon and having a residual amount of palladium catalyst of 5 to 50 ppm and a molecular weight distribution of 1.5

to 3.0.

**73.** The slide guide for automobiles of claim 72,
wherein the polyketone composition further comprises a silicon-based wear resistant agent.

**74.** The slide guide for automobiles of claim 73,
wherein content of the silicon-based wear resistant agent is 2 to 20 weight% based on 100 weight% of the entire polyketone composition.

**75.** The slide guide for automobiles of claim 72,
wherein a molar ratio of ethylene to propylene of the linear alternating polyketone is 99:1 to 85:15.

**76.** The slide guide for automobiles of claim 72,
wherein intrinsic viscosity of the linear alternating polyketone polymer is 1.2 to 2.0 dl/g.

**77.** The slide guide for automobiles of claim 72,
wherein impact strength is 10 $kJ/m^2$ or more and wear resistance in the base state is 0.015 g or less.

**78.** A switch shaft in heating, ventilation and air conditioning (HAVC) systems for automobiles manufactured by injection molding a polyketone composition comprising a linear alternating polyketone polymer comprising carbon monoxide and at least one kind of olefinically unsaturated hydrocarbon and having a residual amount of palladium catalyst of 5 to 50 ppm and a molecular weight distribution of 1.5 to 3.0.

**79.** The switch shaft in heating, ventilation and air conditioning (HAVC) systems for automobiles of claim 78,
wherein the polyketone composition further comprises at least one kind selected from a group comprising a silicon-based wear resistant agent, a glass fiber and a polytetrafluoroethylene resin.

**80.** The switch shaft in heating, ventilation and air conditioning (HAVC) systems for automobiles of claim 78,
wherein a molar ratio of ethylene to propylene of the linear alternating polyketone is 99:1 to 85:15.

**81.** The switch shaft in heating, ventilation and air conditioning (HAVC) systems for automobiles of claim 78,
wherein intrinsic viscosity of the linear alternating polyketone polymer is 1.2 to 2.0 dl/g.

**82.** The switch shaft in heating, ventilation and air conditioning (HAVC) systems for automobiles of claim 78,
wherein impact strength is 10 $kJ/m^2$ or more, and wear amount is 0.015 g or less in a base state.

**83.** An actuator gears for automobiles manufactured by injection molding a polyketone composition comprising a linear alternating polyketone polymer comprising carbon monoxide and at least one kind of olefinically unsaturated hydrocarbon and having a residual amount of palladium catalyst of 5 to 50 ppm and a molecular weight distribution of 1.5 to 3.0.

**84.** The actuator gears for automobiles of claim 83,
wherein the polyketone composition further comprises at least one kind selected from a group comprising a silicon-based wear resistant agent, a glass fiber and a polytetrafluoroethylene resin.

**85.** The actuator gears for automobiles of claim 83,
wherein a molar ratio of ethylene to propylene of the linear alternating polyketone is 99:1 to 85:15.

**86.** The actuator gears for automobiles of claim 83,
wherein intrinsic viscosity of the linear alternating polyketone polymer is 1.2 to 2.0 dl/g.

**87.** The actuator gears for automobiles of claim 83,
wherein impact strength is 10 $kJ/m^2$ or more and wear amount is 0.015 g or less in a base state.

**88.** A trim mounting clip for automobiles manufactured by injection molding a polyketone composition comprising a linear alternating polyketone polymer comprising carbon monoxide and at least one kind of olefinically unsaturated hydrocarbon and having a residual amount of palladium catalyst of 5 to 50 ppm and a molecular weight distribution of 1.5 to 3.0.

**89.** The trim mounting clip for automobiles of claim 88,
wherein the polyketone composition further comprises at least one kind selected from a group comprising a silicon-based wear resistant agent, a glass fiber and a polytetrafluoroethylene resin.

**90.** The trim mounting clip for automobiles of claim 88,
wherein a molar ratio of ethylene to propylene of the linear alternating polyketone is 99:1 to 85:15.

**91.** The trim mounting clip for automobiles of claim 88,
wherein intrinsic viscosity of the linear alternating polyketone polymer is 1.2 to 2.0 dl/g.

**92.** The trim mounting clip for automobiles of claim 88,
wherein impact strength is 10 $kJ/m^2$ or more and wear amount is 0.015 g or less in a base state.

**93.** A cup holder for automobiles manufactured by injection molding a polyketone composition comprising a linear alternating polyketone polymer comprising carbon monoxide and at least one kind of olefinically unsaturated hydrocarbon and having a residual amount of palladium catalyst of 5 to 50 ppm and a molecular weight distribution of 1.5 to 3.0.

**94.** The cup holder for automobiles of claim 93,
wherein the polyketone composition further comprises at least one kind selected from a group comprising a silicon-based wear resistant agent, a glass fiber and a polytetrafluoroethylene resin.

**95.** The cup holder for automobiles of claim 93,
wherein a molar ratio of ethylene to propylene of the linear alternating polyketone is 99:1 to 85:15.

**96.** The cup holder for automobiles of claim 93,
wherein intrinsic viscosity of the linear alternating polyketone polymer is 1.2 to 2.0 dl/g.

**97.** The cup holder for automobiles of claim 93,
wherein impact strength is 10 $kJ/m^2$ or more and wear amount is 0.015 g or less in a base state.

**98.** A roof rack for automobiles manufactured by injection molding a blend comprising 60 to 90 weight% of a linear alternating polyketone polymer comprising carbon monoxide and at least one kind of olefinically unsaturated hydrocarbon and having a residual amount of palladium catalyst of 5 to 50 ppm and a molecular weight distribution of 1.5 to 3.0.

**99.** The roof rack for automobiles of claim 98,
wherein a molar ratio of ethylene to propylene of the linear alternating polyketone is 99:1 to 85:15.

**100.** The roof rack for automobiles of claim 98,
wherein intrinsic viscosity of the linear alternating polyketone polymer is 1.2 to 2.0 dl/g.

**101.** The roof rack for automobiles of claim 98,
wherein impact strength is 10 $kJ/m^2$ and pencil hardness is 3H or more.

**102.** An outside door handle for automobiles manufactured by injection molding a blend comprising a linear alternating polyketone polymer comprising carbon monoxide and at least one kind of olefinically unsaturated hydrocarbon and having a residual amount of palladium catalyst of 5 to 50 ppm and a molecular weight distribution of 1.5 to 3.0.

**103.** The outside door handle for automobiles of claim 102,
wherein a molar ratio of ethylene to propylene of the linear alternating polyketone is 99:1 to 85:15.

**104.** The outside door handle for automobiles of claim 102,
wherein intrinsic viscosity of the linear alternating polyketone polymer is 1.2 to 2.0 dl/g.

**105.** The outside door handle for automobiles of claim 102,
wherein the mineral filler is one selected from a group comprising talc, kaolin, mica, wollastonite, $TiO_2$-coated mica platelets, silica, alumina, borosilicates and oxides.

**106.** The outside door handle for automobiles of claim 102, wherein impact strength is 10 $kJ/m^2$ or more and dimensional change rate is 1.5% or less.

**107.** An air intake garnish for automobiles manufactured by injection molding a blend comprising 60 to 85 weight% of a linear alternating polyketone polymer comprising carbon monoxide and at least one kind of olefinically unsaturated hydrocarbon and having a residual amount of palladium catalyst of 5 to 50 ppm and a molecular weight distribution of 1.5 to 3.0, and 15 to 40 weight% of glass fiber.

**108.** The air intake garnish for automobiles of claim 107, wherein a molar ratio of ethylene to propylene of the linear alternating polyketone is 99:1 to 85:15.

**109.** The air intake garnish for automobiles of claim 107, wherein intrinsic viscosity of the linear alternating polyketone polymer is 1.2 to 2.0 dl/g.

**110.** The air intake garnish for automobiles of claim 107, wherein impact strength is 20 $kJ/m^2$ and wear resistance Rmax is 1.0 or less.

**111.** A polyketone medical transportation tray manufactured by injection molding a polyketone composition comprising a linear alternating polyketone which is polyketone copolymer comprising repeating units represented by general formula (1) and (2) described above, and has y/x of 0.1 to 0.3.

$$-(CH_2CH_2\text{-}CO)x- \qquad (1)$$

$$-(CH_2CH(CH_3)\text{-}CO)y- \qquad (2)$$

(x and y are mole% of each of the general formula (1) and (2) in a polymer)

**112.** The polyketone medical transportation tray of claim 111, wherein the polyketone composition further comprises at least one kind selected from a group comprising a silicon-based wear resistant agent, a glass fiber and a polytetrafluoroethylene resin.

**113.** The medical transportation tray of claim 111, wherein a wear amount is 0.015 g or less at a base state.

**114.** The polyketone medical transportation tray of claim 111, wherein intrinsic viscosity of the polyketone is 1.2 to 2.0 dl/g.

**115.** The polyketone medical transportation tray of claim 111, wherein ligand of a catalyst composition used in polyketone polymerization is (2,2-dimethyl-1,3-dioxane-5,5-diyl)bis(methylene))bis(bis(2-methoxyphenyl)phosphine).

**116.** A polyketone medical pipette manufactured by injection molding a polyketone composition comprising a linear alternating polyketone which is polyketone copolymer comprising repeating units represented by general formula (1) and (2) described above, and has y/x of 0.1 to 0.3.

$$-(CH_2CH_2\text{-}CO)x- \qquad (1)$$

$$-(CH_2CH(CH_3)\text{-}CO)y- \qquad (2)$$

(x and y are mole% of each of the general formula (1) and (2) in a polymer)

**117.** The polyketone medical pipette of claim 116, wherein the polyketone composition further comprises at least one kind selected from a group comprising a silicon-based wear resistant agent, a glass fiber and a polytetrafluoroethylene resin.

**118.** The polyketone medical pipette of claim 116, wherein flexural strength is 180 MPa or more and wear amount is 0.015 g or less at a base state.

**119.** The polyketone medical pipette of claim 116, wherein intrinsic viscosity of the polyketone is 1.2 to 2.0 dl/g.

**120.** The polyketone medical pipette of claim 116, wherein ligand of a catalyst composition used in polyketone polymerization is (2,2-dimethyl-1,3-dioxane-5,5-diyl)bis(methylene))bis(bis(2-methoxyphenyl)phosphine).

**121.** A polyketone refrigerator door closure manufactured by injection molding a polyketone composition comprising a linear alternating polyketone which is polyketone copolymer comprising repeating units represented by general formula (1) and (2) described above, and has y/x of 0.1 to 0.3.

$$-(CH_2CH_2\text{-}CO)x- \qquad (1)$$

$$-(CH_2CH(CH_3)\text{-}CO)y- \qquad (2)$$

(x and y are mole% of each of the general formula (1) and (2) in a polymer)

**122.** The polyketone refrigerator door closure of claim 121, wherein the polyketone composition further comprises a polytetrafluoroethylene resin and thermoplastic polyurethane rein.

**123.** The polyketone refrigerator door closure of claim 121, wherein a wear amount is 0.015 g or less at a base state.

**124.** The polyketone refrigerator door closure of claim 121, wherein intrinsic viscosity of the polyketone is 1.2 to 2.0 dl/g.

**125.** The polyketone refrigerator door closure of claim 121, wherein ligand of a catalyst composition used in polyketone polymerization is (2,2-dimethyl-1,3-dioxane-5,5-diyl)bis(methylene))bis(bis(2-methoxyphenyl)phosphine).

**126.** A polyketone cellular phone polishing fixture manufactured by injection molding a polyketone composition comprising a linear alternating polyketone which is polyketone copolymer comprising repeating units represented by general formula (1) and (2) described above, and has y/x of 0.1 to 0.3.

$$-(CH_2CH_2\text{-}CO)x- \qquad (1)$$

$$-(CH_2CH(CH_3)\text{-}CO)y- \qquad (2)$$

(x and y are mole% of each of the general formula (1) and (2) in a polymer)

**127.** The polyketone cellular phone polishing fixture of claim 126, wherein the polyketone composition further comprises a polytetrafluoroethylene resin.

**128.** The polyketone cellular phone polishing fixture of claim 126, wherein tensile strength is 30 MPa or more.

**129.** The polyketone cellular phone polishing fixture of claim 126, wherein intrinsic viscosity of the polyketone is 1.2 to 2.0 dl/g.

**130.** The polyketone cellular phone polishing fixture of claim 126, wherein ligand of a catalyst composition used in polyketone polymerization is (2,2-dimethyl-1,3-dioxane-5,5-diyl)bis(methylene))bis(bis(2-methoxyphenyl)phosphine).

**131.** A polyketone composition for ATM gear comprising polyketone copolymer comprising repeating units represented by following general formula (1) and (2), and which is comprising 100 parts by weight of polyketone resin having y/x of 0.03 to 0.3 and 0.1 to 15 parts by weight of silicone resin.

$-(CH_2CH_2-CO)x-$ (1)

$-(CH_2CH(CH_3)-CO)y-$ (2)

(x and y are mole% of each of the general formula (1) and (2) in a polymer)

**132.** The polyketone composition for ATM gear of claim 131, wherein the silicone resin is in a form of powder and preferably has a diameter of 1 to 2 μ.

**133.** The polyketone composition for ATM gear of claim 131, wherein intrinsic viscosity of the polyketone resin is 1.2 to 2.0 dl/g.

**134.** The polyketone composition for ATM gear of claim 131, wherein a molecular weight distribution of the polyketone resin is 1.5 to 2.5.

**135.** The polyketone composition for ATM gear of claim 131, wherein an wear amount of the polyketone resin composition is 0.020 g or less in a speed of 50 rpm, a load of 150 N and a wear distance of 3 km under JIS K7218 standard.

FIG.1

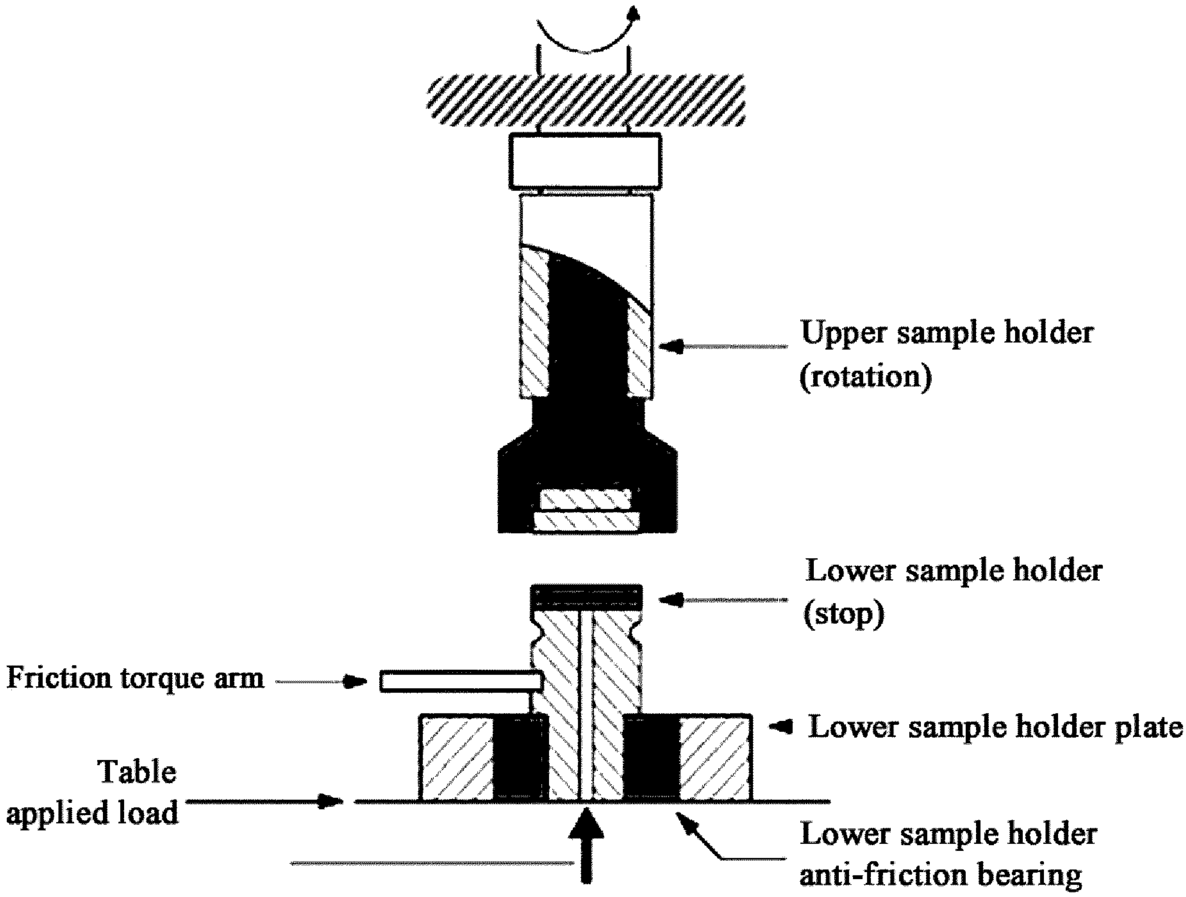
Upper sample holder
(rotation)
Lower sample holder
(stop)
Friction torque arm
Lower sample holder plate
Table
applied load
Lower sample holder
anti-friction bearing

FIG.2

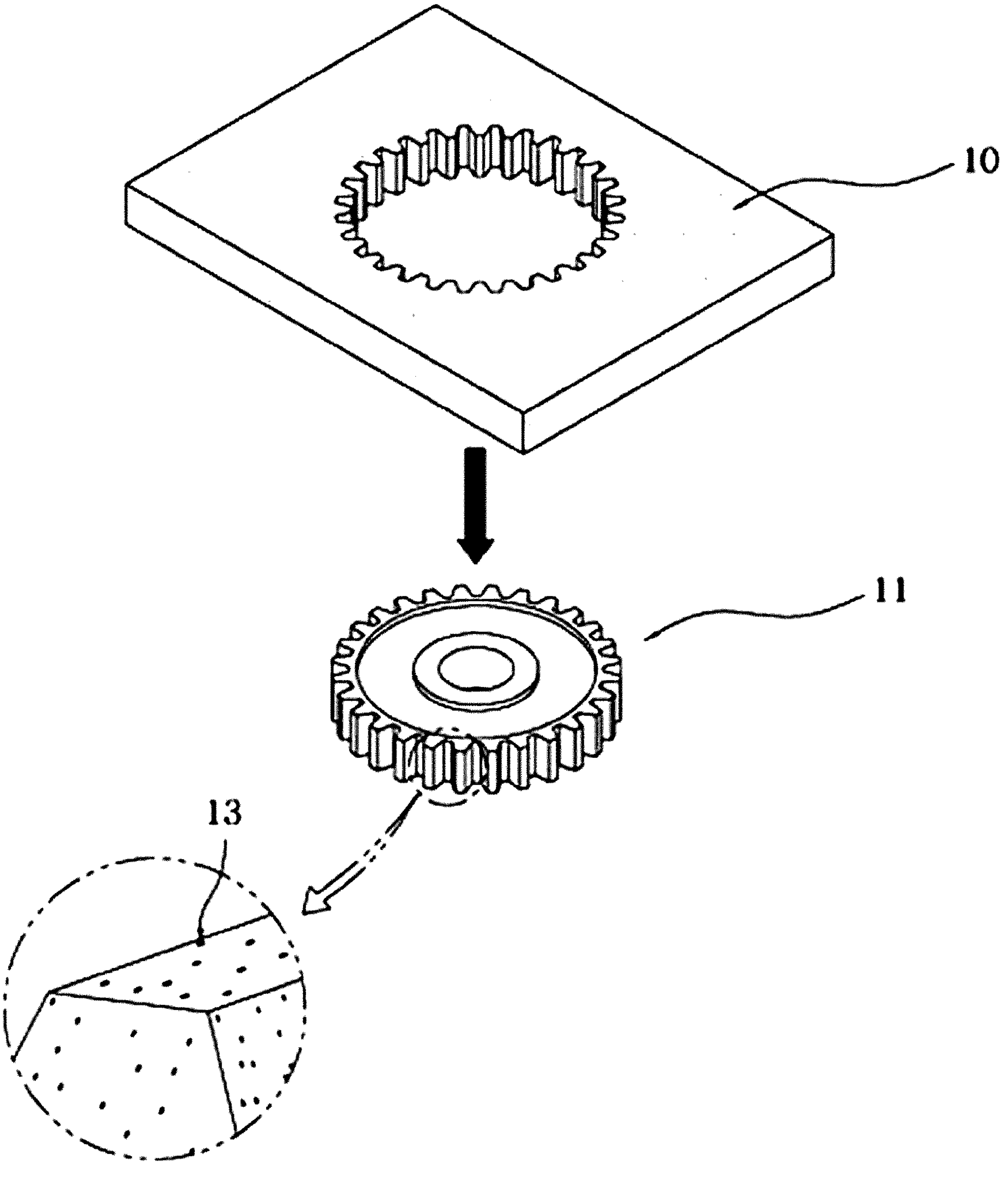

INTERNATIONAL SEARCH REPORT

International application No.
PCT/KR2015/011026

A. CLASSIFICATION OF SUBJECT MATTER

*C08G 67/02(2006.01)i, C08L 83/04(2006.01)i, C08J 5/00(2006.01)i, B29C 45/00(2006.01)i, F24C 15/08(2006.01)i, A47L 7/00(2006.01)i, B60J 3/00(2006.01)i, B60J 5/00(2006.01)i, E05B 85/20(2014.01)i*

According to International Patent Classification (IPC) or to both national classification and IPC

B. FIELDS SEARCHED

Minimum documentation searched (classification system followed by classification symbols)

C08G 67/02; D06M 101:30; D06M 15/53; C08L 73/00; C08L 77/00; D01F 6/76; C08L 101/02; C08L 77/12; D01D 5/06; C08L 83/04; C08J 5/00; B29C 45/00; F24C 15/08; A47L 7/00; B60J 3/00; B60J 5/00; E05B 85/20

Documentation searched other than minimum documentation to the extent that such documents are included in the fields searched
Korean Utility models and applications for Utility models: IPC as above
Japanese Utility models and applications for Utility models: IPC as above

Electronic data base consulted during the international search (name of data base and, where practicable, search terms used)
eKOMPASS (KIPO internal) & Keywords: polyketone, copolymer, injection molding, repeating unit, abrasion resistance

C. DOCUMENTS CONSIDERED TO BE RELEVANT

| Category* | Citation of document, with indication, where appropriate, of the relevant passages | Relevant to claim No. |
|---|---|---|
| X | JP 2007-131651 A (ASAHI KASEI CHEMICALS CORP.) 31 May 2007<br>See paragraphs [0009]-[0011]; claims 1-3. | 1 |
| X | JP 2007-131652 A (ASAHI KASEI CHEMICALS CORP.) 31 May 2007<br>See paragraphs [0011]-[0014]; and claims 1-3. | 1 |
| X | JP 2008-007582 A (ASAHI KASEI CHEMICALS CORP.) 17 January 2008<br>See paragraphs [0012]-[0014]; and claims 1 and 2. | 1 |
| A | JP 2001-207384 A (ASAHI KASEI CORP.) 03 August 2001<br>See paragraphs [0006]-[0011]; and claims 1-3. | 1 |
| A | KR 10-2012-0071814 A (HYOSUNG CORPORATION) 03 July 2012<br>See abstract; and claim 1. | 1 |

☐ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 22 MARCH 2016 (22.03.2016) | **23 MARCH 2016 (23.03.2016)** |

| Name and mailing address of the ISA/KR | Authorized officer |
|---|---|
| Korean Intellectual Property Office<br>Government Complex-Daejeon, 189 Seonsa-ro, Daejeon 302-701, Republic of Korea<br>Facsimile No. 82-42-472-7140 | Telephone No. |

Form PCT/ISA/210 (second sheet) (January 2015)

INTERNATIONAL SEARCH REPORT

International application No.
PCT/KR2015/011026

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

See extra sheet.

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☒ No required additional search fees were timely paid by the applicant. Consequently, this international search report is restricted to the invention first mentioned in the claims; it is covered by claims Nos.:
Claim 1

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

**INTERNATIONAL SEARCH REPORT**
Information on patent family members

International application No.
**PCT/KR2015/011026**

| Patent document cited in search report | Publication date | Patent family member | Publication date |
|---|---|---|---|
| JP 2007-131651 A | 31/05/2007 | NONE | |
| JP 2007-131652 A | 31/05/2007 | NONE | |
| JP 2008-007582 A | 17/01/2008 | NONE | |
| JP 2001-207384 A | 03/08/2001 | JP 4265718 B2 | 20/05/2009 |
| KR 10-2012-0071814 A | 03/07/2012 | NONE | |

Form PCT/ISA/210 (patent family annex) (January 2015)

INTERNATIONAL SEARCH REPORT

International application No.
PCT/KR2015/011026

The invention of group 1 (claim 1): a polyketone composition having excellent abrasion resistance, prepared by injection-molding a polyketone copolymer having repeating units represented by general formulae (1) and (2) in which y/x is 0.03-0.3.

The inventions of group 2 (claims 2-6): a polyketone molded component, in which an abrasion amount measured at a speed of 50 rpm under a load of 150 N in an abrasion distance of 3 km is 0.005 g or less, prepared by injection molding a polyketone composition obtained by blending: a linear alternating polyketone comprising carbon monoxide and at least one type of olefin-based unsaturated hydrocarbon; and at least one selected from the group consisting of silicon, polytetrafluoroethylene, calcium carbonate, maleic acid, molybdenum, glass fiber and magnesium stearate.

The inventions of group 3 (claims 7-10): a microwavable container manufactured by injection-molding a linear alternating polyketone, which comprises carbon monoxide and at least one type of olefin-based unsaturated hydrocarbon and has 50 ppm or less of a residual amount of a palladium catalyst and a molecular weight distribution of 1.5-2.5.

The inventions of group 4 (claims 11-35): an article, which is manufactured by using a polyketone copolymer (y/x is 0.03-0.3) comprising repeating units represented by general formulae (1) and (2), or in which the polyketone copolymer is used as a material.

The inventions of group 5 (claims 36-41): an office polyketone component manufactured by injection molding: a linear alternating polyketone, which comprises carbon monoxide and at least one type of olefin-based unsaturated hydrocarbon and has a molecular weight distribution of 1.5-2.5 and 20 ppm or less of a residual amount of a palladium catalyst; or a composition containing the linear alternating polyketone.

The inventions of group 6 (claims 42-51 and 56-97): an article manufactured by injection molding: a linear alternating polyketone, which comprises carbon monoxide and at least one type of olefin-based unsaturated hydrocarbon and has 5-50 ppm or less of a residual amount of a palladium catalyst and a molecular weight distribution of 1.5-3.0; or a polyketone composition containing the linear alternating polyketone.

The inventions of group 7 (claims 52-55, 98-101 and 107-110): an article manufactured by injection molding a blend comprising: 60-85 wt% of a linear alternating polyketone, which comprises carbon monoxide and at least one type of olefin-based unsaturated hydrocarbon and has 5-50 ppm or less of a residual amount of a palladium catalyst and a molecular weight distribution of 1.5-3.0; and 15-40 wt% of glass fiber.

The inventions of group 8 (claims 102-106): a vehicle outside door handle manufactured by injection molding a blend in which glass fiber, mineral filler and a linear alternating polyketone, which comprises carbon monoxide and at least one type of olefin-based unsaturated hydrocarbon and has 5-50 ppm or less of a residual amount of a palladium catalyst and a molecular weight distribution of 1.5-3.0 ; and 15-40 wt% of glass fiber, are mixed, wherein the glass fiber is in an amount of 5-30 wt% and the mineral filler is in an amount of 10-20 wt% relative to the total weight of the blend.

The inventions of group 9 (claims 111-130): an article manufactured by injection molding a polyketone composition comprising a linear alternating polyketone as a polyketone copolymer having repeating units represented by general formulae (1) and (2) in which y/x is 0.1-0.3.

INTERNATIONAL SEARCH REPORT

International application No.

PCT/KR2015/011026

The inventions of group 10 (claims 131-135): a polyketone resin composition for an ATM gear, containing 0.1-15 parts by weight relative to 100 parts by weight of a polyketone resin as a polyketone copolymer having repeating units represented by general formulae (1) and (2) in which y/x is 0.03-0.3.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 101086028
- US 4870133 A


### Non-patent literature cited in the description


- IUPAC Inorganic Chemistry Nomenclature revised. 1989